# EUROPEAN PATENT APPLICATION

(11) **EP 4 548 935 A2**
(43) Date of publication of application: **07.05.2025**
(21) Application number: 24174999.3
(22) Date of filing: 27.03.2020
(51) Int. Cl.: A61K 45/06

(54) **FGFR TYROSINE KINASE INHIBITORS FOR THE TREATMENT OF UROTHELIAL CARCINOMA**

(30) Priority: 29.03.2019 EP 19166429; 12.04.2019 US 201962833395 P
(62) Divisional of application: 20713656.5
(71) Applicant: JANSSEN Pharmaceutica NV, 2340 Beerse (BE)
(72) Inventor: DE PORRE, Peter Marie Z., 2340 Beerse (BE)
(74) Representative: Carpmaels & Ransford LLP

(57) **Abstract**

Described herein methods of treating urothelial carcinoma with an approved drug product containing a fibroblapst growth factor receptor (FGFR) inhibitor. Also described herein are methods of selling or offering for sale an approved drug product containing a fibroblast growth factor receptor (FGFR) inhibitor.

## Description

### TECHNICAL FIELD

Disclosed herein are methods of treating urothelial carcinoma with an approved drug product containing a fibroblast growth factor receptor (FGFR) inhibitor. Also disclosed are methods of selling or offering for sale an approved drug product containing a fibroblast growth factor receptor (FGFR) inhibitor.

### BACKGROUND

The identification of genetic abnormalities can be useful in selecting the appropriate therapeutic(s) for cancer patients. This is also useful for cancer patients failing the main therapeutic option (front-line therapy) for that cancer type, particularly if there is no accepted standard of care for second and subsequent-line therapy. Fibroblast growth factor receptors (FGFRs) are a family of receptor tyrosine kinases involved in regulating cell survival, proliferation, migration and differentiation. FGFR alterations including FGFR mutations and FGFR fusions or translocations have been observed in some cancers. To date, there are no approved therapies with an FGFR inhibitor that are efficacious in patients with FGFR alterations.

### SUMMARY

Described herein are methods of treating urothelial carcinoma comprising, consisting of, or consisting essentially of administering an approved drug product containing a fibroblast growth factor receptor (FGFR) inhibitor to a patient with a urothelial carcinoma in an amount that is described in a drug product label for said drug product. In certain embodiments, the urothelial carcinoma is locally advanced or metastatic. In further embodiments, administration of the FGFR inhibitor provides improved anti-tumor activity as measured by objective response rate or duration of response relative to a patient with urothelial carcinoma that is not receiving treatment with an FGFR inhibitor. In some embodiments, administration of the FGFR inhibitor results in no more than a grade 3 adverse event.

In further embodiments, the urothelial carcinoma is susceptible to an FGFR2 genetic alteration or an FGFR3 genetic alteration. In certain embodiments, the FGFR2 or FGFR3 genetic alteration is an FGFR3 gene mutation or an FGFR2 or FGFR3 gene fusion. In some embodiments, the FGFR3 gene mutation is R248C, S249C, G370C, Y373C, or any combination thereof. In further embodiments, the FGFR2 or FGFR3 gene fusion is FGFR3-TACC3, FGFR3-BAIAP2L1, FGFR2-BICC1, FGFR2-CASP7, or any combination thereof.

Also described herein are methods of treating urothelial carcinoma further comprising, consisting of, or consisting essentially of evaluating a biological sample from the patient for the presence of one or more FGFR2 or FGFR3 genetic alterations, in particular, the FGFR2 or FGFR3 genetic alterations as described herein, prior to administration of the FGFR inhibitor. In certain embodiments, the biological sample is blood, lymph fluid, bone marrow, a solid tumor sample, or any combination thereof.

In further embodiments, the patient received at least one prior therapy for the treatment of urothelial carcinoma. In some embodiments, the at least one prior therapy for the treatment of urothelial carcinoma is platinum-containing chemotherapy. In certain embodiments, the urothelial carcinoma progressed during or following at least one line of the platinum-containing chemotherapy. In further embodiments, the platinum-containing chemotherapy is neoadjuvant platinum-containing chemotherapy or adjuvant platinum-containing chemotherapy. In still further embodiments, the urothelial carcinoma progressed during or within 12 months following at least one line of the neoadjuvant platinum-containing chemotherapy or adjuvant platinum-containing chemotherapy.

In some embodiments, the FGFR inhibitor is erdafitinib. In further embodiments, erdafitinib is administered daily, in particular once daily. In still further embodiments, erdafitinib is administered orally. In certain embodiments, erdafitinib is administered orally on a continuous daily dosing schedule. In some embodiments, erdafitinib is administered orally at a dose of about 8 mg once daily. In some embodiments, erdafitinib is administered orally at a dose of about 8 mg once daily on a continuous daily dosing schedule. In further embodiments, the dose of erdafitinib is increased from 8 mg once daily to 9 mg once daily at 14 to 21 days after initiating treatment if: (a) the patient exhibits a serum phosphate (PO4) level that is less than about 5.5 mg/dL at 14-21 days after initiating treatment; and (b) administration of erdafitinib at 8 mg once daily resulted in no ocular disorder; or (c) administration of erdafitinib at 8 mg once daily resulted in no Grade 2 or greater adverse reaction. In certain embodiments, erdafitinib is present in a solid dosage form. In further embodiments, the solid dosage form is a tablet.

In still further embodiments, erdafitinib is not co-administered with: (a) a medication that is a strong CYP2C9 inhibitor or CYP3A4 inhibitor; (b) a medication that is a strong CYP2C9 inducer or CYP3A4 inducer; (c) a medication that is a moderate CYP2C9 inducer or CYP3A4 inducer; or (d) a medication that is a serum phosphate level-altering agent. In certain embodiments, erdafitinib is not co-administered with: (a) a medication that is a CYP3A4 substrate; (b) a medication that is a OCT2 substrate; or (c) a medication that is a P-glycotprotein (P-gp) substrate.

Also described herein are methods of treating urothelial carcinoma in a patient comprising, consisting of, or consisting essentially of: (a) evaluating a biological sample from the patient for the presence of one or more fibroblast growth factor receptor (FGFR) gene alterations; and (b) treating the patient with an approved drug product containing an FGFR inhibitor in an amount that is described in a drug product label for said drug product if one or more FGFR gene alterations is present in the sample.

Also provided herein are methods of selling an approved drug product comprising, consisting of, or consisting essentially of erdafitinib, said method comprising, consisting of, or consisting essentially of selling such drug product, wherein a drug product label for a reference listed drug for such drug product includes instructions for treating urothelial carcinoma. In certain embodiments, the drug product is an ANDA drug product, a supplemental New Drug Application drug product or a 505(b)(2) drug product.

Further provided herein are methods of offering for sale an approved drug product comprising erdafitinib, said method comprising, consisting of, or consisting essentially of offering for sale such drug product, wherein a drug product label for a reference listed drug for such drug product includes instructions for treating urothelial carcinoma. In certain embodiments, the drug product is an ANDA drug product, a supplemental New Drug Application drug product or a 505(b)(2) drug product.

Also described herein are methods comprising, consisting of, or consisting essentially of selling an approved drug product comprising erdafitinib, wherein the drug product label for a reference listed drug for such drug product comprises objective response rate or duration of response data. In certain embodiments, the objective response rate data for erdafitinib is about 40.2%, in particular, wherein the patient has locally advanced or metastatic urothelial carcinoma. In certain embodiments, the objective response rate data for erdafitinib is about 32.2%, in particular, wherein the patient has locally advanced or metastatic urothelial carcinoma which has progressed during or following at least one line of prior platinum-containing chemotherapy including within 12 months of neoadjuvant or adjuvant platinum-containing chemotherapy (chemotherapy-relapsed/refractory disease). In further embodiments, the duration of response data for erdafitinib is about 5.6 months, in particular, wherein the patient has locally advanced or metastatic urothelial carcinoma. In further embodiments, the duration of response data for erdafitinib is about 5.4 months, in particular, wherein the patient has locally advanced or metastatic urothelial carcinoma which has progressed during or following at least one line of prior platinum-containing chemotherapy including within 12 months of neoadjuvant or adjuvant platinum-containing chemotherapy (chemotherapy-relapsed/refractory disease).

Still further provided herein are methods of improving objective response rate or duration or response in a patient with urothelial carcinoma, said method comprising, consisting of, or consisting essentially of administering to said patient an approved drug product comprising erdafitinib. In certain embodiments, the objective response rate is about 40.2%, in particular, wherein the patient has locally advanced or metastatic urothelial carcinoma. In certain embodiments, the objective response rate for erdafitinib is about 32.2%, in particular, wherein the patient has locally advanced or metastatic urothelial carcinoma which has progressed during or following at least one line of prior platinum-containing chemotherapy including within 12 months of neoadjuvant or adjuvant platinum-containing chemotherapy (chemotherapy-relapsed/refractory disease). In further embodiments, the duration of response is about 5.6 months, in particular, wherein the patient has locally advanced or metastatic urothelial carcinoma. In further embodiments, the duration of response for erdafitinib is about 5.4 months, in particular, wherein the patient has locally advanced or metastatic urothelial carcinoma which has progressed during or following at least one line of prior platinum-containing chemotherapy including within 12 months of neoadjuvant or adjuvant platinum-containing chemotherapy (chemotherapy-relapsed/refractory disease). In still further embodiments, the approved drug product is an ANDA drug product or a supplemental New Drug Application drug product.

Also provided herein are methods of improving objective response rate or duration of response in a patient with urothelial carcinoma, said method comprising, consisting of, or consisting essentially of providing to said patient an approved drug product comprising erdafitinib. In certain embodiments, the objective response rate is about 40.2%, in particular, wherein the patient has locally advanced or metastatic urothelial carcinoma. In certain embodiments, the objective response rate for erdafitinib is about 32.2%, in particular, wherein the patient has locally advanced or metastatic urothelial carcinoma which has progressed during or following at least one line of prior platinum-containing chemotherapy including within 12 months of neoadjuvant or adjuvant platinum-containing chemotherapy (chemotherapy-relapsed/refractory disease). In further embodiments, the duration of response is about 5.6 months, in particular, wherein the patient has locally advanced or metastatic urothelial carcinoma. In further embodiments, the duration of response for erdafitinib is about 5.4 months, in particular, wherein the patient has locally advanced or metastatic urothelial carcinoma which has progressed during or following at least one line of prior platinum-containing chemotherapy including within 12 months of neoadjuvant or adjuvant platinum-containing chemotherapy (chemotherapy-relapsed/refractory disease). In certain embodiments, the approved drug product is an ANDA drug product or a supplemental New Drug Application drug product. In further embodiments, the drug product label for a reference listed drug for such drug product includes instructions for treating urothelial carcinoma.

Further provided herein are approved drug products with at least one approved indication, wherein said approved drug product comprises erdafitinib. In certain embodiments, the approved drug product is an NDA drug product, an ANDA drug product, a supplemental New Drug Application drug product, or a 505(b)(2) drug product. In further embodiments, a reference listed drug product for the approved drug product includes a drug product label. In still further embodiments, the drug product label comprises objective response rate data. In some embodiments, the objective response rate data for erdafitinib is about 40.2%, in particular, wherein the patient has locally advanced or metastatic urothelial carcinoma. In certain embodiments, the objective response rate data for erdafitinib is about 32.2%, in particular, wherein the patient has locally advanced or metastatic urothelial carcinoma which has progressed during or following at least one line of prior platinum-containing chemotherapy including within 12 months of neoadjuvant or adjuvant platinum-containing chemotherapy (chemotherapy-relapsed/refractory disease). In certain embodiments, the drug product label comprises duration of response data. In further embodiments, the duration of response data for erdafitinib is about 5.6 months, in particular, wherein the patient has locally advanced or metastatic urothelial carcinoma. In further embodiments, the duration of response data for erdafitinib is about 5.4 months, in particular, wherein the patient has locally advanced or metastatic urothelial carcinoma which has progressed during or following at least one line of prior platinum-containing chemotherapy including within 12 months of neoadjuvant or adjuvant platinum-containing chemotherapy (chemotherapy-relapsed/refractory disease).

Further provided herein are approved pharmaceutical products comprising erdafitinib for the treatment of adult patients with locally advanced or metastatic urothelial carcinoma that has (a) susceptible FGFR3 or FGFR2 genetic alterations and (b) progressed during or following at least one line of prior platinum containing chemotherapy including within 12 months of neoadjuvant or adjuvant platinum-containing chemotherapy.

### BRIEF DESCRIPTION OF THE DRAWINGS

The summary, as well as the following detailed description, is further understood when read in conjunction with the appended drawings. For the purpose of illustrating the disclosed methods, the drawings show exemplary embodiments of the methods; however, the methods are not limited to the specific embodiments disclosed. In the drawings:

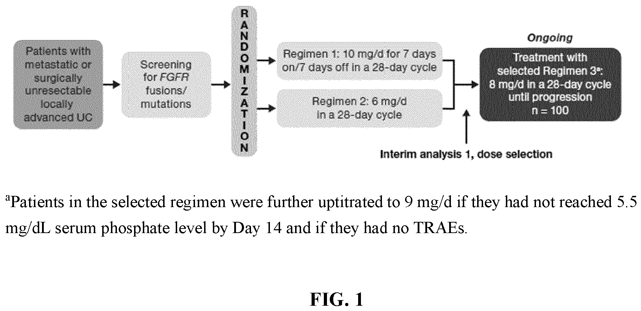

**FIG. 1** represents the study scheme for the Phase 2, multicenter, open-label study to evaluate the efficacy and safety of erdafitinib in subjects with metastatic or surgically unresectable urothelial cancer harboring selected FGFR (fibroblast growth factor receptor) genetic alterations (FGFR translocations or mutations).

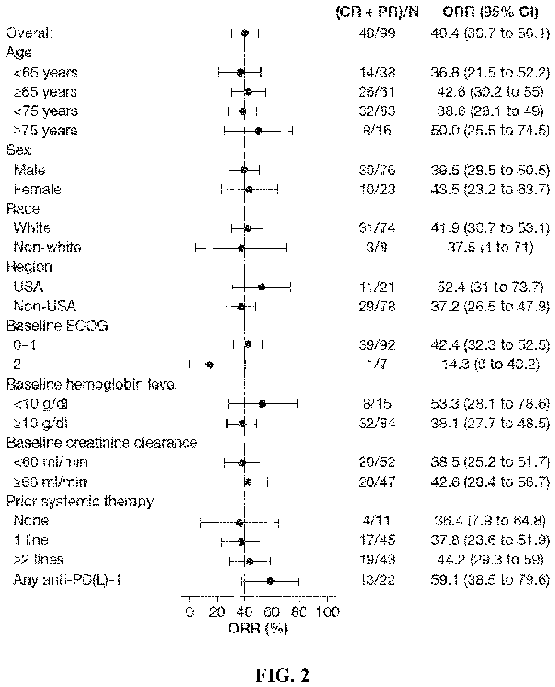

**FIG. 2** shows patient responses to treatment with 8 mg per day continuous erdafitinib (Regimen 3): Objective response rates (ORRs) among patient subgroups.

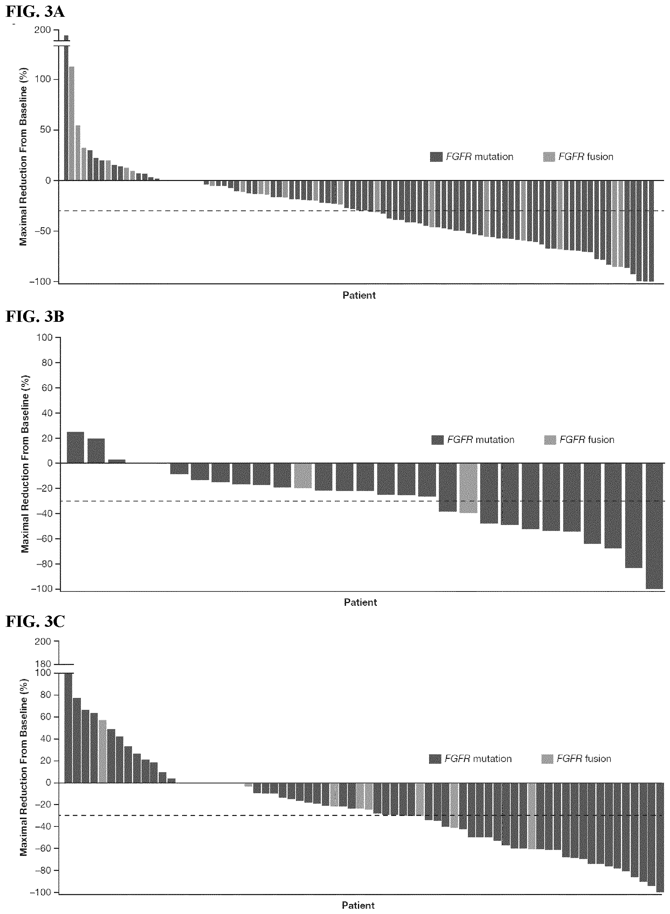

**FIG. 3****,** which comprises **FIGS. 3A-C**, shows waterfall plots of reduction in the sum of target lesion diameters after treatment with erdafitinib. Reductions in patients treated with **(****FIG. 3A****)** 8 mg per day continuous erdafitinib (regimen 3), **(****FIG. 3B****)** 10 mg intermittent erdafitinib (regimen 1), and **(****FIG. 3C****)** 6 mg per day continuous erdafitinib (regimen 2) among all treated patients.

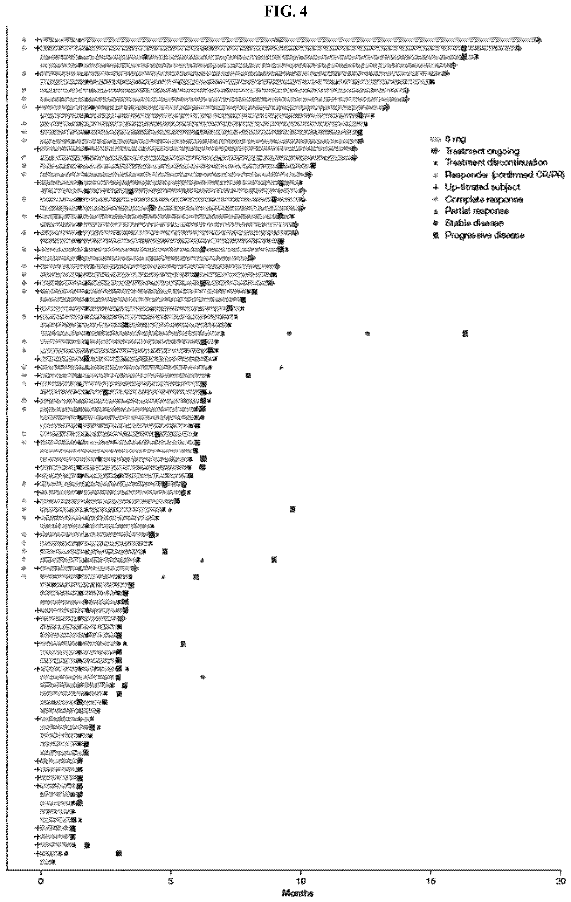

**FIG. 4** is a swimmer plot of responses to treatment with erdafitinib among all patients treated with 8 mg per day continuous erdafitinib. Responses per investigator assessment

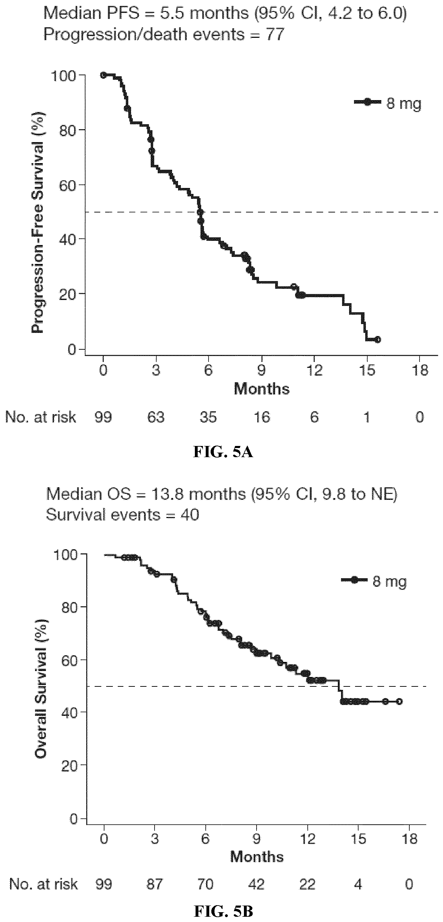

**FIG. 5****,** which comprises **FIGS. 5A-5B****,** depicts progression-free survival and overall survival among patients treated with 8 mg per day continuous erdafitinib (Regimen 3). Kaplan-Meier curve of **(****FIG. 5A****)** progression-free survival and **(****FIG. 5B****)** overall survival after treatment with 8 mg continuous erdafitinib.

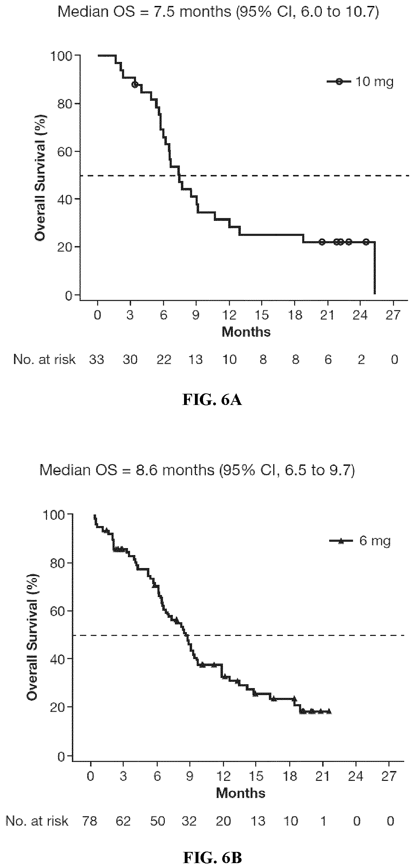

**FIG. 6****,** which comprises **FIGS. 6A-6B****,** depicts overall survival among patients treated with 10 mg intermittent and 6 mg per day continuous erdafitinib. Kaplan-Meier curves of overall survival after treatment with **(****FIG. 6A****)** 10 mg intermittent erdafitinib (regimen 1) and **(****FIG. 6B****)** 6 mg per day continuous erdafitinib (regimen 2).

### DETAILED DESCRIPTION OF ILLUSTRATIVE EMBODIMENTS

It is to be appreciated that certain features of the invention which are, for clarity, described herein in the context of separate embodiments may also be provided in combination in a single embodiment. That is, unless obviously incompatible or specifically excluded, each individual embodiment is deemed to be combinable with any other embodiment(s) and such a combination is considered to be another embodiment. Conversely, various features of the invention that are, for brevity, described in the context of a single embodiment, may also be provided separately or in any sub-combination. Finally, although an embodiment may be described as part of a series of steps or part of a more general structure, each said step may also be considered an independent embodiment in itself, combinable with others.

### Certain Terminology

The transitional terms "comprising," "consisting essentially of," and "consisting" are intended to connote their generally in accepted meanings in the patent vernacular; that is, (i) "comprising," which is synonymous with "including," "containing," or "characterized by," is inclusive or open-ended and does not exclude additional, unrecited elements or method steps; (ii) "consisting of excludes any element, step, or ingredient not specified in the claim; and (iii) "consisting essentially of limits the scope of a claim to the specified materials or steps "and those that do not materially affect the basic and novel characteristic(s)" of the claimed invention. More specifically, the basic and novel characteristics relates to the ability of the method to provide at least one of the benefits described herein, including but not limited to the ability to improve the survivability of the human population relative to the survivability of the comparative human population described elsewhere herein. Embodiments described in terms of the phrase "comprising" (or its equivalents), also provide, as embodiments, those which are independently described in terms of "consisting of and "consisting essentially of."

When a value is expressed as an approximation by use of the descriptor "about," it will be understood that the particular value forms another embodiment. In general, use of the term "about" indicates approximations that can vary depending on the desired properties sought to be obtained by the disclosed subject matter and is to be interpreted in the specific context in which it is used, based on its function. The person skilled in the art will be able to interpret this as a matter of routine. In some cases, the number of significant figures used for a particular value may be one non-limiting method of determining the extent of the word "about." In other cases, the gradations used in a series of values may be used to determine the intended range available to the term "about" for each value. Where present, all ranges are inclusive and combinable. That is, references to values stated in ranges include every value within that range.

If not otherwise specified, the term "about" signifies a variance of ±10% of the associated value, but additional embodiments include those where the variance may be ±5%, ±15%, ±20%, ±25%, or ±50%.

When a list is presented, unless stated otherwise, it is to be understood that each individual element of that list, and every combination of that list, is a separate embodiment. For example, a list of embodiments presented as "A, B, or C" is to be interpreted as including the embodiments, "A," "B," "C.," "A or B," "A or C," "B or C," or "A, B, or C."

As used herein, the singular forms "a," "an," and "the" include the plural.

The following abbreviations are used throughout the disclosure: FGFR (fibroblast growth factor receptor); FGFR3-TACC3 V1 (fusion between genes encoding FGFR3 and transforming acidic coiled-coil containing protein 3 variant 1); FGFR3-TACC3_v3 (fusion between genes encoding FGFR3 and transforming acidic coiled-coil containing protein 3 variant 3); FGFR3-BAIAP2L1 (fusion between genes encoding FGFR3 and brain-specific angiogenesis inhibitor 1-associated protein 2-like protein 1); FGFR2-BICC1 (fusion between genes encoding FGFR2 and bicaudal C homolog 1); FGFR2-CASP7 (fusion between genes encoding FGFR2 and caspase 7).

As used herein, "patient" is intended to mean any animal, in particular, mammals. Thus, the methods are applicable to human and nonhuman animals, although most preferably with humans. The terms "patient" and "subject" and "human" may be used interchangeably.

The terms "treat" and "treatment" refer to the treatment of a patient afflicted with a pathological condition and refers to an effect that alleviates the condition by killing the cancerous cells, but also to an effect that results in the inhibition of the progress of the condition, and includes a reduction in the rate of progress, a halt in the rate of progress, amelioration of the condition, and cure of the condition. Treatment as a prophylactic measure (i.e., prophylaxis) is also included.

The term "cancer" as used herein refers to an abnormal growth of cells which tend to proliferate in an uncontrolled way and, in some cases, to metastasize (spread).

The terms "co-administration" or the like, as used herein, encompass administration of the selected therapeutic agents to a single patient, and are intended to include treatment regimens in which the agents are administered by the same or different route of administration or at the same or different time.

The term "pharmaceutical combination" as used herein, means a product that results from the mixing or combining of more than one active ingredient and includes both fixed and non- fixed combinations of the active ingredients. The term "fixed combination" means that the active ingredients, e.g., erdafitinib and a co-agent, are both administered to a patient simultaneously in the form of a single unit or single dosage form. The term "non-fixed combination" means that the active ingredients, e.g., erdafitinib and a co-agent, are administered to a patient as separate units or separate dosage forms, either simultaneously, concurrently or sequentially with no specific intervening time limits, wherein such administration provides safe and effective levels of the two active ingredients in the body of the human. The latter also applies to cocktail therapy, e.g., the administration of three or more active ingredients.

The term "continuous daily dosing schedule" refers to the administration of a particular therapeutic agent without any drug holidays from the particular therapeutic agent. In some embodiments, a continuous daily dosing schedule of a particular therapeutic agent comprises administration of a particular therapeutic agent every day at roughly the same time each day.

The term "progression-free survival" is defined as the time from first dose to date of documented evidence of disease progression or death, whichever comes first.

The term "duration of response" is defined as the time from initial documentation of response to the date of documented evidence of disease progression or death.

The term "overall survival" is defined as the time from first dose to the date of death. Data for patients who are alive or have unknown status is censored at the last date on which the patient is known to be alive.

The term "placebo" as used herein means administration of a pharmaceutical composition that does not include an FGFR inhibitor.

The term "randomization" as it refers to a clinical trial refers to the time when the patient is confirmed eligible for the clinical trial and gets assigned to a treatment arm.

The terms "kit" and "article of manufacture" are used as synonyms.

"Biological samples" refers to any sample for a patient in which cancerous cells can be obtained and detection of a FGFR genetic alteration is possible. Suitable biological samples include, but are not limited to, blood, lymph fluid, bone marrow, a solid tumor sample, or any combination thereof. In some embodiments, the biological sample can be formalin-fixed paraffin-embedded tissue (FFPET).

### FGFR genetic alterations

Disclosed herein are methods of treating urothelial carcinoma comprising administering an approved drug product containing a fibroblast growth factor receptor (FGFR) inhibitor to a patient with a urothelial carcinoma in an amount that is described in a drug product label for said drug product.

The fibroblast growth factor (FGF) family of protein tyrosine kinase (PTK) receptors regulates a diverse array of physiologic functions including mitogenesis, wound healing, cell differentiation and angiogenesis, and development. Both normal and malignant cell growth as well as proliferation are affected by changes in local concentration of FGFs, extracellular signaling molecules which act as autocrine as well as paracrine factors. Autocrine FGF signaling may be particularly important in the progression of steroid hormone-dependent cancers to a hormone independent state. FGFs and their receptors are expressed at increased levels in several tissues and cell lines and overexpression is believed to contribute to the malignant phenotype. Furthermore, a number of oncogenes are homologues of genes encoding growth factor receptors, and there is a potential for aberrant activation of FGF-dependent signaling in human pancreatic cancer (Knights et al., Pharmacology and Therapeutics 2010 125:1 (105-117); Korc M. et al Current Cancer Drug Targets 2009 9:5 (639-651)).

The two prototypic members are acidic fibroblast growth factor (aFGF or FGF1) and basic fibroblast growth factor (bFGF or FGF2), and to date, at least twenty distinct FGF family members have been identified. The cellular response to FGFs is transmitted via four types of high affinity transmembrane protein tyrosine-kinase fibroblast growth factor receptors (FGFR) numbered 1 to 4 (FGFR1 to FGFR4).

In certain embodiments, the urothelial carcinoma is susceptible to an FGFR2 genetic alteration or an FGFR3 genetic alteration.

As used herein, "FGFR genetic alteration" refers to an alteration in the wild type FGFR gene, including, but not limited to, FGFR fusion genes, FGFR mutations, FGFR amplifications, or any combination thereof. The terms "variant" and "alteration" are used interchangeably herein.

In certain embodiments, the FGFR2 or FGFR3 genetic alteration is an FGFR gene fusion. "FGFR fusion" or "FGFR gene fusion" refers to a gene encoding a portion of FGFR (e.g., FGRF2 or FGFR3) and one of the herein disclosed fusion partners, or a portion thereof, created by a translocation between the two genes. The terms "fusion" and "translocation" are used interchangeable herein. The presence of one or more of the following FGFR fusion genes in a biological sample from a patient can be determined using the disclosed methods or uses: FGFR3-TACC3, FGFR3-BAIAP2L1, FGFR2-BICC1, FGFR2-CASP7, or any combination thereof. In certain embodiments, FGFR3-TACC3 is FGFR3-TACC3 variant 1 (FGFR3-TACC3 v1) or FGFR3-TACC3 variant 3 (FGFR3-TACC3 v3). Table 1 provides the FGFR fusion genes and the FGFR and fusion partner exons that are fused. The sequences of the individual FGFR fusion genes are disclosed in Table 4.

**Table 1**

| **Fusion Gene** | **FGFR Exon** | **Partner Exon** |
|---|---|---|
| **FGFR2** | | |
| FGFR2-BICC1 | 19 | 3 |
| FGFR2-CASP7 | 19 | 4 |
| **FGFR3** | | |
| FGFR3-BAIAP2L1 | 18 | 2 |
| FGFR3-TACC3 v1 | 18 | 11 |
| FGFR3-TACC3 v3 | 18 | 10 |

FGFR genetic alterations include FGFR single nucleotide polymorphism (SNP). "FGFR single nucleotide polymorphism" (SNP) refers to a FGFR2 or FGFR3 gene in which a single nucleotide differs among individuals. In certain embodiments, the FGFR2 or FGFR3 genetic alteration is an FGFR3 gene mutation. In particular, FGFR single nucleotide polymorphism" (SNP) refers to a FGFR3 gene in which a single nucleotide differs among individuals. The presence of one or more of the following FGFR SNPs in a biological sample from a patient can be determined by methods known to those of ordinary skill in the art or methods disclosed in WO 2016/048833, FGFR3 R248C, FGFR3 S249C, FGFR3 G370C, FGFR3 Y373C, or any combination thereof. The sequences of the FGFR SNPs are provided in Table 2.

**Table 2**

| **FGFR3 mutant** | **Sequence** |
|---|---|
| FGFR3 R248C | TCGGACCGCGGCAACTACACCTGCGTCGTGGAGAACAAGTTTGGC AGCATCCGGCAGACGTACACGCTGGACGTGCTGGAG(T)GCTCCCC GCACCGGCCCATCCTGCAGGCGGGGCTGCCGGCCAACCAGACGGC GGTGCTGGGCAGCGACGTGGAGTTCCACTGCAAGGTGTACAGTGA |
| | CGCACAGCCCCACATCCAGTGGCTCAAGCACGTGGAGGTGAATGG CAGCAAGGTGGGCCCGGACGGCACACCCTACGTTACCGTGCTCA (SEQ ID NO:1) |
| FGFR3 S249C | GACCGCGGCAACTACACCTGCGTCGTGGAGAACAAGTTTGGCAGC ATCCGGCAGACGTACACGCTGGACGTGCTGGGTGAGGGCCCTGGG GCGGCGCGGGGGTGGGGGCGGCAGTGGCGGTGGTGGTGAGGGAG GGGGTGGCCCCTGAGCGTCATCTGCCCCCACAGAGCGCT(G)CCCG CACCGGCCCATCCTGCAGGCGGGGCTGCCGGCCAACCAGACGGCG GTGCTGGGCAGCGACGTGGAGTTCCACTGCAAGGTGTACAGTGAC GCACAGCCCCACATCCAGTGGCTCAAGCACGTGGAGGTGAATGGC AGCAAGGTGGGCCCGGACGGCACACCCTACGTTACCGTGCTCAAG GTGGGCCACCGTGTGCACGT (SEQ ID NO:2) |
| FGFR3 G370C | GCGGGCAATTCTATTGGGTTTTCTCATCACTCTGCGTGGCTGGTGG TGCTGCCAGCCGAGGAGGAGCTGGTGGAGGCTGACGAGGCG(T)GC AGTGTGTATGCAGGCATCCTCAGCTACGGGGTGGGCTTCTTCCTGT TCATCCTGGTGGTGGCGGCTGTGACGCTCTGCCGCCTGCGCAGCCC CCCCAAGAAAGGCCTGGGCTCCCCCACCGTGCACAAGATCTCCCG CTTCCCG (SEQ ID NO:3) |
| FGFR3 Y373C* | CTAGAGGTTCTCTCCTTGCACAACGTCACCTTTGAGGACGCCGGGG AGTACACCTGCCTGGCGGGCAATTCTATTGGGTTTTCTCATCACTC TGCGTGGCTGGTGGTGCTGCCAGCCGAGGAGGAGCTGGTGGAGGC TGACGAGGCGGGCAGTGTGT(G)TGCAGGCATCCTCAGCTACGGGG TGGGCTTCTTCCTGTTCATCCTGGTGGTGGCGGCTGTGACGCTCTG CCGCCTGCGCAGCCCCCCCAAGAAAGGCCTGGGCTCCCCCACCGT GCACAAGATCTCCCGCTTCCCGCTCAAGC (SEQ ID NO:4) |

| | |
|---|---|
| Sequences correspond to nucleotides 920-1510 of FGFR3 (Genebank ID # NM_000142.4). Nucleotides in bold underline represent the SNP. *Sometimes mistakenly referred to as Y375C in the literature. | |

As used herein, "FGFR genetic alteration gene panel" includes one or more of the above listed FGFR genetic alterations. In some embodiments, the FGFR genetic alteration gene panel is dependent upon the patient's cancer type.

The FGFR genetic alteration gene panel that is used in the evaluating step of the disclosed methods is based, in part, on the patient's cancer type. For patients with urothelial carcinoma, a suitable FGFR genetic alteration gene panel can comprise FGFR3-TACC3 vl, FGFR3-TACC3 v3, FGFR3-BAIAP2L1, FGFR2-BICC1, FGFR2-CASP7, FGFR3 R248C, FGFR3 S249C, FGFR3 G370C, or FGFR3 Y373C, or any combination thereof.

### FGFR inhibitors for use in the disclosed methods or uses

Suitable FGFR inhibitors for use in the disclosed methods are provided herein.

In some embodiments, if one or more FGFR genetic alterations are present in the sample, the urothelial carcinoma patient can be treated with a FGFR inhibitor disclosed in U.S. Publication No. 2013/0072457 A1 (incorporated herein by reference), including any tautomeric or stereochemically isomeric form thereof, and a N-oxide thereof, a pharmaceutically acceptable salt thereof, or a solvate thereof (suitable R groups are also disclosed in U.S. Publication No. 2013/0072457 A1).

In some aspects, for example, the patient may be treated with N-(3,5-dimethoxyphenyl)-N'-(1-methylethyl)-N-[3-(1-methyl-1H-pyrazol-4-yl)quinoxalin-6-yl]ethane-1,2-diamine (referred to herein "JNJ-42756493" or "JNJ493" or erdafitinib), including any tautomeric form thereof, N-oxides thereof, pharmaceutically acceptable salts thereof, or solvates thereof. In some embodiments, the FGFR inhibitor can be the compound of formula (I): 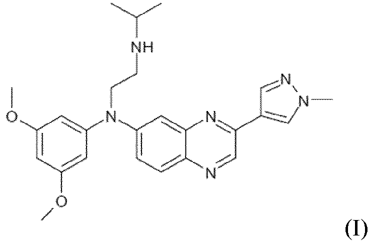 or a pharmaceutically acceptable salt thereof. In some aspects, the pharmaceutically acceptable salt is a HCl salt. In preferred aspects, erdafitinib base is used.

In some embodiments, the urothelial carcinoma patient can be treated with a FGFR inhibitor wherein the FGFR inhibitor is N-[5-[2-(3,5-Dimethoxyphenyl)ethyl]-2H-pyrazol-3-yl]-4-(3,5- diemthylpiperazin-1-yl)benzamide (AZD4547), as described in Gavine, P.R., et al., AZD4547: An Orally Bioavailable, Potent, and Selective Inhibitor of the Fibroblast Growth Factor Receptor Tyrosine Kinase Family, Cancer Res. April 15, 2012 72; 2045: 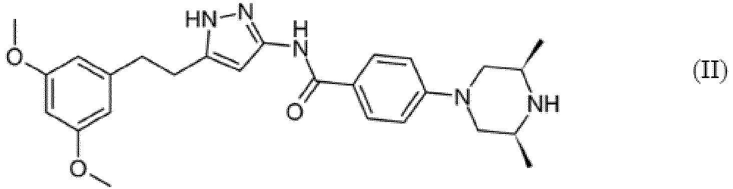 including, when chemically possible, any tautomeric or stereochemically isomeric form thereof, and a N-oxide thereof, a pharmaceutically acceptable salt thereof, or a solvate thereof.

In some embodiments, the urothelial carcinoma patient can be treated with a FGFR inhibitor wherein the FGFR inhibitor is 3-(2,6- Dichloro-3,5- dimethoxy-phenyl)-1-{6-[4-(4-ethyl-piperazin-1-yl)-phenylamino]-pyrimid-4- yl}-methyl-urea (NVP-BGJ398) as described in Int'l Publ. No. WO2006/000420: 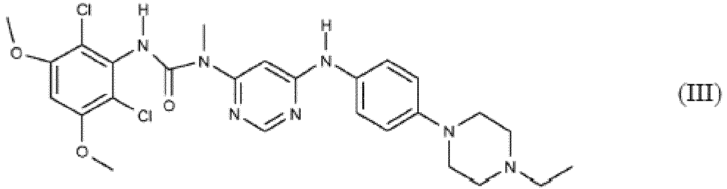 including, when chemically possible, any tautomeric or stereochemically isomeric form thereof, and a N-oxide thereof, a pharmaceutically acceptable salt thereof, or a solvate thereof.

In some embodiments, the urothelial carcinoma patient can be treated with a FGFR inhibitor wherein the FGFR inhibitor is 4-amino-5-fluoro-3-[6-(4-methylpiperazin-1-yl)-lH-benzimidazol-2-yl]- lH-quinolin-2-one (dovitinib) as described in Int't Publ. No. WO2006/127926: 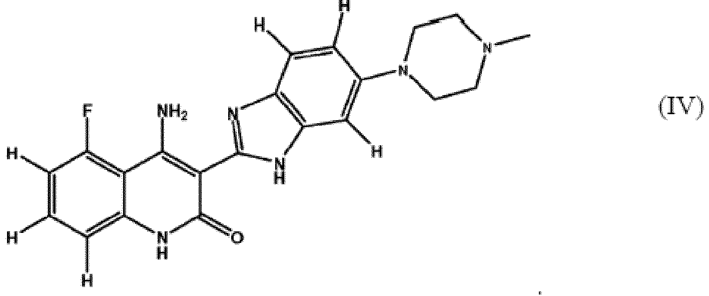 including, when chemically possible, any tautomeric or stereochemically isomeric form thereof, and a N-oxide thereof, a pharmaceutically acceptable salt thereof, or a solvate thereof.

In some embodiments, the urothelial carcinoma patient can be treated with a FGFR inhibitor wherein the FGFR inhibitor is 6-(7-((l -Aminocyclopropyl)-methoxy)-6-methoxyquinolin-4-yloxy)-N-methyl-1-naphthamide (AL3810) (lucitanib; E-3810), as described in Bello, E. et al., E-3810 Is a Potent Dual Inhibitor of VEGFR and FGFR that Exerts Antitumor Activity in Multiple Preclinical Models, Cancer Res February 15, 2011 71(A)1396-1405 and Int'l Publ. No. WO2008/112408: 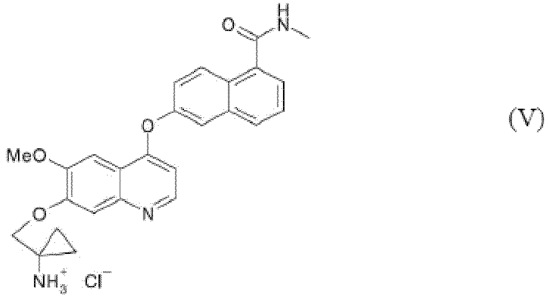 including, when chemically possible, any tautomeric or stereochemically isomeric form thereof, and a N-oxide thereof, a pharmaceutically acceptable salt thereof, or a solvate thereof.

Additional suitable FGFR inhibitors include BAY1163877 (Bayer), BAY1179470 (Bayer), TAS-120 (Taiho), ARQ087 (ArQule), ASP5878 (Astellas), FF284 (Chugai), FP-1039 (GSK/FivePrime), Blueprint, LY-2874455 (Lilly), RG-7444 (Roche), or any combination thereof, including, when chemically possible, any tautomeric or stereochemical isomeric forms thereof, N-oxides thereof, pharmaceutically acceptable salts thereof, or solvates thereof.

In an embodiment the FGFR inhibitor generally, and erdafitinib more specifically, is administered as a pharmaceutically acceptable salt. In a preferred embodiment the FGFR inhibitor generally, and erdafitinib more specifically, is administered in base form. In an embodiment the FGFR inhibitor generally, and erdafitinib more specifically, is administered as a pharmaceutically acceptable salt in an amount corresponding to 8 mg base equivalent or corresponding to 9 mg base equivalent. In an embodiment the FGFR inhibitor generally, and erdafitinib more specifically, is administered in base form in an amount of 8 mg or 9 mg.

The salts can be prepared by for instance reacting the FGFR inhibitor generally, and erdafitinib more specifically, with an appropriate acid in an appropriate solvent.

Acid addition salts may be formed with acids, both inorganic and organic. Examples of acid addition salts include salts formed with an acid selected from the group consisting of acetic, hydrochloric, hydriodic, phosphoric, nitric, sulphuric, citric, lactic, succinic, maleic, malic, isethionic, fumaric, benzenesulphonic, toluenesulphonic, methanesulphonic (mesylate), ethanesulphonic, naphthalenesulphonic, valeric, acetic, propanoic, butanoic, malonic, glucuronic and lactobionic acids. Another group of acid addition salts includes salts formed from acetic, adipic, ascorbic, aspartic, citric, DL-Lactic, fumaric, gluconic, glucuronic, hippuric, hydrochloric, glutamic, DL-malic, methanesulphonic, sebacic, stearic, succinic and tartaric acids.

In an embodiment, the FGFR inhibitor generally, and erdafitinib more specifically, is administered in the form of a solvate. As used herein, the term "solvate" means a physical association of erdafitinib with one or more solvent molecules. This physical association involves varying degrees of ionic and covalent bonding, including hydrogen bonding. In certain instances, the solvate will be capable of isolation, for example when one or more solvent molecules are incorporated in the crystal lattice of the crystalline solid. The term "solvate" is intended to encompass both solution-phase and isolatable solvates. Non-limiting examples of solvents that may form solvates include water, isopropanol, ethanol, methanol, DMSO, ethyl acetate, acetic acid or ethanolamine and the like.

Solvates are well known in pharmaceutical chemistry. They can be important to the processes for the preparation of a substance (e.g. in relation to their purification, the storage of the substance (e.g. its stability) and the ease of handling of the substance and are often formed as part of the isolation or purification stages of a chemical synthesis. A person skilled in the art can determine by means of standard and long used techniques whether a hydrate or other solvate has formed by the isolation conditions or purification conditions used to prepare a given compound. Examples of such techniques include thermogravimetric analysis (TGA), differential scanning calorimetry (DSC), X-ray crystallography (e.g. single crystal X-ray crystallography or X-ray powder diffraction) and Solid-State NMR (SS-NMR, also known as Magic Angle Spinning NMR or MAS-NMR). Such techniques are as much a part of the standard analytical toolkit of the skilled chemist as NMR, IR, HPLC and MS. Alternatively the skilled person can deliberately form a solvate using crystallization conditions that include an amount of the solvent required for the particular solvate. Thereafter the standard methods described above, can be used to establish whether solvates had formed. Also encompassed are any complexes (e.g. inclusion complexes or clathrates with compounds such as cyclodextrins, or complexes with metals).

Furthermore, the compound may have one or more polymorph (crystalline) or amorphous forms.

The compounds include compounds with one or more isotopic substitutions, and a reference to a particular element includes within its scope all isotopes of the element. For example, a reference to hydrogen includes within its scope ¹H, ²H (D), and ³H (T). Similarly, references to carbon and oxygen include within their scope respectively ¹²C, ¹³C and ¹⁴C and ¹⁶O and ¹⁸O The isotopes may be radioactive or nonradioactive. In one embodiment, the compounds contain no radioactive isotopes. Such compounds are preferred for therapeutic use. In another embodiment, however, the compound may contain one or more radioisotopes. Compounds containing such radioisotopes may be useful in a diagnostic context.

### Methods of Treatment/Compounds for Use

Described herein are methods of treating urothelial carcinoma comprising administering an approved drug product containing a fibroblast growth factor receptor (FGFR) inhibitor to a patient with a urothelial carcinoma in an amount that is described in a drug product label for said drug product.

Also described herein are approved drug products containing a fibroblast growth factor receptor (FGFR) inhibitor for use in the treatment of urothelial carcinoma in a patient, wherein the approved drug product is administered in an amount that is described in a drug product label for said drug product.

Also described herein are uses of approved drug products containing a fibroblast growth factor receptor (FGFR) inhibitor in the manufacture of a medicament for the treatment of urothelial carcinoma in a patient, wherein the medicament is administered in an amount that is described in a drug product label for said drug product.

In certain embodiments, the urothelial carcinoma is locally advanced or metastatic. In certain embodiments, the patient is a high-risk patient, in particular a high-risk patient with metastatic or surgically unresectable urothelial cancer, in particular metastatic or surgically unresectable urothelial cancer harboring select FGFR genetic alterations (FGFR translocations or mutations), in particular FGFR genetic alterations as defined herein. A high-risk patient is a patient meeting one or more of the following criteria: age ≥75 years; ECOG PS 2; hemoglobin <10 g/dL; visceral metastases, in particular of the liver, lung and/or bone; and 2 or 3 Bellmunt risk factors. In an embodiment the hemoglobin level is measured in whole blood.

In certain embodiments, administration of the FGFR inhibitor provides improved anti-tumor activity as measured by objective response rate, progression-free survival, duration of response, or overall survival relative to a patient with urothelial carcinoma that is not receiving treatment with an FGFR inhibitor. In certain embodiments, administration of the FGFR inhibitor provides improved anti-tumor activity as measured by objective response rate or duration of response relative to a patient with urothelial carcinoma that is not receiving treatment with an FGFR inhibitor. In certain embodiments, administration of the FGFR inhibitor provides improved anti-tumor activity as measured by objective response rate relative to a patient with urothelial carcinoma that is not receiving treatment with an FGFR inhibitor. In certain embodiments, administration of the FGFR inhibitor provides improved anti-tumor activity as measured by complete objective response rate relative to a patient with urothelial carcinoma that is not receiving treatment with an FGFR inhibitor. In certain embodiments, administration of the FGFR inhibitor provides improved anti-tumor activity as measured by partial objective response rate relative to a patient with urothelial carcinoma that is not receiving treatment with an FGFR inhibitor. In certain embodiments, administration of the FGFR inhibitor provides improved anti-tumor activity as measured by partial objective response rate and complete objective response rate relative to a patient with urothelial carcinoma that is not receiving treatment with an FGFR inhibitor. In certain embodiments, administration of the FGFR inhibitor provides improved anti-tumor activity as measured by progression-free survival relative to a patient with urothelial carcinoma that is not receiving treatment with an FGFR inhibitor. In certain embodiments, administration of the FGFR inhibitor provides improved anti-tumor activity as measured by duration of response relative to a patient with urothelial carcinoma that is not receiving treatment with an FGFR inhibitor. In certain embodiments, administration of the FGFR inhibitor provides improved anti-tumor activity as measured by overall survival relative to a patient with urothelial carcinoma that is not receiving treatment with an FGFR inhibitor.

In certain embodiments, the improvement in anti-tumor activity is relative to treatment with placebo. In certain embodiments, the improvement in anti-tumor activity is relative to no treatment. In certain embodiments, the improvement in anti-tumor activity is relative to standard of care.

To assess objective response rate or future progression, it is necessary to estimate the overall tumor burden at baseline and use this as a comparator for subsequent measurements. Measurable disease is defined by the presence of at least one measurable lesion.

In some embodiments, administration of a safe and effective amount of the FGFR inhibitor results in no more than a grade 2 adverse event. In other embodiments, administration of a safe and effective amount of the FGFR inhibitor results in no more than a grade 3 adverse event. In some embodiments, administration of a safe and effective amount of the FGFR inhibitor results in no more than a grade 4 adverse event.

Also described herein are methods, approved drug products, and uses further comprising evaluating a biological sample from the patient for the presence of one or more FGFR2 or FGFR3 genetic alterations prior to administration of the FGFR inhibitor. In certain embodiments, the methods, approved drug products, and uses disclosed herein further comprising evaluating a biological sample from the patient for the presence of an FGFR3 gene mutation or an FGFR2 or FGFR3 gene fusion. In certain embodiments, the methods, approved drug products, and uses disclosed herein further comprises evaluating a biological sample from the patient for the presence of an FGFR3 gene mutation. In some embodiments, the methods, approved drug products, and uses disclosed herein further comprising evaluating a biological sample from the patient for the presence of FGFR3 R248C, FGFR3 S249C, FGFR3 G370C, FGFR3 Y373C, or any combination thereof. In some embodiments, the methods, approved drug products, and uses disclosed herein further comprises evaluating a biological sample from the patient for the presence of FGFR3 R248C. In some embodiments, the methods, approved drug products, and uses disclosed herein further comprises evaluating a biological sample from the patient for the presence of FGFR3 S249C. In some embodiments, the methods, approved drug products, and uses disclosed herein further comprises evaluating a biological sample from the patient for the presence of FGFR3 G370C. In some embodiments, the methods, approved drug products, and uses disclosed herein further comprises evaluating a biological sample from the patient for the presence of FGFR3 Y373C.

In certain embodiments, the methods, approved drug products, and uses disclosed herein further comprises evaluating a biological sample from the patient for the presence of an FGFR3 gene fusion or an FGFR2 gene fusion. In some embodiments, the methods, approved drug products, and uses disclosed herein further comprises evaluating a biological sample from the patient for the presence of FGFR3-TACC3, FGFR3-BAIAP2L1, FGFR2-BICC1, FGFR2-CASP7, or any combination thereof. In some embodiments, the methods and uses disclosed herein further comprises evaluating a biological sample from the patient for the presence of FGFR3-TACC3. In some embodiments, the methods, approved drug products, and uses disclosed herein further comprises evaluating a biological sample from the patient for the presence of FGFR3-BAIAP2L1. In some embodiments, the methods, approved drug products, and uses disclosed herein further comprises evaluating a biological sample from the patient for the presence of FGFR2-BICC1. In some embodiments, the methods, approved drug products, and uses disclosed herein further comprises evaluating a biological sample from the patient for the presence of FGFR2-CASP7.

Further provided herein are approved pharmaceutical products comprising erdafitinib for the treatment of adult patients with locally advanced or metastatic urothelial carcinoma that has (a) susceptible FGFR3 or FGFR2 genetic alterations and (b) progressed during or following at least one line of prior platinum containing chemotherapy including within 12 months of neoadjuvant or adjuvant platinum-containing chemotherapy.

### Evaluating a sample for the presence of one or more FGFR genetic alterations

Also provided herein are methods of treating urothelial carcinoma in a patient comprising: (a) evaluating a biological sample from the patient for the presence of one or more fibroblast growth factor receptor (FGFR) gene alterations; and (b) treating the patient with an approved drug product containing an FGFR inhibitor in an amount that is described in a drug product label for said drug product if one or more FGFR gene alterations is present in the sample.

Also described herein are approved drug products containing a fibroblast growth factor receptor (FGFR) inhibitor for use in the treatment of urothelial carcinoma in a patient, wherein the approved drug product is administered in an amount that is described in a drug product label for said drug product, and wherein a biological sample from the patient is evaluated for the presence of one or more FGFR gene alterations.

Also described herein are uses of approved drug products containing a fibroblast growth factor receptor (FGFR) inhibitor in the manufacture of a medicament for the treatment of urothelial carcinoma in a patient, wherein the medicament is administered in an amount that is described in a drug product label for said drug product, and wherein a biological sample from the patient is evaluated for the presence of one or more FGFR gene alterations.

The following methods for evaluating a biological sample for the presence of one or more FGFR genetic alterations apply equally to any of the above disclosed methods of treatment and uses.

The disclosed methods are suitable for treating cancer in a patient if one or more FGFR genetic alterations are present in a biological sample from the patient. In some embodiments, the FGFR genetic alteration can be one or more FGFR fusion genes. In some embodiments, the FGFR genetic alteration can be one or more FGFR mutations. In some embodiments, the FGFR genetic alteration can be one or more FGFR amplifications. In some embodiments, a combination of the one or more FGFR genetic alterations can be present in the biological sample from the patient. For example, in some embodiments, the FGFR genetic alterations can be one or more FGFR fusion genes and one or more FGFR mutations. In some embodiments, the FGFR genetic alterations can be one or more FGFR fusion genes and one or more FGFR amplifications. In some embodiments, the FGFR genetic alterations can be one or more FGFR mutations and one or more FGFR amplifications. In yet other embodiments, the FGFR genetic alterations can be one or more FGFR fusion genes, mutations, and amplifications. Exemplary FGFR fusion genes are provided in Table 1 and include, but are not limited to: FGFR2-BICC1; FGFR2-CASP7; FGFR3-BAIAP2L1; FGFR3-TACC3 V1; FGFR3-TACC3 V3; or a combination thereof.

Suitable methods for evaluating a biological sample for the presence of one or more FGFR genetic alterations are described in the methods section herein and in WO 2016/048833, which are incorporated herein in their entirety. For example, and without intent to be limiting, evaluating a biological sample for the presence of one or more FGFR genetic alterations can comprise any combination of the following steps: isolating RNA from the biological sample; synthesizing cDNA from the RNA; and amplifying the cDNA (preamplified or non-preamplified). In some embodiments, evaluating a biological sample for the presence of one or more FGFR genetic alterations can comprise: amplifying cDNA from the patient with a pair of primers that bind to and amplify one or more FGFR genetic alterations; and determining whether the one or more FGFR genetic alterations are present in the sample. In some aspects, the cDNA can be pre-amplified. In some aspects, the evaluating step can comprise isolating RNA from the sample, synthesizing cDNA from the isolated RNA, and pre-amplifying the cDNA.

Suitable primer pairs for performing an amplification step include, but are not limited to, those disclosed in WO 2016/048833, as exemplified below:

**Table 3**

| **Target** | **Forward Primer** | **Reverse Primer 5'-3'** |
|---|---|---|
| FGFR3-TACC3 V1 | GACCTGGACCGTGTCCTTACC (SEQ ID NO:5) | CTTCCCCAGTTCCAGGTTCTT (SEQ ID NO:6) |
| FGFR3-TACC3 V3 | AGGACCTGGACCGTGTCCTT (SEQ ID NO:7) | TATAGGTCCGGTGGACAGGG (SEQ ID NO:8) |
| FGFR3-BAIAP2L1 | CTGGACCGTGTCCTTACCGT (SEQ ID NO:9) | GCAGCCCAGGATTGAACTGT (SEQ ID NO:10) |
| FGFR2-BICC1 | TGGATCGAATTCTCACTCTCACA (SEQ ID NO:11) | GCCAAGCAATCTGCGTATTTG (SEQ ID NO:12) |
| FGFR2-CASP7 | GCTCTTCAATACAGCCCTGATCA (SEQ ID NO:13) | ACTTGGATCGAATTCTCACTCTCA (SEQ ID NO:14) |
| FGFR2-CCDC6 | TGGATCGAATTCTCACTCTCACA (SEQ ID NO:15) | GCAAAGCCTGAATTTTCTTGAATAA (SEQ ID NO:16) |
| FGFR3 R248C | GCATCCGGCAGACGTACA (SEQ ID NO:17) | CCCCGCCTGCAGGAT (SEQ ID NO:18) |
| FGFR3 S249C | GCATCCGGCAGACGTACA (SEQ ID NO:19) | CCCCGCCTGCAGGAT (SEQ ID NO:20) |
| FGFR3 G370C | AGGAGCTGGTGGAGGCTGA (SEQ ID NO:21) | CCGTAGCTGAGGATGCCTG (SEQ ID NO:22) |
| FGFR3 Y373C | CTGGTGGAGGCTGACGAG (SEQ ID NO:23) | AGCCCACCCCGTAGCT (SEQ ID NO:24) |
| FGFR3 R248C | GTCGTGGAGAACAAGTTTGGC (SEQ ID NO:25) | GTCTGGTTGGCCGGCAG (SEQ ID NO:26) |
| FGFR3 S249C | GTCGTGGAGAACAAGTTTGGC (SEQ ID NO:27) | GTCTGGTTGGCCGGCAG (SEQ ID NO:28) |
| FGFR3 G370C | AGGAGCTGGTGGAGGCTGA (SEQ ID NO:29) | CCGTAGCTGAGGATGCCTG (SEQ ID NO:30) |
| FGFR3 Y373C | GACGAGGCGGGCAGTG (SEQ ID NO:31) | GAAGAAGCCCACCCCGTAG (SEQ ID NO:32) |

The presence of one or more FGFR genetic alterations can be evaluated at any suitable time point including upon diagnosis, following tumor resection, following first-line therapy, during clinical treatment, or any combination thereof.

For example, a biological sample taken from a patient may be analyzed to determine whether a condition or disease, such as cancer, that the patient is or may be suffering from is one which is characterized by a genetic abnormality or abnormal protein expression which leads to up-regulation of the levels or activity of FGFR or to sensitization of a pathway to normal FGFR activity, or to upregulation of these growth factor signaling pathways such as growth factor ligand levels or growth factor ligand activity or to upregulation of a biochemical pathway downstream of FGFR activation.

Examples of such abnormalities that result in activation or sensitization of the FGFR signal include loss of, or inhibition of apoptotic pathways, up-regulation of the receptors or ligands, or presence of genetic alterations of the receptors or ligands e.g. PTK variants. Tumors with genetic alterations of FGFR1, FGFR2 or FGFR3 or FGFR4 or up-regulation, in particular over-expression of FGFR1, or gain-of-function genetic alterations of FGFR2 or FGFR3 may be particularly sensitive to FGFR inhibitors.

The methods, approved drug products, and uses can further comprise evaluating the presence of one or more FGFR genetic alterations in the biological sample before the administering step.

The diagnostic tests and screens are typically conducted on a biological sample selected from tumor biopsy samples, blood samples (isolation and enrichment of shed tumor cells), stool biopsies, sputum, chromosome analysis, pleural fluid, peritoneal fluid, buccal spears, biopsy, circulating DNA, or urine. In certain embodiments, the biological sample is blood, lymph fluid, bone marrow, a solid tumor sample, or any combination thereof. In certain embodiments, the biological sample is a solid tumor sample.

Methods of identification and analysis of genetic alterations and up-regulation of proteins are known to a person skilled in the art. Screening methods could include, but are not limited to, standard methods such as reverse-transcriptase polymerase chain reaction (RT PCR) or in-situ hybridization such as fluorescence in situ hybridization (FISH).

Identification of an individual carrying a genetic alteration in FGFR, in particular an FGFR genetic alteration as described herein, may mean that the patient would be particularly suitable for treatment with erdafitinib. Tumors may preferentially be screened for presence of a FGFR variant prior to treatment. The screening process will typically involve direct sequencing, oligonucleotide microarray analysis, or a mutant specific antibody. In addition, diagnosis of tumor with such genetic alteration could be performed using techniques known to a person skilled in the art and as described herein such as RT-PCR and FISH.

In addition, genetic alterations of, for example FGFR, can be identified by direct sequencing of, for example, tumor biopsies using PCR and methods to sequence PCR products directly as hereinbefore described. The skilled artisan will recognize that all such well-known techniques for detection of the over expression, activation or mutations of the aforementioned proteins could be applicable in the present case.

In screening by RT-PCR, the level of mRNA in the tumor is assessed by creating a cDNA copy of the mRNA followed by amplification of the cDNA by PCR. Methods of PCR amplification, the selection of primers, and conditions for amplification, are known to a person skilled in the art. Nucleic acid manipulations and PCR are carried out by standard methods, as described for example in Ausubel, F.M. et al., eds. (2004) Current Protocols in Molecular Biology, John Wiley & Sons Inc., or Innis, M.A. et al., eds. (1990) PCR Protocols: a guide to methods and applications, Academic Press, San Diego. Reactions and manipulations involving nucleic acid techniques are also described in Sambrook et al., (2001), 3rd Ed, Molecular Cloning: A Laboratory Manual, Cold Spring Harbor Laboratory Press. Alternatively, a commercially available kit for RT-PCR (for example Roche Molecular Biochemicals) may be used, or methodology as set forth in United States patents 4,666,828; 4,683,202; 4,801,531; 5,192,659, 5,272,057, 5,882,864, and 6,218,529 and incorporated herein by reference. An example of an in-situ hybridization technique for assessing mRNA expression would be fluorescence in-situ hybridization (FISH) (see Angerer (1987) Meth. Enzymol., 152: 649).

Generally, in situ hybridization comprises the following major steps: (1) fixation of tissue to be analyzed; (2) prehybridization treatment of the sample to increase accessibility of target nucleic acid, and to reduce nonspecific binding; (3) hybridization of the mixture of nucleic acids to the nucleic acid in the biological structure or tissue; (4) post-hybridization washes to remove nucleic acid fragments not bound in the hybridization, and (5) detection of the hybridized nucleic acid fragments. The probes used in such applications are typically labelled, for example, with radioisotopes or fluorescent reporters. Preferred probes are sufficiently long, for example, from about 50, 100, or 200 nucleotides to about 1000 or more nucleotides, to enable specific hybridization with the target nucleic acid(s) under stringent conditions. Standard methods for carrying out FISH are described in Ausubel, F.M. et al., eds. (2004) Current Protocols in Molecular Biology, John Wiley & Sons Inc and Fluorescence In Situ Hybridization: Technical Overview by John M. S. Bartlett in Molecular Diagnosis of Cancer, Methods and Protocols, 2nd ed.; ISBN: 1-59259-760-2; March 2004, pps. 077-088; Series: Methods in Molecular Medicine.

Methods for gene expression profiling are described by (DePrimo et al. (2003), BMC Cancer, 3:3). Briefly, the protocol is as follows: double-stranded cDNA is synthesized from total RNA Using a (dT)24 oligomer (SEQ ID NO: 38: tttttttttt tttttttttt tttt) for priming first-strand cDNA synthesis, followed by second strand cDNA synthesis with random hexamer primers. The double-stranded cDNA is used as a template for in vitro transcription of cRNA using biotinylated ribonucleotides. cRNA is chemically fragmented according to protocols described by Affymetrix (Santa Clara, CA, USA), and then hybridized overnight on Human Genome Arrays.

Alternatively, the protein products expressed from the mRNAs may be assayed by immunohistochemistry of tumor samples, solid phase immunoassay with microtitre plates, Western blotting, 2-dimensional SDS-polyacrylamide gel electrophoresis, ELISA, flow cytometry and other methods known in the art for detection of specific proteins. Detection methods would include the use of site-specific antibodies. The skilled person will recognize that all such well-known techniques for detection of upregulation of FGFR, and/or VEGFR, or detection of FGFR, and/or VEGFR variants or mutants could be applicable in the present case.

Abnormal levels of proteins such as FGFR can be measured using standard enzyme assays, for example, those assays described herein. Activation or overexpression could also be detected in a tissue sample, for example, a tumor tissue. By measuring the tyrosine kinase activity with an assay such as that from Chemicon International. The tyrosine kinase of interest would be immunoprecipitated from the sample lysate and its activity measured.

Alternative methods for the measurement of the over expression or activation of FGFR including the isoforms thereof, include the measurement of microvessel density. This can for example be measured using methods described by Orre and Rogers (Int J Cancer (1999), 84(2) 101-8). Assay methods also include the use of markers.

Therefore, all of these techniques could also be used to identify tumors particularly suitable for treatment with the compounds of the invention.

Erdafitinib is in particular useful in treatment of a patient having a genetic alterated FGFR, in particular a mutated FGFR. In certain embodiments, the urothelial carcinoma is susceptible to an FGFR2 genetic alteration or an FGFR3 genetic alteration. In certain embodiments, the FGFR2 or FGFR3 genetic alteration is an FGFR3 gene mutation or an FGFR2 or FGFR3 gene fusion. In some embodiments, the FGFR3 gene mutation is R248C, S249C, G370C, Y373C, or any combination thereof. In further embodiments, the FGFR2 or FGFR3 gene fusion is FGFR3-TACC3, FGFR3-BAIAP2L1, FGFR2-BICC1, FGFR2-CASP7, or any combination thereof.

### Pharmaceutical Compositions and Routes of Administration

In view of its useful pharmacological properties, the FGFR inhibitor generally, and erdafitinib more specifically, may be formulated into various pharmaceutical forms for administration purposes.

In one embodiment the pharmaceutical composition (e.g. formulation) comprises at least one active compound of the invention together with one or more pharmaceutically acceptable carriers, adjuvants, excipients, diluents, fillers, buffers, stabilisers, preservatives, lubricants, or other materials well known to those skilled in the art and optionally other therapeutic or prophylactic agents.

To prepare the pharmaceutical compositions, an effective amount of the FGFR inhibitor generally, and erdafitinib more specifically, as the active ingredient is combined in intimate admixture with a pharmaceutically acceptable carrier, which carrier may take a wide variety of forms depending on the form of preparation desired for administration. The pharmaceutical compositions can be in any form suitable for oral, parenteral, topical, intranasal, ophthalmic, otic, rectal, intra-vaginal, or transdermal administration. These pharmaceutical compositions are desirably in unitary dosage form suitable, preferably, for administration orally, rectally, percutaneously, or by parenteral injection. For example, in preparing the compositions in oral dosage form, any of the usual pharmaceutical media may be employed, such as, for example, water, glycols, oils, alcohols and the like in the case of oral liquid preparations such as suspensions, syrups, elixirs and solutions; or solid carriers such as starches, sugars, kaolin, lubricants, binders, disintegrating agents and the like in the case of powders, pills, capsules and tablets.

The pharmaceutical compositions of the invention, in particular capsules and/or tablets, may include one or more pharmaceutically acceptable excipients (pharmaceutically acceptable carrier) such as disintegrants, diluents, fillers, binders, buffering agents, lubricants, glidants, thickening agents, sweetening agents, flavors, colorants, preservatives and the like. Some excipients can serve multiple purposes.

Suitable disintegrants are those that have a large coefficient of expansion. Examples thereof are hydrophilic, insoluble or poorly water-soluble crosslinked polymers such as crospovidone (crosslinked polyvinylpyrrolidone) and croscarmellose sodium (crosslinked sodium carboxymethylcellulose). The amount of disintegrant in the tablets according to the present invention may conveniently range from about 2.5 to about 15 % w/w and preferably range from about 2.5 to 7 % w/w, in particular range from about 2.5 to 5 % w/w. Because disintegrants by their nature yield sustained release formulations when employed in bulk, it is advantageous to dilute them with an inert substance called a diluent or filler.

A variety of materials may be used as diluents or fillers. Examples are lactose monohydrate, anhydrous lactose, sucrose, dextrose, mannitol, sorbitol, starch, cellulose (e.g. micro-crystalline cellulose (Avicel^{™}), silicified microcrystalline cellulose), dihydrated or anhydrous dibasic calcium phosphate, and others known in the art, and mixtures thereof (e.g. spray-dried mixture of lactose monohydrate (75 %) with microcrystalline cellulose (25 %) which is commercially available as Microcelac^{™}). Preferred are microcrystalline cellulose and mannitol. The total amount of diluent or filler in the pharmaceutical compositions of the present invention may conveniently range from about 20 % to about 95 % w/w and preferably ranges from about 55 % to about 95 % w/w, or from about 70 % to about 95 % w/w, or from about 80% to about 95% w/w, or from about 85 % to about 95%.

Lubricants and glidants can be employed in the manufacture of certain dosage forms, and will usually be employed when producing tablets. Examples of lubricants and glidants are hydrogenated vegetable oils, e.g hydrogenated Cottonseed oil, magnesium stearate, stearic acid, sodium lauryl sulfate, magnesium lauryl sulfate, colloidal silica, colloidal anhydrous silica talc, mixtures thereof, and others known in the art. Interesting lubricants are magnesium stearate, and mixtures of magnesium stearate with colloidal silica, magnesium stearate being preferred. A preferred glidant is colloidal anhydrous silica.

If present, glidants generally comprise 0.2 to 7.0 % w/w of the total composition weight, in particular 0.5 to 1.5% w/w, more in particular 1 to 1.5% w/w.

If present, lubricants generally comprise 0.2 to 7.0 % w/w of the total composition weight, in particular 0.2 to 2 % w/w, or 0.5 to 2% w/w, or 0.5 to 1.75% w/w, or 0.5 to 1.5% w/w.

Binders can optionally be employed in the pharmaceutical compositions of the present invention. Suitable binders are water-soluble polymers, such as alkylcelluloses such as methylcellulose ; hydroxyalkylcelluloses such as hydroxymethylcellulose, hydroxyethylcellulose, hydroxypropylcellulose and hydroxybutylcellulose ; hydroxyalkyl alkylcelluloses such as hydroxyethyl methylcellulose and hydroxypropyl methylcellulose ; carboxyalkylcelluloses such as carboxymethylcellulose ; alkali metal salts of carboxyalkylcelluloses such as sodium carboxymethylcellulose; carboxyalkylalkyl-celluloses such as carboxymethylethylcellulose ; carboxyalkylcellulose esters ; starches ; pectines such as sodium carboxymethylamylopectine ; chitin derivates such as chitosan ; di-, oligo- and polysaccharides such as trehalose, cyclodextrins and derivatives thereof, alginic acid, alkali metal and ammonium salts thereof, carrageenans, galactomannans, tragacanth, agar agar, gummi arabicum, guar gummi and xanthan gummi ; polyacrylic acids and the salts thereof ; polymethacrylic acids, the salts and esters thereof, methacrylate copolymers ; polyvinylpyrrolidone (PVP), polyvinylalcohol (PVA) and copolymers thereof, e.g. PVP-VA. Preferably, the water-soluble polymer is a hydroxyalkyl alkylcelluloses, such as for example hydroxypropylmethyl cellulose, e.g. hydroxypropylmethyl cellulose 15 cps.

Other excipients such as coloring agents and pigments may also be added to the compositions of the invention. Coloring agents and pigments include titanium dioxide and dyes suitable for food. A coloring agent or a pigment is an optional ingredient in the formulation of the invention, but when used the coloring agent can be present in an amount up to 3.5 % w/w based on the total composition weight.

Flavors are optional in the composition and may be chosen from synthetic flavor oils and flavoring aromatics or natural oils, extracts from plants leaves, flowers, fruits and so forth and combinations thereof. These may include cinnamon oil, oil of wintergreen, peppermint oils, bay oil, anise oil, eucalyptus, thyme oil. Also useful as flavors are vanilla, citrus oil, including lemon, orange, grape, lime and grapefruit, and fruit essences, including apple, banana, pear, peach, strawberry, raspberry, cherry, plum, pineapple, apricot and so forth, The amount of flavor may depend on a number of factors including the organoleptic effect desired. Generally the flavor will be present in an amount from about 0 % to about 3 % (w/w).

Formaldehyde scavengers are compounds that are capable of absorbing formaldehyde. They include compounds comprising a nitrogen center that is reactive with formaldehyde, such as to form one or more reversible or irreversible bonds between the formaldehyde scavenger and formaldehyde. For example, the formaldehyde scavenger comprises one or more nitrogen atoms/centers that are reactive with formaldehyde to form a schiff base imine that is capable of subsequently binding with formaldehyde. For example, the formaldehyde scavenger comprises one or more nitrogen centers that are reactive with formaldehyde to form one or more 5-8 membered cyclic rings. The formaldehyde scavenger preferably comprises one or more amine or amide groups. For example, the formaldehyde scavenger can be an amino acid, an amino sugar, an alpha amine compound, or a conjugate or derivative thereof, or a mixture thereof. The formaldehyde scavenger may comprise two or more amines and/or amides.

Formaldehyde scavengers include, for example, glycine, alanine, serine, threonine, cysteine, valine, lecuine, isoleucine, methionine, phenylalanine, tyrosine, aspartic acid, glutamic acid, arginine, lysine, ornithine, citrulline, taurine pyrrolysine, meglumine, histidine, aspartame, proline, tryptophan, citrulline, pyrrolysine, asparagine, glutamine, or a conjugate or mixture thereof; or, whenever possible, pharmaceutically acceptable salts thereof.

In an aspect of the invention, the formaldehyde scavenger is meglumine or a pharmaceutically acceptable salt thereof, in particular meglumine base.

It is another object of the invention to provide a process of preparing a pharmaceutical composition as described herein, in particular in the form of a tablet or a capsule, characterized by blending a formaldehyde scavenger, in particular meglumine, and erdafitinib, a pharmaceutically acceptable salt thereof or a solvate thereof, in particular erdafitinib base, with a pharmaceutically acceptable carrier and compressing said blend into tablets or filling said blend in capsules.

Because of their ease in administration, tablets and capsules represent the most advantageous oral dosage unit form, in which case solid pharmaceutical carriers are obviously employed. For parenteral compositions, the carrier will usually comprise sterile water, at least in large part, though other ingredients, to aid solubility for example, may be included. Injectable solutions, for example, may be prepared in which the carrier comprises saline solution, glucose solution or a mixture of saline and glucose solution. Injectable suspensions may also be prepared in which case appropriate liquid carriers, suspending agents and the like may be employed. In the compositions suitable for percutaneous administration, the carrier optionally comprises a penetration enhancing agent and/or a suitable wetting agent, optionally combined with suitable additives of any nature in minor proportions, which additives do not cause a significant deleterious effect to the skin. Said additives may facilitate the administration to the skin and/or may be helpful for preparing the desired compositions. These compositions may be administered in various ways, e.g., as a transdermal patch, as a spot-on, as an ointment. It is especially advantageous to formulate the aforementioned pharmaceutical compositions in dosage unit form for ease of administration and uniformity of dosage. Dosage unit form as used in the specification and claims herein refers to physically discrete units suitable as unitary dosages, each unit containing a predetermined quantity of active ingredient calculated to produce the desired therapeutic effect in association with the required pharmaceutical carrier. Examples of such dosage unit forms are tablets (including scored or coated tablets), capsules, pills, powder packets, wafers, injectable solutions or suspensions, teaspoonfuls, tablespoonfuls and the like, and segregated multiples thereof.

It is especially advantageous to formulate the aforementioned pharmaceutical compositions in dosage unit form for ease of administration and uniformity of dosage. Dosage unit form as used herein refers to physically discrete units suitable as unitary dosages, each unit containing a predetermined quantity of active ingredient, calculated to produce the desired therapeutic effect, in association with the required pharmaceutical carrier. Examples of such dosage unit forms are tablets (including scored or coated tablets), capsules, pills, powder packets, wafers, injectable solutions or suspensions, teaspoonfuls, tablespoonfuls and the like, and segregated multiples thereof. Preferred forms are tablets and capsules.

In certain embodiments, the FGFR inhibitor is present in a solid unit dosage form, and a solid unit dosage form suitable for oral administration. The unit dosage form may contain about 1, 2, 3, 4, 5, 6, 7, 8, 9, or 10 mg of the FGFR inhibitor per unit dose form or an amount in a range bounded by two of these values, in particular 3, 4 or 5 mg per unit dose.

Depending on the mode of administration, the pharmaceutical composition will preferably comprise from 0.05 to 99 % by weight, more preferably from 0.1 to 70 % by weight, even more preferably from 0.1 to 50 % by weight of the compound of the present invention, and, from 1 to 99.95 % by weight, more preferably from 30 to 99.9 % by weight, even more preferably from 50 to 99.9 % by weight of a pharmaceutically acceptable carrier, all percentages being based on the total weight of the composition.

Tablets or capsules of the present invention may further be film-coated e.g. to improve taste, to provide ease of swallowing and an elegant appearance. Polymeric film-coating materials are known in the art. Preferred film coatings are water-based film coatings opposed to solvent based film coatings because the latter may contain more traces of aldehydes. A preferred film-coating material is Opadry^{®} II aqueous film coating system, e.g. Opadry^{®} II 85F, such as Opadry^{®} II 85F92209. Further preferred film coatings are water-based film coatings that protects from environmental moisture, such as Readilycoat^{®} (e.g. Readilycoat^{®} D), AquaPolish^{®} MS, Opadry^{®} amb, Opadry^{®} amb II, which are aqueous moisture barrier film coating systems. A preferred film-coating is Opadry^{®} amb II, a high performance moisture barrier film coating which is a PVA-based immediate release system, without polyethylene glycol.

In tablets according to the invention, the film coat in terms of weight preferably accounts for about 4 % (w/w) or less of the total tablet weight.

For capsules according to the present invention, hypromellose (HPMC) capsules are preferred over gelatin capsules.

In an aspect of the invention, the pharmaceutical compositions as described herein, in particular in the form of a capsule or a tablet, comprise from 0.5 mg to 20 mg base equivalent, or from 2 mg to 20 mg base equivalent, or from 0.5 mg to 12 mg base equivalent, or from 2 mg to 12 mg base equivalent, or from 2 mg to 10 mg base equivalent, or from 2 mg to 6 mg base equivalent, or 2 mg base equivalent, 3 mg base equivalent, 4 mg base equivalent, 5 mg base equivalent, 6 mg base equivalent, 7 mg base equivalent, 8 mg base equivalent, 9 mg base equivalent, 10 mg base equivalent, 11 mg base equivalent or 12 mg base equivalent of erdafitinib, a pharmaceutically acceptable salt thereof or a solvate thereof. In particular, the pharmaceutical compositions as described herein comprise 3mg base equivalent, 4 mg base equivalent or 5 mg base equivalent of erdafitinib, a pharmaceutically acceptable salt thereof or a solvate thereof.

In an aspect of the invention, the pharmaceutical compositions as described herein, in particular in the form of a capsule or a tablet, comprise from 0.5 mg to 20 mg, or from 2 mg to 20 mg, or from 0.5 mg to 12 mg, or from 2 mg to 12 mg, or from 2 mg to 10 mg, or from 2 mg to 6 mg, or 2 mg, 3 mg, 4 mg, 5 mg, 6 mg, 7 mg, 8 mg, 9 mg, 10 mg, 11 mg or 12 mg of erdafitinib base. In particular, the pharmaceutical compositions as described herein comprise 3mg, 4 mg or 5 mg of erdafitinib base. In particular, the pharmaceutical compositions as described herein comprise 3mg, 4 mg or 5 mg of erdafitinib base and from about 0.5 to about 5 % w/w, from about 0.5 to about 3 % w/w, from about 0.5 to about 2% w/w, from about 0.5 to about 1.5% w/w, or from about 0.5 to about 1% w/w of a formaldehyde scavenger, in particular meglumine. In particular, the pharmaceutical compositions as described herein comprise 3mg, 4 mg or 5 mg of erdafitinib base and from about 0.5 to about 1.5% w/w or from about 0.5 to about 1% w/w of a formaldehyde scavenger, in particular meglumine.

In an aspect of the invention, more than one, e.g. two, pharmaceutical compositions as described herein can be administered in order to obtain a desired dose, e.g. a daily dose.

The amount of formaldehyde scavenger, in particular meglumine, in the pharmaceutical compositions according to the present invention may range from about 0.1 to about 10 % w/w, about 0.1 to about 5 % w/w, from about 0.1 to about 3 % w/w, from about 0.1 to about 2% w/w, from about 0.1 to about 1.5% w/w, from about 0.1 to about 1% w/w, from about 0.5 to about 5 % w/w, from about 0.5 to about 3 % w/w, from about 0.5 to about 2% w/w, from about 0.5 to about 1.5% w/w, from about 0.5 to about 1% w/w.

The term "safe and effective amount" refers to an amount of an active ingredient that elicits the desired biological or medicinal response in a subject's biological system without the risks outweighing the benefits of such response in accordance with the Federal Food, Drug, and Cosmetic Act, as amended (secs. 201-902, 52 Stat. 1040 et seq., as amended; 21 U.S.C. §§ 321-392). Safety is often measured by toxicity testing to determine the highest tolerable dose or the optimal dose of an active pharmaceutical ingredient needed to achieve the desired benefit.

Studies that look at safety also seek to identify any potential adverse effects that may result from exposure to the drug. Efficacy is often measured by determining whether an active pharmaceutical ingredient demonstrates a health benefit over a placebo or other intervention when tested in an appropriate situation, such as a tightly controlled clinical trial.

The term "acceptable" with respect to a formulation, composition or ingredient, as used herein, means that the beneficial effects of that formulation, composition or ingredient on the general health of the human being treated substantially outweigh its detrimental effects, to the extent any exist.

All formulations for oral administration are in dosage form suitable for such administration.

### Methods of Dosing and Treatment Regimens

The FGFR inhibitor generally, and erdafitinib specifically, is administered in an amount sufficient to exert its anti-tumor activity. Those skilled in the art could easily determine the effective amount from the test results presented hereinafter. In general, it is contemplated that a therapeutically effective amount would be from 0.005 mg/kg to 100 mg/kg body weight, and in particular from 0.005 mg/kg to 10 mg/kg body weight. It may be appropriate to administer the required dose as single, two, three, four or more sub-doses at appropriate intervals throughout the day. Said sub-doses may be formulated as unit dosage forms, for example, containing 0.5 to 500 mg, in particular 1 mg to 500 mg, more in particular 10 mg to 500 mg of active ingredient per unit dosage form.

In one aspect, described herein are methods of treating urothelial carcinoma comprising, consisting of, or consisting essentially of administering a safe and effective amount of an FGFR inhibitor to a patient with urothelial carcinoma, wherein the FGFR inhibitor is administered orally. In some embodiments, the FGFR inhibitor generally, and erdafitinib specifically is administered daily, in particular once daily. In some embodiments, the FGFR inhibitor generally, and erdafitinib specifically is administered twice-a-day. In some embodiments, the FGFR inhibitor generally, and erdafitinib specifically is administered three times a day. In some embodiments, the FGFR inhibitor generally, and erdafitinib specifically is administered four times a day. In some embodiments, the FGFR inhibitor generally, and erdafitinib specifically is administered every other day. In some embodiments, the FGFR inhibitor generally, and erdafitinib specifically is administered weekly. In some embodiments, the FGFR inhibitor generally, and erdafitinib specifically is administered twice a week. In some embodiments, the FGFR inhibitor generally, and erdafitinib specifically is administered every other week. In some embodiments, the FGFR inhibitor generally, and erdafitinib specifically is administered orally on a continuous daily dosage schedule.

In general, doses of the FGFR inhibitor, and erdafitinib specifically, employed for treatment of the diseases or conditions described herein in humans are typically in the range of about 1 to 20 mg per day. In some embodiments, the FGFR inhibitor, and erdafitinib specifically, is administered orally to the human at a dose of about 1 mg per day, about 2 mg per day, about 3 mg per day, about 4 mg per day, about 5 mg per day, about 6 mg per day, about 7 mg per day, about 8 mg per day, about 9 mg per day, about 10 mg per day, about 11 mg per day, about 12 mg per day, about 13 mg per day, about 14 mg per day, about 15 mg per day, about 16 mg per day, about 17 mg per day, about 18 mg per day, about 19 mg per day or about 20 mg per day.

In certain embodiments, erdafitinib is administered orally at a dose of about 8 mg once daily. In further embodiments, the dose of erdafitinib is increased from 8 mg once daily to 9 mg once daily at 14 to 21 days after initiating treatment if: (a) the patient exhibits a serum phosphate (PO₄) level that is less than about 5.5 mg/dL at 14-21 days after initiating treatment; and (b) administration of erdafitinib at 8 mg once daily resulted in no ocular disorder; or (c) administration of erdafitinib at 8 mg once daily resulted in no Grade 2 or greater adverse reaction.

In certain embodiments, the dose of erdafitinib is increased from 8 mg once daily to 9 mg once daily at 14 days after initiating treatment. In certain embodiments, the dose of erdafitinib is increased from 8 mg once daily to 9 mg once daily at 15 days after initiating treatment. In certain embodiments, the dose of erdafitinib is increased from 8 mg once daily to 9 mg once daily at 16 days after initiating treatment. In certain embodiments, the dose of erdafitinib is increased from 8 mg once daily to 9 mg once daily at 17 days after initiating treatment. In certain embodiments, the dose of erdafitinib is increased from 8 mg once daily to 9 mg once daily at 18 days after initiating treatment. In certain embodiments, the dose of erdafitinib is increased from 8 mg once daily to 9 mg once daily at 19 days after initiating treatment. In certain embodiments, the dose of erdafitinib is increased from 8 mg once daily to 9 mg once daily at 20 days after initiating treatment.

In an embodiment, erdafitinib is administered at a dose of 10 mg. In an embodiment, erdafitinib is administered at a dose of 10 mg intermittently. In an embodiment, erdafitinib is administered at a dose of 10 mg intermittently 7 days on/7 days off.

In an embodiment, erdafitinib is administered at a dose of 8 mg, in particular 8 mg once daily. In an embodiment, erdafitinib is administered at a dose of 8 mg, in particular 8 mg once daily, with an option to uptitrate to 9 mg depending on serum phosphate levels (e.g. serum phosphate levels are < 5.5 mg/dL, or are < 7 mg/dL or range from and include 7 mg/dL to ≤9 mg/dL or are ≤9 mg/dL), and depending on treatment-related adverse events observed. In an embodiment, the levels of serum phosphate for determining whether or not to up-titrate are measured on a treatment day during the first cycle of erdafitinib treatment, in particular on day 14 ± 2 days, more in particular on day 14, of erdafitinib administration.

In an embodiment, the treatment cycle as used herein is a 28-day cycle.

In one embodiment, the desired dose is conveniently presented in a single dose or in divided doses administered simultaneously (or over a short period of time) or at appropriate intervals, for example as two, three, four or more sub-doses per day. In some embodiments, the FGFR inhibitor is conveniently presented in divided doses that are administered simultaneously (or over a short period of time) once a day. In some embodiments, the FGFR inhibitor is conveniently presented in divided doses that are administered in equal portions twice-a-day. In some embodiments, the FGFR inhibitor is conveniently presented in divided doses that are administered in equal portions three times a day. In some embodiments, the FGFR inhibitor is conveniently presented in divided doses that are administered in equal portions four times a day.

In certain embodiments, the desired dose may be delivered in 1, 2, 3, 4, 5, 6, 7, 8, 9, or 10 fractional unit dosages throughout the course of the day, such that the total amount of FGFR inhibitor delivered by the fractional unit dosages over the course of the day provides the total daily dosages.

In some embodiments, the amount of the FGFR inhibitor that is given to the human varies depending upon factors such as, but not limited to, condition and severity of the disease or condition, and the identity (e.g., weight) of the human, and the particular additional therapeutic agents that are administered (if applicable).

In further embodiments, the patient received at least one prior therapy for the treatment of urothelial carcinoma. In some embodiments, the at least one prior therapy for the treatment of urothelial carcinoma is platinum-containing chemotherapy. In certain embodiments, the urothelial carcinoma progressed during or following at least one line of the platinum-containing chemotherapy. In further embodiments, the platinum-containing chemotherapy is neoadjuvant platinum-containing chemotherapy or adjuvant platinum-containing chemotherapy. In still further embodiments, the urothelial carcinoma progressed during or within 12 months following at least one line of the neoadjuvant platinum-containing chemotherapy or adjuvant platinum-containing chemotherapy.

In still further embodiments, erdafitinib is not co-administered with: (a) a medication that is a strong CYP2C9 inhibitor or CYP3A4 inhibitor; (b) a medication that is a strong CYP2C9 inducer or CYP3A4 inducer; (c) a medication that is a moderate CYP2C9 inducer or CYP3A4 inducer; or (d) a medication that is a serum phosphate level-altering agent. In certain embodiments, erdafitinib is not co-administered with a medication that is a strong CYP2C9 inhibitor or CYP3A4 inhibitor. In certain embodiments, erdafitinib is not co-administered with a medication that is a strong CYP2C9 inhibitor. In certain embodiments, erdafitinib is not co-administered with a medication that is a strong CYP3A4 inhibitor. In certain embodiments, erdafitinib is not co-administered with a medication that is a strong CYP2C9 inducer or CYP3A4 inducer. In certain embodiments, erdafitinib is not co-administered with a medication that is a strong CYP2C9 inducer. In certain embodiments, erdafitinib is not co-administered with a medication that is a strong CYP3A4 inducer. In certain embodiments, erdafitinib is not co-administered with a medication that is a moderate CYP2C9 inducer or CYP3A4 inducer. In certain embodiments, erdafitinib is not co-administered with a medication that is a moderate CYP2C9 inducer. In certain embodiments, erdafitinib is not co-administered with a medication that is a moderate CYP3A4 inducer. In certain embodiments, erdafitinib is not co-administered with a medication that is a serum phosphate level-altering agent.

In certain embodiments, erdafitinib is not co-administered with: (a) a medication that is a CYP3A4 substrate; (b) a medication that is a OCT2 substrate; or (c) a medication that is a P-glycotprotein (P-gp) substrate. In certain embodiments, erdafitinib is not co-administered with a medication that is a CYP3A4 substrate. In certain embodiments, erdafitinib is not co-administered with a medication that is a OCT2 substrate. In certain embodiments, erdafitinib is not co-administered with a medication that is a P-glycotprotein (P-gp) substrate.

Non-limiting examples of strong CYP3A4 inhibitors include Boceprevir, Aprepitant, Clarithromycin, Conivaptan, grapefruit juice, Indinavir, Lopinavir Itraconazole, Mibefradil Ketoconazole, Nefazodone, Ritonavir, Posaconazole, Nelfinavir, Saquinavir, Conivaptan, Telaprevir, Boceprevir, Telithromycin, Clarithromycin, Voriconazole, Clotrimazole, Diltiazem, Erythromycin, Fluconazole, Verapamil, and Troleandomycin.

Non-limiting examples of moderate to strong CYP3A4 inducers include Avasimibe, St. John's wort, Carbamazepine, Efavirenz, Phenytoin, Etravirine, Bosentan, Nafcillin, Rifampin, Modafinil, Rifabutin, and Barbiturates.

A non-limiting example of a strong CYP2C9 inhibitor is fluconazole.

Non-limiting examples of CYP2C9 inducers include Carbamiazepine, rifampin, Enzalutamide, secobarbital, Nevirapine, St. John's wort, and phenobarbital.

### Kits/Articles of Manufacture

For use in the methods of use described herein, kits and articles of manufacture are also described. Such kits include a package or container that is compartmentalized to receive one or more dosages of the pharmaceutical compositions disclosed herein. Suitable containers include, for example, bottles. In one embodiment, the containers are formed from a variety of materials such as glass or plastic.

The articles of manufacture provided herein contain packaging materials. Packaging materials for use in packaging pharmaceutical products include, e.g., U.S. Patent Nos. 5,323,907, 5,052,558 and 5,033,252. Examples of pharmaceutical packaging materials include, but are not limited to, blister packs, bottles, tubes, bags, containers, bottles, and any packaging material suitable for a selected formulation and intended mode of administration and treatment.

A kit typically includes labels listing contents and/or instructions for use, and package inserts with instructions for use. A set of instructions will also typically be included.

In one embodiment, a label is on or associated with the container. In one embodiment, a label is on a container when letters, numbers or other characters forming the label are attached, molded or etched into the container itself; a label is associated with a container when it is present within a receptacle or carrier that also holds the container, e.g., as a package insert.

In one embodiment, a label is used to indicate that the contents are to be used for a specific therapeutic application. The label also indicates directions for use of the contents, such as in the methods described herein.

In certain embodiments, the pharmaceutical compositions are presented in a pack or dispenser device which contains one or more unit dosage forms containing a compound provided herein. The pack, for example, contains metal or plastic foil, such as a blister pack. In one embodiment, the pack or dispenser device is accompanied by instructions for administration. In one embodiment, the pack or dispenser is also accompanied with a notice associated with the container in form prescribed by a governmental agency regulating the manufacture, use, or sale of pharmaceuticals, which notice is reflective of approval by the agency of the form of the drug for human or veterinary administration. Such notice, for example, is the labeling approved by the U.S. Food and Drug Administration for prescription drugs, or the approved product insert. In one embodiment, compositions containing a compound provided herein formulated in a compatible pharmaceutical carrier are also prepared, placed in an appropriate container, and labeled for treatment of an indicated condition.

### Methods of Sale

Provided herein are methods of selling an approved drug product comprising erdafitinib, said method comprising selling such drug product, wherein a drug product label for a reference listed drug for such drug product includes instructions for treating urothelial carcinoma. In certain embodiments, the drug product is an ANDA drug product, a supplemental New Drug Application drug product or a 505(b)(2) drug product.

The term, "drug product" or "approved drug product" is product that contains an active pharmaceutical ingredient that has been approved for marketing for at least one indication by a governmental authority, e.g., the Food and Drug Administration or the similar authority in other countries.

The term "Reference Listed Drug (RLD)" is a drug product to which new generic versions are compared to show that they are bioequivalent. 21 CFR 314.3(b)) It is also a medicinal product that has been granted marketing authorization by a Member State of the European Union or by the Commission on the basis of a completed dossier, i.e., with the submission of quality, pre-clinical and clinical data in accordance with Articles 8(3), 10a, 10b or 10c of Directive 2001/83/EC and to which the application for marketing authorization for a generic/hybrid medicinal product refers, by demonstration of bioequivalence, usually through the submission of the appropriate bioavailability studies.

In the United States, a company seeking approval to market a generic equivalent must refer to the RLD in its Abbreviated New Drug Application (ANDA). For example, an ANDA applicant relies on the FDA's finding that a previously approved drug product, i.e., the RLD, is safe and effective, and must demonstrate, among other things, that the proposed generic drug product is the same as the RLD in certain ways. Specifically, with limited exceptions, a drug product for which an ANDA is submitted must have, among other things, the same active ingredient(s), conditions of use, route of administration, dosage form, strength, and (with certain permissible differences) labeling as the RLD. The RLD is the listed drug to which the ANDA applicant must show its proposed ANDA drug product is the same with respect to active ingredient(s), dosage form, route of administration, strength, labeling, and conditions of use, among other characteristics. In the electronic Orange Book, there is a column for RLDs and a column for reference standards. In the printed version of the Orange Book, the RLDs and reference standards are identified by specific symbol. For an ANDA based on an approved suitability petition (a petitioned ANDA), the reference listed drug generally is the listed drug referenced in the approved suitability petition.

A reference standard is the drug product selected by FDA that an applicant seeking approval of an ANDA must use in conducting an in vivo bioequivalence study required for approval. FDA generally selects a single reference standard that ANDA applicants must use in in vivo bioequivalence testing. Ordinarily, FDA will select the reference listed drug as the reference standard. However, in some instances (e.g., where the reference listed drug has been withdrawn from sale and FDA has determined it was not withdrawn for reasons of safety or effectiveness, and FDA selects an ANDA as the reference standard), the reference listed drug and the reference standard may be different.

FDA identifies reference listed drugs in the Prescription Drug Product, OTC Drug Product, and Discontinued Drug Product Lists. Listed drugs identified as reference listed drugs represent drug products upon which an applicant can rely in seeking approval of an ANDA. FDA intends to update periodically the reference listed drugs identified in the Prescription Drug Product, OTC Drug Product, and Discontinued Drug Product Lists, as appropriate.

FDA also identifies reference standards in the Prescription Drug Product and OTC Drug Product Lists. Listed drugs identified as reference standards represent the FDA's best judgment at this time as to the appropriate comparator for purposes of conducting any in vivo bioequivalence studies required for approval.

In some instances when FDA has not designated a listed drug as a reference listed drug, such listed drug may be shielded from generic competition. If FDA has not designated a reference listed drug for a drug product the applicant intends to duplicate, the potential applicant may ask FDA to designate a reference listed drug for that drug product.

FDA may, on its own initiative, select a new reference standard when doing so will help to ensure that applications for generic drugs may be submitted and evaluated, e.g., in the event that the listed drug currently selected as the reference standard has been withdrawn from sale for other than safety and efficacy reasons.

The different abbreviated approval pathways for drug products under the FD&C Act include the abbreviated approval pathways described in section 505(j) and 505(b)(2) of the FD&C Act (21 U.S.C. 355(j) and 21 U.S.C. 23 355(b)(2), respectively).

According to the FDA (https://www.fda.gov / downloads/Drugs/GuidanceComplianceRegulatory Information/Guid ances/ UCM579751.pdf), the contents of which is incorporated herein by reference), NDAs and ANDAs can be divided into the following four categories:
(1) A "stand-alone NDA" is an application submitted under section 505(b)(1) and approved under section 505(c) of the FD&C Act that contains full reports of investigations of safety and effectiveness that were conducted by or for the applicant or for which the applicant has a right of reference or use.
(2) A 505(b)(2) application is an NDA submitted under section 505(b)(1) and approved under section 505(c) of the FD&C Act that contains full reports of investigations of safety and effectiveness, where at least some of the information required for approval comes from studies not conducted by or for the applicant and for which the applicant has not obtained a right of reference or use.
(3) An ANDA is an application for a duplicate of a previously approved drug product that was submitted and approved under section 505(j) of the FD&C Act. An ANDA relies on FDA's finding that the previously approved drug product, i.e., the reference listed drug (RLD), is safe and effective. An ANDA generally must contain information to show that the proposed generic product (a) is the same as the RLD with respect to the active ingredient(s), conditions of use, route of administration, dosage form, strength, and labeling (with certain permissible differences) and (b) is bioequivalent to the RLD. An ANDA may not be submitted if studies are necessary to establish the safety and effectiveness of the proposed product.
(4) A petitioned ANDA is a type of ANDA for a drug product that differs from the RLD in its dosage form, route of administration, strength, or active ingredient (in a product with more than one active ingredient) and for which FDA has determined, in response to a petition submitted under section 505(j)(2)(C) of the FD&C Act (suitability petition), that studies are not necessary to establish the safety and effectiveness of the proposed drug product.

A scientific premise underlying the Hatch-Waxman Amendments is that a drug product approved in an ANDA under section 505(j) of the FD&C Act is presumed to be therapeutically equivalent to its RLD. Products classified as therapeutically equivalent can be substituted with the full expectation that the substituted product will produce the same clinical effect and safety profile as the prescribed product when administered to patients under the conditions specified in the labeling. In contrast to an ANDA, a 505(b)(2) application allows greater flexibility as to the characteristics of the proposed product. A 505(b)(2) application will not necessarily be rated therapeutically equivalent to the listed drug it references upon approval.

In Europe, Applicants identify in the application form for its generic/hybrid medicinal product, which is the same as a ANDA or sNDA drug product, the reference medicinal product (product name, strength, pharmaceutical form, MAH, first authorization, Member State/Community), which is synonymous with a RLD, as follows:
1. The medicinal product that is or has been authorized in the EEA, used as the basis for demonstrating that the data protection period defined in the European pharmaceutical legislation has expired. This reference medicinal product, identified for the purpose of calculating expiry of the period of data protection, may be for a different strength, pharmaceutical form, administration route or presentation than the generic/hybrid medicinal product.
2. The medicinal product, the dossier of which is cross-referred to in the generic/hybrid application (product name, strength, pharmaceutical form, MAH, marketing authorization number). This reference medicinal product may have been authorized through separate procedures and under a different name than the reference medicinal product identified for the purpose of calculating expiry of the period of data protection. The product information of this reference medicinal product will, in principle, serve as the basis for the product information claimed for the generic/hybrid medicinal product.
3. The medicinal product (product name, strength, pharmaceutical form, MAH, Member State of source) used for the bioequivalence study(ies) (where applicable).

The term "therapeutically equivalent to a reference listed drug" means that the drug product is a generic equivalent, i.e., pharmaceutical equivalents, of the reference listed drug product and, as such, is rated an AB therapeutic equivalent to the reference listed drug product by the FDA whereby actual or potential bioequivalence problems have been resolved with adequate in vivo and/or in vitro evidence supporting bioequivalence.

"Pharmaceutical equivalents" means drug products in identical dosage forms and route(s) of administration that contain identical amounts of the identical active drug ingredient as the reference listed drug.

FDA classifies as therapeutically equivalent those products that meet the following general criteria: (1) they are approved as safe and effective; (2) they are pharmaceutical equivalents in that they (a) contain identical amounts of the same active drug ingredient in the same dosage form and route of administration, and (b) meet compendial or other applicable standards of strength, quality, purity, and identity; (3) they are bioequivalent in that (a) they do not present a known or potential bioequivalence problem, and they meet an acceptable in vitro standard, or (b) if they do present such a known or potential problem, they are shown to meet an appropriate bioequivalence standard; (4) they are adequately labeled; and (5) they are manufactured in compliance with Current Good Manufacturing Practice regulations

The term "bioequivalent" or "bioequivalence" is the absence of a significant difference in the rate and extent to which the active ingredient or active moiety in pharmaceutical equivalents or pharmaceutical alternatives becomes available at the site of drug action when administered at the same molar dose under similar conditions in an appropriately designed study. Section 505 (j)(8)(B) of the FD&C Act describes one set of conditions under which a test and reference listed drug shall be considered bioequivalent:
the rate and extent of absorption of the [test] drug do not show a significant difference from the rate and extent of absorption of the [reference] drug when administered at the same molar dose of the therapeutic ingredient under similar experimental conditions in either a single dose or multiple doses; or
the extent of absorption of the [test] drug does not show a significant difference from the extent of absorption of the [reference] drug when administered at the same molar dose of the therapeutic ingredient under similar experimental conditions in either a single dose or multiple doses and the difference from the [reference] drug in the rate of absorption of the drug is intentional, is reflected in its proposed labeling, is not essential to the attainment of effective body drug concentrations on chronic use, and is considered medically insignificant for the drug.

Where these above methods are not applicable (e.g., for drug products that are not intended to be absorbed into the bloodstream), other scientifically valid in vivo or in vitro test methods to demonstrate bioequivalence may be appropriate.

For example, bioequivalence may sometimes be demonstrated using an in vitro bioequivalence standard, especially when such an in vitro test has been correlated with human in vivo bioavailability data. In other situations, bioequivalence may sometimes be demonstrated through comparative clinical trials or pharmacodynamic studies.

The terms "sale" or "selling" means transferring a drug product, e.g., a pharmaceutical composition or an oral dosage form, from a seller to a buyer.

The term "offering for sale" means the proposal of a sale by a seller to a buyer for a drug product, e.g., a pharmaceutical composition and an oral dosage form.

Further provided herein are methods of offering for sale an approved drug product comprising erdafitinib, said method comprising offering for sale such drug product, wherein a drug product label for a reference listed drug for such drug product includes instructions for treating urothelial carcinoma. In certain embodiments, the drug product is an ANDA drug product, a supplemental New Drug Application drug product or a 505(b)(2) drug product.

Also described herein are methods comprising selling an approved drug product comprising erdafitinib, wherein the drug product label for a reference listed drug for such drug product comprises objective response rate or duration of response data. In certain embodiments, the drug product label for a reference listed drug for such drug product comprises objective response rate data. In certain embodiments, the objective response rate data is complete response rate data. In certain embodiments, the objective response rate data is partial response rate data. In certain embodiments, the objective response rate data is complete response data and partial response rate data. In certain embodiments, the objective response rate data for erdafitinib is about 40.2%, in particular, wherein the patient has locally advanced or metastatic urothelial carcinoma. In certain embodiments, the objective response rate data for erdafitinib is about 32.2%, in particular, wherein the patient has locally advanced or metastatic urothelial carcinoma which has progressed during or following at least one line of prior platinum-containing chemotherapy including within 12 months of neoadjuvant or adjuvant platinum-containing chemotherapy (chemotherapy-relapsed/refractory disease). In certain embodiments, the complete objective response rate data for erdafitinib is about 2.3%, in particular, wherein the patient has locally advanced or metastatic urothelial carcinoma which has progressed during or following at least one line of prior platinum-containing chemotherapy including within 12 months of neoadjuvant or adjuvant platinum-containing chemotherapy (chemotherapy-relapsed/refractory disease). In certain embodiments, the partial objective response rate data for erdafitinib is about 29.9%, in particular, wherein the patient has locally advanced or metastatic urothelial carcinoma which has progressed during or following at least one line of prior platinum-containing chemotherapy including within 12 months of neoadjuvant or adjuvant platinum-containing chemotherapy (chemotherapy-relapsed/refractory disease). In certain embodiments, the complete objective response rate data and the partial objective response rate data for erdafitinib is about 32.2%, in particular, wherein the patient has locally advanced or metastatic urothelial carcinoma which has progressed during or following at least one line of prior platinum-containing chemotherapy including within 12 months of neoadjuvant or adjuvant platinum-containing chemotherapy (chemotherapy-relapsed/refractory disease). In further embodiments, the duration of response data for erdafitinib is about 5.6 months, in particular, wherein the patient has locally advanced or metastatic urothelial carcinoma. In further embodiments, the duration of response data for erdafitinib is about 5.4 months, in particular, wherein the patient has locally advanced or metastatic urothelial carcinoma which has progressed during or following at least one line of prior platinum-containing chemotherapy including within 12 months of neoadjuvant or adjuvant platinum-containing chemotherapy (chemotherapy -relapsed/refractory disease).

Still further provided herein are methods of improving objective response rate or duration of response in a patient with urothelial carcinoma, said method comprising administering to said patient an approved drug product comprising erdafitinib. Also provided herein are methods of improving objective response rate in a patient with urothelial carcinoma, said method comprising administering to said patient an approved drug product comprising erdafitinib. Also provided herein are methods of improving complete objective response rate in a patient with urothelial carcinoma, said method comprising administering to said patient an approved drug product comprising erdafitinib. Also provided herein are methods of improving partial objective response rate in a patient with urothelial carcinoma, said method comprising administering to said patient an approved drug product comprising erdafitinib. In certain embodiments, the objective response rate for erdafitinib is about 40.2%, in particular, wherein the patient has locally advanced or metastatic urothelial carcinoma. In certain embodiments, the objective response rate for erdafitinib is about 32.2%, in particular, wherein the patient has locally advanced or metastatic urothelial carcinoma which has progressed during or following at least one line of prior platinum-containing chemotherapy including within 12 months of neoadjuvant or adjuvant platinum-containing chemotherapy (chemotherapy-relapsed/refractory disease). In certain embodiments, the complete objective response rate data for erdafitinib is about 2.3%, in particular, wherein the patient has locally advanced or metastatic urothelial carcinoma which has progressed during or following at least one line of prior platinum-containing chemotherapy including within 12 months of neoadjuvant or adjuvant platinum-containing chemotherapy (chemotherapy-relapsed/refractory disease). In certain embodiments, the partial objective response rate data for erdafitinib is about 29.9%, in particular, wherein the patient has locally advanced or metastatic urothelial carcinoma which has progressed during or following at least one line of prior platinum-containing chemotherapy including within 12 months of neoadjuvant or adjuvant platinum-containing chemotherapy (chemotherapy-relapsed/refractory disease). In certain embodiments, the complete objective response rate data and the partial objective response rate data for erdafitinib is about 32.2%, in particular, wherein the patient has locally advanced or metastatic urothelial carcinoma which has progressed during or following at least one line of prior platinum-containing chemotherapy including within 12 months of neoadjuvant or adjuvant platinum-containing chemotherapy (chemotherapy-relapsed/refractory disease). In further embodiments, the duration of response for erdafitinib is about 5.6 months, in particular, wherein the patient has locally advanced or metastatic urothelial carcinoma. In further embodiments, the duration of response for erdafitinib is about 5.4 months, in particular, wherein the patient has locally advanced or metastatic urothelial carcinoma which has progressed during or following at least one line of prior platinum-containing chemotherapy including within 12 months of neoadjuvant or adjuvant platinum-containing chemotherapy (chemotherapy-relapsed/refractory disease). In still further embodiments, the approved drug product is an ANDA drug product or a supplemental New Drug Application drug product.

Also provided herein are methods of improving objective response rate or duration of response in a patient with urothelial carcinoma, said method comprising providing to said patient an approved drug product comprising erdafitinib. Also provided herein are methods of improving objective response rate in a patient with urothelial carcinoma, said method comprising providing to said patient an approved drug product comprising erdafitinib. Also provided herein are methods of improving complete objective response rate in a patient with urothelial carcinoma, said method comprising providing to said patient an approved drug product comprising erdafitinib. Also provided herein are methods of improving partial objective response rate in a patient with urothelial carcinoma, said method comprising providing to said patient an approved drug product comprising erdafitinib. In certain embodiments, the objective response rate for erdafitinib is about 40.2%, in particular, wherein the patient has locally advanced or metastatic urothelial carcinoma. In certain embodiments, the objective response rate data for erdafitinib is about 32.2%, in particular, wherein the patient has locally advanced or metastatic urothelial carcinoma which has progressed during or following at least one line of prior platinum-containing chemotherapy including within 12 months of neoadjuvant or adjuvant platinum-containing chemotherapy (chemotherapy-relapsed/refractory disease). In certain embodiments, the complete objective response rate data for erdafitinib is about 2.3%, in particular, wherein the patient has locally advanced or metastatic urothelial carcinoma which has progressed during or following at least one line of prior platinum-containing chemotherapy including within 12 months of neoadjuvant or adjuvant platinum-containing chemotherapy (chemotherapy-relapsed/refractory disease). In certain embodiments, the partial objective response rate data for erdafitinib is about 29.9%, in particular, wherein the patient has locally advanced or metastatic urothelial carcinoma which has progressed during or following at least one line of prior platinum-containing chemotherapy including within 12 months of neoadjuvant or adjuvant platinum-containing chemotherapy (chemotherapy-relapsed/refractory disease). In certain embodiments, the complete objective response rate data and the partial objective response rate data for erdafitinib is about 32.2%, in particular, wherein the patient has locally advanced or metastatic urothelial carcinoma which has progressed during or following at least one line of prior platinum-containing chemotherapy including within 12 months of neoadjuvant or adjuvant platinum-containing chemotherapy (chemotherapy-relapsed/refractory disease). In further embodiments, the duration of response for erdafitinib is about 5.6 months, in particular, wherein the patient has locally advanced or metastatic urothelial carcinoma. In further embodiments, the duration of response for erdafitinib is about 5.4 months, in particular, wherein the patient has locally advanced or metastatic urothelial carcinoma which has progressed during or following at least one line of prior platinum-containing chemotherapy including within 12 months of neoadjuvant or adjuvant platinum-containing chemotherapy (chemotherapy-relapsed/refractory disease). In certain embodiments, the approved drug product is an ANDA drug product or a supplemental New Drug Application drug product. In further embodiments, the drug product label for a reference listed drug for such drug product includes instructions for treating urothelial carcinoma.

Further provided herein are approved drug products with at least one approved indication, wherein said approved drug product comprises erdafitinib. In certain embodiments, the approved drug product is a NDA drug product, an ANDA drug product, a supplemental New Drug Application drug product, or a 505(b)(2) drug product. In further embodiments, a reference listed drug product for the approved drug product includes a drug product label. In still further embodiments, the drug product label comprises objective response rate data. In still further embodiments, the drug product label comprises partial objective response rate data. In still further embodiments, the drug product label comprises complete objective response rate data. In still further embodiments, the drug product label comprises partial and complete objective response rate data. In some embodiments, the objective response rate data for erdafitinib is about 40.2%, in particular, wherein the patient has locally advanced or metastatic urothelial carcinoma. In certain embodiments, the objective response rate data for erdafitinib is about 32.2%, in particular, wherein the patient has locally advanced or metastatic urothelial carcinoma which has progressed during or following at least one line of prior platinum-containing chemotherapy including within 12 months of neoadjuvant or adjuvant platinum-containing chemotherapy (chemotherapy-relapsed/refractory disease). In certain embodiments, the complete objective response rate data for erdafitinib is about 2.3%, in particular, wherein the patient has locally advanced or metastatic urothelial carcinoma which has progressed during or following at least one line of prior platinum-containing chemotherapy including within 12 months of neoadjuvant or adjuvant platinum-containing chemotherapy (chemotherapy-relapsed/refractory disease). In certain embodiments, the partial objective response rate data for erdafitinib is about 29.9%, in particular, wherein the patient has locally advanced or metastatic urothelial carcinoma which has progressed during or following at least one line of prior platinum-containing chemotherapy including within 12 months of neoadjuvant or adjuvant platinum-containing chemotherapy (chemotherapy-relapsed/refractory disease). In certain embodiments, the complete objective response rate data and the partial objective response rate data for erdafitinib is about 32.2%, in particular, wherein the patient has locally advanced or metastatic urothelial carcinoma which has progressed during or following at least one line of prior platinum-containing chemotherapy including within 12 months of neoadjuvant or adjuvant platinum-containing chemotherapy (chemotherapy-relapsed/refractory disease). In certain embodiments, the drug product label comprises duration of response data. In further embodiments, the duration of response data for erdafitinib is about 5.6 months. In further embodiments, the duration of response data for erdafitinib is about 5.4 months, in particular, wherein the patient has locally advanced or metastatic urothelial carcinoma which has progressed during or following at least one line of prior platinum-containing chemotherapy including within 12 months of neoadjuvant or adjuvant platinum-containing chemotherapy (chemotherapy-relapsed/refractory disease).

### Nucleotide Sequences of FGFR fusion genes

The nucleotide sequences for the FGFR fusion cDNA are provided in Table 4. The underlined sequences correspond to either FGFR3 or FGFR2, the sequences in black represent the fusion partners and the sequence in *italic* fonts represent the intron sequence of the FGFR3 gene.

**Table 4**

| | |
|---|---|
| FGFR3-TACC3 v1 (2850 base pairs) (SEQ ID NO:33) | >ATGGGCGCCCCTGCCTGCGCCCTCGCGCTCTGCGTGGCCGTGGCCATCGT GGCCGGCGCCTCCTCGGAGTCCTTGGGGACGGAGCAGCGCGTCGTGGGGC GAGCGGCAGAAGTCCCGGGCCCAGAGCCCGGCCAGCAGGAGCAGTTGGT CTTCGGCAGCGGGGATGCTGTGGAGCTGAGCTGTCCCCCGCCCGGGGGTG GTCCCATGGGGCCCACTGTCTGGGTCAAGGATGGCACAGGGCTGGTGCCC TCGGAGCGTGTCCTGGTGGGGCCCCAGCGGCTGCAGGTGCTGAATGCCTC CCACGAGGACTCCGGGGCCTACAGCTGCCGGCAGCGGCTCACGCAGCGC GTACTGTGCCACTTCAGTGTGCGGGTGACAGACGCTCCATCCTCGGGAGA TGACGAAGACGGGGAGGACGAGGCTGAGGACACAGGTGTGGACACAGG GGCCCCTTACTGGACACGGCCCGAGCGGATGGACAAGAAGCTGCTGGCC GTGCCGGCCGCCAACACCGTCCGCTTCCGCTGCCCAGCCGCTGGCAACCC CACTCCCTCCATCTCCTGGCTGAAGAACGGCAGGGAGTTCCGCGGCGAGC ACCGCATTGGAGGCATCAAGCTGCGGCATCAGCAGTGGAGCCTGGTCATG GAAAGCGTGGTGCCCTCGGACCGCGGCAACTACACCTGCGTCGTGGAGA ACAAGTTTGGCAGCATCCGGCAGACGTACACGCTGGACGTGCTGGAGCGC TCCCCGCACCGGCCCATCCTGCAGGCGGGGCTGCCGGCCAACCAGACGGC GGTGCTGGGCAGCGACGTGGAGTTCCACTGCAAGGTGTACAGTGACGCAC AGCCCCACATCCAGTGGCTCAAGCACGTGGAGGTGAATGGCAGCAAGGT GGGCCCGGACGGCACACCCTACGTTACCGTGCTCAAGACGGCGGGCGCTA ACACCACCGACAAGGAGCTAGAGGTTCTCTCCTTGCACAACGTCACCTTT GAGGACGCCGGGGAGTACACCTGCCTGGCGGGCAATTCTATTGGGTTTTC TCATCACTCTGCGTGGCTGGTGGTGCTGCCAGCCGAGGAGGAGCTGGTGG |
| | AGGCTGACGAGGCGGGCAGTGTGTATGCAGGCATCCTCAGCTACGGGGT GGGCTTCTTCCTGTTCATCCTGGTGGTGGCGGCTGTGACGCTCTGCCGCCT GCGCAGCCCCCCCAAGAAAGGCCTGGGCTCCCCCACCGTGCACAAGATCT CCCGCTTCCCGCTCAAGCGACAGGTGTCCCTGGAGTCCAACGCGTCCATG AGCTCCAACACACCACTGGTGCGCATCGCAAGGCTGTCCTCAGGGGAGGG CCCCACGCTGGCCAATGTCTCCGAGCTCGAGCTGCCTGCCGACCCCAAAT GGGAGCTGTCTCGGGCCCGGCTGACCCTGGGCAAGCCCCTTGGGGAGGGC TGCTTCGGCCAGGTGGTCATGGCGGAGGCCATCGGCATTGACAAGGACCG GGCCGCCAAGCCTGTCACCGTAGCCGTGAAGATGCTGAAAGACGATGCC ACTGACAAGGACCTGTCGGACCTGGTGTCTGAGATGGAGATGATGAAGAT GATCGGGAAACACAAAAACATCATCAACCTGCTGGGCGCCTGCACGCAG GGCGGGCCCCTGTACGTGCTGGTGGAGTACGCGGCCAAGGGTAACCTGCG GGAGTTTCTGCGGGCGCGGCGGCCCCCGGGCCTGGACTACTCCTTCGACA CCTGCAAGCCGCCCGAGGAGCAGCTCACCTTCAAGGACCTGGTGTCCTGT GCCTACCAGGTGGCCCGGGGCATGGAGTACTTGGCCTCCCAGAAGTGCAT CCACAGGGACCTGGCTGCCCGCAATGTGCTGGTGACCGAGGACAACGTG ATGAAGATCGCAGACTTCGGGCTGGCCCGGGACGTGCACAACCTCGACTA CTACAAGAAGACGACCAACGGCCGGCTGCCCGTGAAGTGGATGGCGCCT GAGGCCTTGTTTGACCGAGTCTACACTCACCAGAGTGACGTCTGGTCCTTT GGGGTCCTGCTCTGGGAGATCTTCACGCTGGGGGGCTCCCCGTACCCCGG CATCCCTGTGGAGGAGCTCTTCAAGCTGCTGAAGGAGGGCCACCGCATGG ACAAGCCCGCCAACTGCACACACGACCTGTACATGATCATGCGGGAGTGC TGGCATGCCGCGCCCTCCCAGAGGCCCACCTTCAAGCAGCTGGTGGAGGA CCTGGACCGTGTCCTTACCGTGACGTCCACCGACGTAAAGGCGACACAGG AGGAGAACCGGGAGCTGAGGAGCAGGTGTGAGGAGCTCCACGGGAAGA ACCTGGAACTGGGGAAGATCATGGACAGGTTCGAAGAGGTTGTGTACCA GGCCATGGAGGAAGTTCAGAAGCAGAAGGAACTTTCCAAAGCTGAAATC CAGAAAGTTCTAAAAGAAAAAGACCAACTTACCACAGATCTGAACTCCAT GGAGAAGTCCTTCTCCGACCTCTTCAAGCGTTTTGAGAAACAGAAAGAGG TGATCGAGGGCTACCGCAAGAACGAAGAGTCACTGAAGAAGTGCGTGGA GGATTACCTGGCAAGGATCACCCAGGAGGGCCAGAGGTACCAAGCCCTG AAGGCCCACGCGGAGGAGAAGCTGCAGCTGGCAAACGAGGAGATCGCCC AGGTCCGGAGCAAGGCCCAGGCGGAAGCGTTGGCCCTCCAGGCCAGCCT GAGGAAGGAGCAGATGCGCATCCAGTCGCTGGAGAAGACAGTGGAGCAG AAGACTAAAGAGAACGAGGAGCTGACCAGGATCTGCGACGACCTCATCT CCAAGATGGAGAAGATCTGA |
| FGFR3-TACC3 v3 (2955 base pairs) (SEQ ID NO:34 | >ATGGGCGCCCCTGCCTGCGCCCTCGCGCTCTGCGTGGCCGTGGCCATCGT GGCCGGCGCCTCCTCGGAGTCCTTGGGGACGGAGCAGCGCGTCGTGGGGC GAGCGGCAGAAGTCCCGGGCCCAGAGCCCGGCCAGCAGGAGCAGTTGGT CTTCGGCAGCGGGGATGCTGTGGAGCTGAGCTGTCCCCCGCCCGGGGGTG GTCCCATGGGGCCCACTGTCTGGGTCAAGGATGGCACAGGGCTGGTGCCC TCGGAGCGTGTCCTGGTGGGGCCCCAGCGGCTGCAGGTGCTGAATGCCTC CCACGAGGACTCCGGGGCCTACAGCTGCCGGCAGCGGCTCACGCAGCGC |
| | GTACTGTGCCACTTCAGTGTGCGGGTGACAGACGCTCCATCCTCGGGAGA TGACGAAGACGGGGAGGACGAGGCTGAGGACACAGGTGTGGACACAGG GGCCCCTTACTGGACACGGCCCGAGCGGATGGACAAGAAGCTGCTGGCC GTGCCGGCCGCCAACACCGTCCGCTTCCGCTGCCCAGCCGCTGGCAACCC CACTCCCTCCATCTCCTGGCTGAAGAACGGCAGGGAGTTCCGCGGCGAGC ACCGCATTGGAGGCATCAAGCTGCGGCATCAGCAGTGGAGCCTGGTCATG GAAAGCGTGGTGCCCTCGGACCGCGGCAACTACACCTGCGTCGTGGAGA ACAAGTTTGGCAGCATCCGGCAGACGTACACGCTGGACGTGCTGGAGCGC TCCCCGCACCGGCCCATCCTGCAGGCGGGGCTGCCGGCCAACCAGACGGC GGTGCTGGGCAGCGACGTGGAGTTCCACTGCAAGGTGTACAGTGACGCAC AGCCCCACATCCAGTGGCTCAAGCACGTGGAGGTGAATGGCAGCAAGGT GGGCCCGGACGGCACACCCTACGTTACCGTGCTCAAGACGGCGGGCGCTA ACACCACCGACAAGGAGCTAGAGGTTCTCTCCTTGCACAACGTCACCTTT GAGGACGCCGGGGAGTACACCTGCCTGGCGGGCAATTCTATTGGGTTTTC TCATCACTCTGCGTGGCTGGTGGTGCTGCCAGCCGAGGAGGAGCTGGTGG AGGCTGACGAGGCGGGCAGTGTGTATGCAGGCATCCTCAGCTACGGGGT GGGCTTCTTCCTGTTCATCCTGGTGGTGGCGGCTGTGACGCTCTGCCGCCT GCGCAGCCCCCCCAAGAAAGGCCTGGGCTCCCCCACCGTGCACAAGATCT CCCGCTTCCCGCTCAAGCGACAGGTGTCCCTGGAGTCCAACGCGTCCATG AGCTCCAACACACCACTGGTGCGCATCGCAAGGCTGTCCTCAGGGGAGGG CCCCACGCTGGCCAATGTCTCCGAGCTCGAGCTGCCTGCCGACCCCAAAT GGGAGCTGTCTCGGGCCCGGCTGACCCTGGGCAAGCCCCTTGGGGAGGGC TGCTTCGGCCAGGTGGTCATGGCGGAGGCCATCGGCATTGACAAGGACCG GGCCGCCAAGCCTGTCACCGTAGCCGTGAAGATGCTGAAAGACGATGCC ACTGACAAGGACCTGTCGGACCTGGTGTCTGAGATGGAGATGATGAAGAT GATCGGGAAACACAAAAACATCATCAACCTGCTGGGCGCCTGCACGCAG GGCGGGCCCCTGTACGTGCTGGTGGAGTACGCGGCCAAGGGTAACCTGCG GGAGTTTCTGCGGGCGCGGCGGCCCCCGGGCCTGGACTACTCCTTCGACA CCTGCAAGCCGCCCGAGGAGCAGCTCACCTTCAAGGACCTGGTGTCCTGT GCCTACCAGGTGGCCCGGGGCATGGAGTACTTGGCCTCCCAGAAGTGCAT CCACAGGGACCTGGCTGCCCGCAATGTGCTGGTGACCGAGGACAACGTG ATGAAGATCGCAGACTTCGGGCTGGCCCGGGACGTGCACAACCTCGACTA CTACAAGAAGACGACCAACGGCCGGCTGCCCGTGAAGTGGATGGCGCCT GAGGCCTTGTTTGACCGAGTCTACACTCACCAGAGTGACGTCTGGTCCTTT GGGGTCCTGCTCTGGGAGATCTTCACGCTGGGGGGCTCCCCGTACCCCGG CATCCCTGTGGAGGAGCTCTTCAAGCTGCTGAAGGAGGGCCACCGCATGG ACAAGCCCGCCAACTGCACACACGACCTGTACATGATCATGCGGGAGTGC TGGCATGCCGCGCCCTCCCAGAGGCCCACCTTCAAGCAGCTGGTGGAGGA CCTGGACCGTGTCCTTACCGTGACGTCCACCGACGTGCCAGGCCCACCCC CAGGTGTTCCCGCGCCTGGGGGCCCACCCCTGTCCACCGGACCTATAGTG GACCTGCTCCAGTACAGCCAGAAGGACCTGGATGCAGTGGTAAAGGCGA CACAGGAGGAGAACCGGGAGCTGAGGAGCAGGTGTGAGGAGCTCCACGG GAAGAACCTGGAACTGGGGAAGATCATGGACAGGTTCGAAGAGGTTGTG |
| | TACCAGGCCATGGAGGAAGTTCAGAAGCAGAAGGAACTTTCCAAAGCTG AAATCCAGAAAGTTCTAAAAGAAAAAGACCAACTTACCACAGATCTGAA CTCCATGGAGAAGTCCTTCTCCGACCTCTTCAAGCGTTTTGAGAAACAGA AAGAGGTGATCGAGGGCTACCGCAAGAACGAAGAGTCACTGAAGAAGTG CGTGGAGGATTACCTGGCAAGGATCACCCAGGAGGGCCAGAGGTACCAA GCCCTGAAGGCCCACGCGGAGGAGAAGCTGCAGCTGGCAAACGAGGAGA TCGCCCAGGTCCGGAGCAAGGCCCAGGCGGAAGCGTTGGCCCTCCAGGC CAGCCTGAGGAAGGAGCAGATGCGCATCCAGTCGCTGGAGAAGACAGTG GAGCAGAAGACTAAAGAGAACGAGGAGCTGACCAGGATCTGCGACGACC TCATCTCCAAGATGGAGAAGATCTGA |
| FGFR3-BAIAP2L1 (3765 base pairs) (SEQ ID NO:35) | >ATGGGCGCCCCTGCCTGCGCCCTCGCGCTCTGCGTGGCCGTGGCCATCGT GGCCGGCGCCTCCTCGGAGTCCTTGGGGACGGAGCAGCGCGTCGTGGGGC GAGCGGCAGAAGTCCCGGGCCCAGAGCCCGGCCAGCAGGAGCAGTTGGT CTTCGGCAGCGGGGATGCTGTGGAGCTGAGCTGTCCCCCGCCCGGGGGTG GTCCCATGGGGCCCACTGTCTGGGTCAAGGATGGCACAGGGCTGGTGCCC TCGGAGCGTGTCCTGGTGGGGCCCCAGCGGCTGCAGGTGCTGAATGCCTC CCACGAGGACTCCGGGGCCTACAGCTGCCGGCAGCGGCTCACGCAGCGC GTACTGTGCCACTTCAGTGTGCGGGTGACAGACGCTCCATCCTCGGGAGA TGACGAAGACGGGGAGGACGAGGCTGAGGACACAGGTGTGGACACAGG GGCCCCTTACTGGACACGGCCCGAGCGGATGGACAAGAAGCTGCTGGCC GTGCCGGCCGCCAACACCGTCCGCTTCCGCTGCCCAGCCGCTGGCAACCC CACTCCCTCCATCTCCTGGCTGAAGAACGGCAGGGAGTTCCGCGGCGAGC ACCGCATTGGAGGCATCAAGCTGCGGCATCAGCAGTGGAGCCTGGTCATG GAAAGCGTGGTGCCCTCGGACCGCGGCAACTACACCTGCGTCGTGGAGA ACAAGTTTGGCAGCATCCGGCAGACGTACACGCTGGACGTGCTGGAGCGC TCCCCGCACCGGCCCATCCTGCAGGCGGGGCTGCCGGCCAACCAGACGGC GGTGCTGGGCAGCGACGTGGAGTTCCACTGCAAGGTGTACAGTGACGCAC AGCCCCACATCCAGTGGCTCAAGCACGTGGAGGTGAATGGCAGCAAGGT GGGCCCGGACGGCACACCCTACGTTACCGTGCTCAAGTCCTGGATCAGTG AGAGTGTGGAGGCCGACGTGCGCCTCCGCCTGGCCAATGTGTCGGAGCGG GACGGGGGCGAGTACCTCTGTCGAGCCACCAATTTCATAGGCGTGGCCGA GAAGGCCTTTTGGCTGAGCGTTCACGGGCCCCGAGCAGCCGAGGAGGAG CTGGTGGAGGCTGACGAGGCGGGCAGTGTGTATGCAGGCATCCTCAGCTA CGGGGTGGGCTTCTTCCTGTTCATCCTGGTGGTGGCGGCTGTGACGCTCTG CCGCCTGCGCAGCCCCCCCAAGAAAGGCCTGGGCTCCCCCACCGTGCACA AGATCTCCCGCTTCCCGCTCAAGCGACAGGTGTCCCTGGAGTCCAACGCG TCCATGAGCTCCAACACACCACTGGTGCGCATCGCAAGGCTGTCCTCAGG GGAGGGCCCCACGCTGGCCAATGTCTCCGAGCTCGAGCTGCCTGCCGACC CCAAATGGGAGCTGTCTCGGGCCCGGCTGACCCTGGGCAAGCCCCTTGGG GAGGGCTGCTTCGGCCAGGTGGTCATGGCGGAGGCCATCGGCATTGACAA GGACCGGGCCGCCAAGCCTGTCACCGTAGCCGTGAAGATGCTGAAAGAC GATGCCACTGACAAGGACCTGTCGGACCTGGTGTCTGAGATGGAGATGAT GAAGATGATCGGGAAACACAAAAACATCATCAACCTGCTGGGCGCCTGC |
| | ACGCAGGGCGGGCCCCTGTACGTGCTGGTGGAGTACGCGGCCAAGGGTA ACCTGCGGGAGTTTCTGCGGGCGCGGCGGCCCCCGGGCCTGGACTACTCC TTCGACACCTGCAAGCCGCCCGAGGAGCAGCTCACCTTCAAGGACCTGGT GTCCTGTGCCTACCAGGTGGCCCGGGGCATGGAGTACTTGGCCTCCCAGA AGTGCATCCACAGGGACCTGGCTGCCCGCAATGTGCTGGTGACCGAGGAC AACGTGATGAAGATCGCAGACTTCGGGCTGGCCCGGGACGTGCACAACCT CGACTACTACAAGAAGACGACCAACGGCCGGCTGCCCGTGAAGTGGATG GCGCCTGAGGCCTTGTTTGACCGAGTCTACACTCACCAGAGTGACGTCTG GTCCTTTGGGGTCCTGCTCTGGGAGATCTTCACGCTGGGGGGCTCCCCGTA CCCCGGCATCCCTGTGGAGGAGCTCTTCAAGCTGCTGAAGGAGGGCCACC GCATGGACAAGCCCGCCAACTGCACACACGACCTGTACATGATCATGCGG GAGTGCTGGCATGCCGCGCCCTCCCAGAGGCCCACCTTCAAGCAGCTGGT GGAGGACCTGGACCGTGTCCTTACCGTGACGTCCACCGACAATGTTATGG AACAGTTCAATCCTGGGCTGCGAAATTTAATAAACCTGGGGAAAAATTAT GAGAAAGCTGTAAACGCTATGATCCTGGCAGGAAAAGCCTACTACGATG GAGTGGCCAAGATCGGTGAGATTGCCACTGGGTCCCCCGTGTCAACTGAA CTGGGACATGTCCTCATAGAGATTTCAAGTACCCACAAGAAACTCAACGA GAGTCTTGATGAAAATTTTAAAAAATTCCACAAAGAGATTATCCATGAGC TGGAGAAGAAGATAGAACTTGACGTGAAATATATGAACGCAACTCTAAA AAGATACCAAACAGAACACAAGAATAAATTAGAGTCTTTGGAGAAATCC CAAGCTGAGTTGAAGAAGATCAGAAGGAAAAGCCAAGGAAGCCGAAAC GCACTCAAATATGAACACAAAGAAATTGAGTATGTGGAGACCGTTACTTC TCGTCAGAGTGAAATCCAGAAATTCATTGCAGATGGTTGCAAAGAGGCTC TGCTTGAAGAGAAGAGGCGCTTCTGCTTTCTGGTTGATAAGCACTGTGGC TTTGCAAACCACATACATTATTATCACTTACAGTCTGCAGAACTACTGAAT TCCAAGCTGCCTCGGTGGCAGGAGACCTGTGTTGATGCCATCAAAGTGCC AGAGAAAATCATGAATATGATCGAAGAAATAAAGACCCCAGCCTCTACC CCCGTGTCTGGAACTCCTCAGGCTTCACCCATGATCGAGAGAAGCAATGT GGTTAGGAAAGATTACGACACCCTTTCTAAATGCTCACCAAAGATGCCCC CCGCTCCTTCAGGCAGAGCATATACCAGTCCCTTGATCGATATGTTTAATA ACCCAGCCACGGCTGCCCCGAATTCACAAAGGGTAAATAATTCAACAGGT ACTTCCGAAGATCCCAGTTTACAGCGATCAGTTTCGGTTGCAACGGGACT GAACATGATGAAGAAGCAGAAAGTGAAGACCATCTTCCCGCACACTGCG GGCTCCAACAAGACCTTACTCAGCTTTGCACAGGGAGATGTCATCACGCT GCTCATCCCCGAGGAGAAGGATGGCTGGCTCTATGGAGAACACGACGTGT CCAAGGCGAGGGGTTGGTTCCCGTCGTCGTACACGAAGTTGCTGGAAGAA AATGAGACAGAAGCAGTGACCGTGCCCACGCCAAGCCCCACACCAGTGA GAAGCATCAGCACCGTGAACTTGTCTGAGAATAGCAGTGTTGTCATCCCC CCACCCGACTACTTGGAATGCTTGTCCATGGGGGCAGCTGCCGACAGGAG AGCAGATTCGGCCAGGACGACATCCACCTTTAAGGCCCCAGCGTCCAAGC CCGAGACCGCGGCTCCTAACGATGCCAACGGGACTGCAAAGCCGCCTTTT CTCAGCGGAGAAAACCCCTTTGCCACTGTGAAACTCCGCCCGACTGTGAC GAATGATCGCTCGGCACCCATCATTCGATGA |
| FGFR2-BICC1 (4989 base pairs) (SEQ ID NO:36) | >ATGGTCAGCTGGGGTCGTTTCATCTGCCTGGTCGTGGTCACCATGGCAAC CTTGTCCCTGGCCCGGCCCTCCTTCAGTTTAGTTGAGGATACCACATTAGA GCCAGAAGAGCCACCAACCAAATACCAAATCTCTCAACCAGAAGTGTAC GTGGCTGCGCCAGGGGAGTCGCTAGAGGTGCGCTGCCTGTTGAAAGATGC CGCCGTGATCAGTTGGACTAAGGATGGGGTGCACTTGGGGCCCAACAATA GGACAGTGCTTATTGGGGAGTACTTGCAGATAAAGGGCGCCACGCCTAGA GACTCCGGCCTCTATGCTTGTACTGCCAGTAGGACTGTAGACAGTGAAAC TTGGTACTTCATGGTGAATGTCACAGATGCCATCTCATCCGGAGATGATG AGGATGACACCGATGGTGCGGAAGATTTTGTCAGTGAGAACAGTAACAA CAAGAGAGCACCATACTGGACCAACACAGAAAAGATGGAAAAGCGGCTC CATGCTGTGCCTGCGGCCAACACTGTCAAGTTTCGCTGCCCAGCCGGGGG GAACCCAATGCCAACCATGCGGTGGCTGAAAAACGGGAAGGAGTTTAAG CAGGAGCATCGCATTGGAGGCTACAAGGTACGAAACCAGCACTGGAGCC TCATTATGGAAAGTGTGGTCCCATCTGACAAGGGAAATTATACCTGTGTA GTGGAGAATGAATACGGGTCCATCAATCACACGTACCACCTGGATGTTGT GGAGCGATCGCCTCACCGGCCCATCCTCCAAGCCGGACTGCCGGCAAATG CCTCCACAGTGGTCGGAGGAGACGTAGAGTTTGTCTGCAAGGTTTACAGT GATGCCCAGCCCCACATCCAGTGGATCAAGCACGTGGAAAAGAACGGCA GTAAATACGGGCCCGACGGGCTGCCCTACCTCAAGGTTCTCAAGGCCGCC GGTGTTAACACCACGGACAAAGAGATTGAGGTTCTCTATATTCGGAATGT AACTTTTGAGGACGCTGGGGAATATACGTGCTTGGCGGGTAATTCTATTG GGATATCCTTTCACTCTGCATGGTTGACAGTTCTGCCAGCGCCTGGAAGA GAAAAGGAGATTACAGCTTCCCCAGACTACCTGGAGATAGCCATTTACTG CATAGGGGTCTTCTTAATCGCCTGTATGGTGGTAACAGTCATCCTGTGCCG AATGAAGAACACGACCAAGAAGCCAGACTTCAGCAGCCAGCCGGCTGTG CACAAGCTGACCAAACGTATCCCCCTGCGGAGACAGGTAACAGTTTCGGC TGAGTCCAGCTCCTCCATGAACTCCAACACCCCGCTGGTGAGGATAACAA CACGCCTCTCTTCAACGGCAGACACCCCCATGCTGGCAGGGGTCTCCGAG TATGAACTTCCAGAGGACCCAAAATGGGAGTTTCCAAGAGATAAGCTGAC ACTGGGCAAGCCCCTGGGAGAAGGTTGCTTTGGGCAAGTGGTCATGGCGG AAGCAGTGGGAATTGACAAAGACAAGCCCAAGGAGGCGGTCACCGTGGC CGTGAAGATGTTGAAAGATGATGCCACAGAGAAAGACCTTTCTGATCTGG TGTCAGAGATGGAGATGATGAAGATGATTGGGAAACACAAGAATATCAT AAATCTTCTTGGAGCCTGCACACAGGATGGGCCTCTCTATGTCATAGTTG AGTATGCCTCTAAAGGCAACCTCCGAGAATACCTCCGAGCCCGGAGGCCA CCCGGGATGGAGTACTCCTATGACATTAACCGTGTTCCTGAGGAGCAGAT GACCTTCAAGGACTTGGTGTCATGCACCTACCAGCTGGCCAGAGGCATGG AGTACTTGGCTTCCCAAAAATGTATTCATCGAGATTTAGCAGCCAGAAAT GTTTTGGTAACAGAAAACAATGTGATGAAAATAGCAGACTTTGGACTCGC CAGAGATATCAACAATATAGACTATTACAAAAAGACCACCAATGGGCGG CTTCCAGTCAAGTGGATGGCTCCAGAAGCCCTGTTTGATAGAGTATACAC TCATCAGAGTGATGTCTGGTCCTTCGGGGTGTTAATGTGGGAGATCTTCAC TTTAGGGGGCTCGCCCTACCCAGGGATTCCCGTGGAGGAACTTTTTAAGC |
| | TGCTGAAGGAAGGACACAGAATGGATAAGCCAGCCAACTGCACCAACGA ACTGTACATGATGATGAGGGACTGTTGGCATGCAGTGCCCTCCCAGAGAC CAACGTTCAAGCAGTTGGTAGAAGACTTGGATCGAATTCTCACTCTCACA ACCAATGAGATCATGGAGGAAACAAATACGCAGATTGCTTGGCCATCAA AACTGAAGATCGGAGCCAAATCCAAGAAAGATCCCCATATTAAGGTTTCT GGAAAGAAAGAAGATGTTAAAGAAGCCAAGGAAATGATCATGTCTGTCT TAGACACAAAAAGCAATCGAGTCACACTGAAGATGGATGTTTCACATACA GAACATTCACATGTAATCGGCAAAGGTGGCAACAATATTAAAAAAGTGA TGGAAGAAACCGGATGCCATATCCACTTTCCAGATTCCAACAGGAATAAC CAAGCAGAAAAAAGCAACCAGGTATCTATAGCGGGACAACCAGCAGGAG TAGAATCTGCCCGAGTTAGAATTCGGGAGCTGCTTCCTTTGGTGCTGATGT TTGAGCTACCAATTGCTGGAATTCTTCAACCGGTTCCTGATCCTAATTCCC CCTCTATTCAGCATATATCACAAACGTACAATATTTCAGTATCATTTAAAC AGCGTTCCCGAATGTATGGTGCTACTGTCATAGTACGAGGGTCTCAGAAT AACACTAGTGCTGTGAAGGAAGGAACTGCCATGCTGTTAGAACATCTTGC TGGGAGCTTAGCATCAGCTATTCCTGTGAGCACACAACTAGATATTGCAG CTCAACATCATCTCTTTATGATGGGTCGAAATGGGAGCAACATCAAACAT ATCATGCAGAGAACAGGTGCTCAGATCCACTTTCCTGATCCCAGTAATCC ACAAAAGAAATCTACCGTCTACCTCCAGGGCACCATTGAGTCTGTCTGTC TTGCAAGGCAATATCTCATGGGTTGTCTTCCTCTTGTGTTGATGTTTGATA TGAAGGAAGAAATTGAAGTAGATCCACAATTCATTGCGCAGTTGATGGAA CAGCTTGATGTCTTCATCAGTATTAAACCAAAGCCCAAACAGCCAAGCAA GTCTGTGATTGTGAAAAGTGTTGAGCGAAATGCCTTAAATATGTATGAAG CAAGGAAATGTCTCCTCGGACTTGAAAGCAGTGGGGTTACCATAGCAACC AGTCCATCCCCAGCATCCTGCCCTGCCGGCCTGGCATGTCCCAGCCTGGA TATCTTAGCTTCAGCAGGCCTTGGACTCACTGGACTAGGTCTTTTGGGACC CACCACCTTATCTCTGAACACTTCAACAACCCCAAACTCACTCTTGAATGC TCTTAATAGCTCAGTCAGTCCTTTGCAAAGTCCAAGTTCTGGTACACCCAG CCCCACATTATGGGCACCCCCACTTGCTAATACTTCAAGTGCCACAGGTTT TTCTGCTATACCACACCTTATGATTCCATCTACTGCCCAAGCCACATTAAC TAATATTTTGTTGTCTGGAGTGCCCACCTATGGGCACACAGCTCCATCTCC CCCTCCTGGCTTGACTCCTGTTGATGTCCATATCAACAGTATGCAGACCGA AGGCAAAAAAATCTCTGCTGCTTTAAATGGACATGCACAGTCTCCAGATA TAAAATATGGTGCAATATCCACTTCATCACTTGGAGAAAAAGTGCTGAGT GCAAATCACGGGGATCCGTCCATCCAGACAAGTGGGTCTGAGCAGACATC TCCCAAATCAAGCCCCACTGAAGGTTGTAATGATGCTTTTGTTGAAGTAG GCATGCCTCGAAGTCCTTCCCATTCTGGGAATGCTGGTGACTTGAAACAG ATGATGTGTCCCTCCAAGGTTTCCTGTGCCAAAAGGCAGACAGTGGAACT ATTGCAAGGCACGAAAAACTCACACTTACACAGCACTGACAGGTTGCTCT CAGACCCTGAACTGAGTGCTACCGAAAGCCCTTTGGCTGACAAGAAGGCT CCAGGGAGTGAGCGCGCTGCAGAGAGGGCAGCAGCTGCCCAGCAAAACT CCGAAAGGGCCCACCTTGCTCCACGGTCATCATATGTCAACATGCAGGCA TTTGACTATGAACAGAAGAAGCTATTAGCCACCAAAGCTATGTTAAAGAA |
| | ACCAGTGGTGACGGAGGTCAGAACGCCCACAAATACCTGGAGTGGCCTG GGTTTTTCTAAATCCATGCCAGCTGAAACTATCAAGGAGTTGAGAAGGGC CAATCATGTGTCCTATAAGCCCACAATGACAACCACTTATGAGGGCTCAT CCATGTCCCTTTCACGGTCCAACAGTCGTGAGCACTTGGGAGGTGGAAGC GAATCTGATAACTGGAGAGACCGAAATGGAATTGGACCTGGAAGTCATA GTGAATTTGCAGCTTCTATTGGCAGCCCTAAGCGTAAACAAAACAAATCA ACGGAACACTATCTCAGCAGTAGCAATTACATGGACTGCATTTCCTCGCT GACAGGAAGCAATGGCTGTAACTTAAATAGCTCTTTCAAAGGTTCTGACC TCCCTGAGCTCTTCAGCAAACTGGGCCTGGGCAAATACACAGATGTTTTC CAGCAACAAGAGATCGATCTTCAGACATTCCTCACTCTCACAGATCAGGA TCTGAAGGAGCTGGGAATAACTACTTTTGGTGCCAGGAGGAAAATGCTGC TTGCAATTTCAGAACTAAATAAAAACCGAAGAAAGCTTTTTGAATCGCCA AATGCACGCACCTCTTTCCTGGAAGGTGGAGCGAGTGGAAGGCTACCCCG TCAGTATCACTCAGACATTGCTAGTGTCAGTGGCCGCTGGTAG |
| FGFR2-CASP7 (3213 base pairs) (SEQ ID NO:37) | >ATGGTCAGCTGGGGTCGTTTCATCTGCCTGGTCGTGGTCACCATGGCAAC CTTGTCCCTGGCCCGGCCCTCCTTCAGTTTAGTTGAGGATACCACATTAGA GCCAGAAGAGCCACCAACCAAATACCAAATCTCTCAACCAGAAGTGTAC GTGGCTGCGCCAGGGGAGTCGCTAGAGGTGCGCTGCCTGTTGAAAGATGC CGCCGTGATCAGTTGGACTAAGGATGGGGTGCACTTGGGGCCCAACAATA GGACAGTGCTTATTGGGGAGTACTTGCAGATAAAGGGCGCCACGCCTAGA GACTCCGGCCTCTATGCTTGTACTGCCAGTAGGACTGTAGACAGTGAAAC TTGGTACTTCATGGTGAATGTCACAGATGCCATCTCATCCGGAGATGATG AGGATGACACCGATGGTGCGGAAGATTTTGTCAGTGAGAACAGTAACAA CAAGAGAGCACCATACTGGACCAACACAGAAAAGATGGAAAAGCGGCTC CATGCTGTGCCTGCGGCCAACACTGTCAAGTTTCGCTGCCCAGCCGGGGG GAACCCAATGCCAACCATGCGGTGGCTGAAAAACGGGAAGGAGTTTAAG CAGGAGCATCGCATTGGAGGCTACAAGGTACGAAACCAGCACTGGAGCC TCATTATGGAAAGTGTGGTCCCATCTGACAAGGGAAATTATACCTGTGTA GTGGAGAATGAATACGGGTCCATCAATCACACGTACCACCTGGATGTTGT GGAGCGATCGCCTCACCGGCCCATCCTCCAAGCCGGACTGCCGGCAAATG CCTCCACAGTGGTCGGAGGAGACGTAGAGTTTGTCTGCAAGGTTTACAGT GATGCCCAGCCCCACATCCAGTGGATCAAGCACGTGGAAAAGAACGGCA GTAAATACGGGCCCGACGGGCTGCCCTACCTCAAGGTTCTCAAGGCCGCC GGTGTTAACACCACGGACAAAGAGATTGAGGTTCTCTATATTCGGAATGT AACTTTTGAGGACGCTGGGGAATATACGTGCTTGGCGGGTAATTCTATTG GGATATCCTTTCACTCTGCATGGTTGACAGTTCTGCCAGCGCCTGGAAGA GAAAAGGAGATTACAGCTTCCCCAGACTACCTGGAGATAGCCATTTACTG CATAGGGGTCTTCTTAATCGCCTGTATGGTGGTAACAGTCATCCTGTGCCG AATGAAGAACACGACCAAGAAGCCAGACTTCAGCAGCCAGCCGGCTGTG CACAAGCTGACCAAACGTATCCCCCTGCGGAGACAGGTAACAGTTTCGGC TGAGTCCAGCTCCTCCATGAACTCCAACACCCCGCTGGTGAGGATAACAA CACGCCTCTCTTCAACGGCAGACACCCCCATGCTGGCAGGGGTCTCCGAG TATGAACTTCCAGAGGACCCAAAATGGGAGTTTCCAAGAGATAAGCTGAC |
| | ACTGGGCAAGCCCCTGGGAGAAGGTTGCTTTGGGCAAGTGGTCATGGCGG AAGCAGTGGGAATTGACAAAGACAAGCCCAAGGAGGCGGTCACCGTGGC CGTGAAGATGTTGAAAGATGATGCCACAGAGAAAGACCTTTCTGATCTGG TGTCAGAGATGGAGATGATGAAGATGATTGGGAAACACAAGAATATCAT AAATCTTCTTGGAGCCTGCACACAGGATGGGCCTCTCTATGTCATAGTTG AGTATGCCTCTAAAGGCAACCTCCGAGAATACCTCCGAGCCCGGAGGCCA CCCGGGATGGAGTACTCCTATGACATTAACCGTGTTCCTGAGGAGCAGAT GACCTTCAAGGACTTGGTGTCATGCACCTACCAGCTGGCCAGAGGCATGG AGTACTTGGCTTCCCAAAAATGTATTCATCGAGATTTAGCAGCCAGAAAT GTTTTGGTAACAGAAAACAATGTGATGAAAATAGCAGACTTTGGACTCGC CAGAGATATCAACAATATAGACTATTACAAAAAGACCACCAATGGGCGG CTTCCAGTCAAGTGGATGGCTCCAGAAGCCCTGTTTGATAGAGTATACAC TCATCAGAGTGATGTCTGGTCCTTCGGGGTGTTAATGTGGGAGATCTTCAC TTTAGGGGGCTCGCCCTACCCAGGGATTCCCGTGGAGGAACTTTTTAAGC TGCTGAAGGAAGGACACAGAATGGATAAGCCAGCCAACTGCACCAACGA ACTGTACATGATGATGAGGGACTGTTGGCATGCAGTGCCCTCCCAGAGAC CAACGTTCAAGCAGTTGGTAGAAGACTTGGATCGAATTCTCACTCTCACA ACCAATGAGATGGCAGATGATCAGGGCTGTATTGAAGAGCAGGGGGTTG AGGATTCAGCAAATGAAGATTCAGTGGATGCTAAGCCAGACCGGTCCTCG TTTGTACCGTCCCTCTTCAGTAAGAAGAAGAAAAATGTCACCATGCGATC CATCAAGACCACCCGGGACCGAGTGCCTACATATCAGTACAACATGAATT TTGAAAAGCTGGGCAAATGCATCATAATAAACAACAAGAACTTTGATAA AGTGACAGGTATGGGCGTTCGAAACGGAACAGACAAAGATGCCGAGGCG CTCTTCAAGTGCTTCCGAAGCCTGGGTTTTGACGTGATTGTCTATAATGAC TGCTCTTGTGCCAAGATGCAAGATCTGCTTAAAAAAGCTTCTGAAGAGGA CCATACAAATGCCGCCTGCTTCGCCTGCATCCTCTTAAGCCATGGAGAAG AAAATGTAATTTATGGGAAAGATGGTGTCACACCAATAAAGGATTTGACA GCCCACTTTAGGGGGGATAGATGCAAAACCCTTTTAGAGAAACCCAAACT CTTCTTCATTCAGGCTTGCCGAGGGACCGAGCTTGATGATGGCATCCAGG CCGACTCGGGGCCCATCAATGACACAGATGCTAATCCTCGATACAAGATC CCAGTGGAAGCTGACTTCCTCTTCGCCTATTCCACGGTTCCAGGCTATTAC TCGTGGAGGAGCCCAGGAAGAGGCTCCTGGTTTGTGCAAGCCCTCTGCTC CATCCTGGAGGAGCACGGAAAAGACCTGGAAATCATGCAGATCCTCACC AGGGTGAATGACAGAGTTGCCAGGCACTTTGAGTCTCAGTCTGATGACCC ACACTTCCATGAGAAGAAGCAGATCCCCTGTGTGGTCTCCATGCTCACCA AGGAACTCTACTTCAGTCAATAG |

### EXAMPLES

These examples are provided for illustrative purposes only and not to limit the scope of the claims provided herein.

### EXAMPLE 1: Phase 2, multi center, open-label study (NCT02365597)

A Phase 2, multicenter, open-label study was conducted to evaluate the efficacy and safety of erdafitinib in subjects with metastatic or surgically unresectable urothelial cancer harboring select FGFR genetic alterations (FGFR translocations or mutations).

The study comprises a Screening Phase (molecular screening at any time prior to first dose and study screening within 30 days of first dose), a treatment phase, and a posttreatment follow-up phase. The treatment phase comprises the period from first dose until the end-of-treatment visit. The follow-up phase extends until the subject has died, withdraws consent, is lost to follow-up, or the end of study, whichever comes first.

Study treatment was administered on 28-day cycles. Prior to interim analysis 1, there were 2 treatment regimens. Patients were randomized 1:1 to 28-day cycles to the following 2 regimens until a regimen was selected for further study: Regimen 1 (10 mg once daily intermittent (7 days on/7 days); Regimen 2 (6 mg once daily continuous). Randomization was stratified according to performance status (0 to 1 vs. 2), hemoglobin value (<10 vs. ≥10 g per dl), *FGFR* alteration type (mutation vs. fusion), prior treatment status (chemotherapy-resistant vs. chemotherapy naive), and disease distribution (presence or absence of visceral [liver, lung, bone] metastases). Starting dose selection was based on phase 1 efficacy and tolerability.

Based on interim analysis and pharmacokinetic-pharmacodynamic modeling of serum phosphate levels, starting dose was increased to 8 mg per day continuous (Regimen 3). Thus, after interim analysis, this became a single-arm study. Dosing was further individualized through pharmacodynamically-guided uptitration to 9 mg per day in patients who did not reach target serum phosphate level (≥5.5 mg per dl was associated with improved response rate in phase 1) by day 14 and in whom no treatment-related adverse events were observed. Treatment continued until disease progression or unacceptable adverse event(s) per investigator. Patients with investigator-assessed disease progression could continue erdafitinib at the discretion of the investigator and sponsor. See **FIG. 1** for the Phase 2 study scheme.

### Objectives

### Primary Objective

- To evaluate the objective response rate (complete response [CR]+ partial response [PR]) of the selected dose regimen in subjects with metastatic or surgically unresectable urothelial cancers that harbor specific FGFR genomic alterations.

### Secondary Objectives

- To evaluate the objective response rate of the selected dose regimen in chemo-refractory subjects
- To evaluate progression-free survival (PFS), duration of response, and overall survival of the selected dose regimen in all and chemo-refractory subjects
- To evaluate the response rate in biomarker-specific subgroups (translocations versus mutations) with the selected dose regimen
- To evaluate the objective response rate, PFS, duration of response, and overall survival of the other dose regimens tested
- To evaluate the safety and pharmacokinetics of erdafitinib of all dose regimens

### Patients

Included patients were adults with measurable urothelial cancer per Response Evaluation Criteria in Solid Tumors version 1.1.

Patients were required to have at least 1 FGFR2/FGFR3 mutation or fusion per central lab testing of RNA from formalin-fixed, paraffin-embedded tumor samples, using a custom reverse transcriptase polymerase chain reaction assay.

Patients had progressed during or following at least 1 line of prior systemic chemotherapy or within 12 months of receiving neoadjuvant or adjuvant chemotherapy.

Chemotherapy-naive patients who were ineligible for cisplatin per protocol criteria were allowed. Ineligibility for cisplatin was based on impaired renal function, defined as 1) glomerular filtration rate < 60 mL/min/1.73 m² by 24-hour urine measurement; 2) calculated by the Cockcroft-Gault equation; or 3) grade 2 or higher peripheral neuropathy (Common Terminology Criteria for Adverse Events [CTCAE] version 4.0 (National Cancer Institute. CTCAE v4.0. NCI, NIH, DHHS. May 29, 2009. NIH publication # 09-7473: 2009.).

Eastern Cooperative Oncology Group (ECOG) performance status (five-point scale in which higher numbers reflect greater disability) 0-2 was required.

There was no limit on the number of prior treatment lines.

Prior immunotherapy (e.g., treatment with an immune checkpoint inhibitor) was allowed.

Patients were required to have adequate bone marrow, liver and renal (creatinine clearance ≥ 40 mL/min) function.

Patients with phosphate levels persistently above upper limit of normal despite medical management, uncontrolled cardiovascular disease, brain metastases, known hepatitis B or C, or known HIV infection were excluded.

### Assessments

Patients were assessed for efficacy per RECIST v. 1. 1 using computed tomography or magnetic resonance imaging scan of chest, abdomen, and pelvis during screening, once every 6 weeks for the first 3 months, once every 12 weeks for the next 9 months, then once every 4 to 6 months until progression. All objective responses required confirmation by an additional investigator assessment within 4 to 6 weeks of first assessment. Disease evaluations for regimen 3 were also performed by an independent radiographic review committee. Patients were contacted every 12 weeks for survival assessment.
Safety was evaluated based on clinical laboratory tests, physical exams, electrocardiograms, and ophthalmology examinations. Adverse events and abnormalities were assessed by investigator and graded per NCI CTCAE v.4.0

### End Points

The primary end point of this study is Objective Response Rate to the selected regimen (Regimen 3).

Secondary end points include progression-free survival (PFS), response duration, Overall Survival, safety, response rate in biomarker-specific subgroups, and pharmacokinetics.

### Statistical Analysis

The study was designed to enroll 180 patients with specified FGFR alterations. Of these, ≥88 were required in the selected regimen. Primary hypothesis was that objective response rate (ORR) in regimen 3 would be >25%. The study had an 85% power to reject the null hypothesis that ORR was ≤25%, with one-sided α of 0.025, given true response rate of 42%. Responses were assessed by investigators and an independent radiological review committee. Progression-free survival and overall survival were estimated using Kaplan-Meier product limit method. Data from patients who were progression free and alive or with unknown status were censored at last tumor assessment. Efficacy end points were analysed at primary analysis cut-off.

### Results

### Patients

2214 patients were assessed for eligibility. Of 210 eligible/treated patients, 33 were enrolled in regimen 1, 78 in regimen 2, and 99 in the selected phase 2 dose regimen, regimen 3.

Among patients treated with regimen 3, at the cutoff date for primary analysis and after 40 deaths, median survival follow-up time was 11.0 months (interquartile range, 0.7+ to 17.4 [95% confidence interval (CI), 9.1 to 12.2]). Median number of monthly cycles received was 5.0 (range, 1 to 18); median treatment duration was 5.3 months. In regimen 3, 41 of 99 patients were uptitrated to 9 mg per day erdafitinib; 13 patients continued treatment for at least 4 weeks beyond progression, as allowed per protocol.

Among patients treated with regimen 1 or 2, at the cutoff date for the primary analysis, the median survival follow-up time was 22.9 months in the group receiving regimen 1 (interquartile range, 1.7+ to 25.3+ [95% CI, 20.5 to 24.5]) and 18.5 months (interquartile range, 0.4+ to 21.6 [95% CI, 15.0 to 19.4) in the group receiving regimen 2. The median numbers of cycles in regimens 1 and 2 were 5.0 (range, 1 to 25) and 4.5 (range, 1 to 22), respectively. Median treatment durations were 4.4 and 3.9 months in regimens 1 and 2, respectively.

Demographic and baseline disease characteristics of patients in regimens 1 through 3 are presented in Table 5.

**Table 5: Demographic and Baseline Disease Characteristics**

| | | | **Regimen 1 10 mg intermittent dose (n = 33)** | **Regimen 2 6 mg continuous dose (n = 78)** | **Regimen 3 8 mg continuous dose (n = 99)** |
|---|---|---|---|---|---|
| Age (year), median (range) | | | 68 (53-88) | 65 (42-88) | 68 (36-87) |
| Sex | | | | | |
| | Male | | 22 (67) | 54 (69) | 76 (77) |
| | Female | | 11 (33) | 24 (31) | 23 (23) |
| ECOG performance status | | | | | |
| | 0 | | 11 (33) | 22 (28) | 50 (51) |
| | 1 | | 15 (46) | 41 (53) | 42 (42) |
| | 2 | | 7 (21) | 15 (19) | 7 (7) |
| Pretreatment | | | | | |
| | Chemotherapy-resistant^{b} | | 29 (88) | 73 (94) | 87 (88) |
| | Chemotherapy-naïve^{c} | | 4 (12) | 5 (6) | 12 (12) |
| | Prior immunotherapy | | 3 (9) | 8 (10) | 22 (22) |
| Number of lines of prior treatment | | | | | |
| | 0 | | 3 (9) | 5 (6) | 11 (11) |
| | 1 | | 13 (39) | 35 (45) | 45 (46) |
| | 2 | | 12 (36) | 24 (31) | 29 (29) |
| | 3 | | 4(12) | 12 (15) | 10 (10) |
| | > 3 | | 1 (3) | 2 (3) | 4 (4) |
| Visceral metastases | | | | | |
| | Present* | | 24 (73) | 59 (76) | 78 (79) |
| | | Bone | 6 (18) | 15 (19) | 21 (21) |
| | | Liver | 11 (33) | 25 (32) | 20 (20) |
| | | Lung | 15 (46) | 41 (53) | 57 (58) |
| | Absent | | 9 (27) | 19 (24) | 21(21) |
| Hemoglobin level | | | | | |
| | ≥10 g/dl | | 29 (88) | 62 (79) | 84 (85) |
| | <10 g/dl | | 4 (12) | 16 (21) | 15 (15) |
| Tumor Location | | | | | |
| | Upper tract | | 11 (33) | 22 (28) | 23 (23) |
| | Lower tract | | 22 (67) | 56 (72) | 76 (77) |
| Creatinine clearance rate | | | | | |
| | | < 60 mL/min | 12 (36) | 41 (53) | 52 (53) |
| | | ≥ 60 mL/min | 21 (64) | 37 (47) | 47 (47) |
| *FGFR* alterations^{d} | | | | | |
| | ***FGFR2* or *FGFR3* fusion** | | **3 (9)** | **12 (15)** | **25 (25)** |
| | | *FGFR2-BICC1* | 0 | 1 (1) | 2 (2) |
| | | *FGFR2-CASP7* | 0 | 1 (1) | 3 (3) |
| | | *FGFR3-BAIAP2L1* | 1 (3) | 1 (1) | 1 (1) |
| | | *FGFR3-TACC3 V1* | 2 (6) | 7 (9) | 11 (11) |
| | | *FGFR3-TACC3 V3* | 0 | 0 | 6 (6) |
| | | *FGFR2-BICC1*/*FGFR2-CASP7* | 0 | 1 (1) | 0 |
| | | *FGFR2-CASP7*/*FGFR3-BAIAP2L1* | 0 | 1 (1) | 0 |
| | | *FGFR2-CASP7*/*FGFR3-TACC3 V1* | 0 | 0 | 1 (1) |
| | | *FGFR2-CASP7*/*FGFR3-TACC3 V3* | 0 | 0 | 1 (1) |
| | ***FGFR3* mutation** | | **27 (82)** | **62 (80)** | **74 (75)** |
| | | *FGFR3* G370C | 7 (21) | 11 (14) | 4 (4) |
| | | *FGFR3* R248C | *5 (15)* | 14(18) | 13 (13) |
| | | *FGFR3* S249C | 8 (24) | 20 (26) | 45 (46) |
| | | *FGFR3* Y373C | 4 (12) | 15 (19) | 12 (12) |
| | | *FGFR3* G370C and *FGFR3* S249C | 1 (3) | 1 (1) | 0 |
| | | *FGFR3* R48C and *FGFR3* Y373C | 1 (3) | 1 (1) | 0 |
| | | *FGFR3* S249C and *FGFR3* Y373C | 1 (3) | 0 | 0 |
| ***FGFR2*/*3* fusions and mutations** | | | **3 (9)** | **4 (5)** | **0** |
| | | *FGFR3* G370C/*FGFR2-BICC1* | 0 | 1 (1) | 0 |
| | | *FGFR3 G370C*/*FGFR3-TACC3 V1* | 0 | 1 (1) | 0 |
| | | *FGFR3 R248C*/*FGFR3-TACC3 V1* | 1 (3) | 1 (1) | 0 |
| | | *FGFR3* S249C/*FGFR3-BAIAP2L1* | 1 (3) | 0 | 0 |
| | | *FGFR3* R248C & *S249*/*FGFR3-TACC3 V1* | 0 | 1 (1) | 0 |
| | | *FGFR3* S249C & *Y373C*/*FGFR2-* | 1(3) | 0 | 0 |
| *CASP7*/*FGFR3-BAIAP2L1*/*FGFR3-TACC3 V1*/*FGFR3*_*TACC3 V3* | | | | | |
| All values are n (%) unless noted. | | | | | |
| *Patients could have more than one visceral metastatic site. | | | | | |
| ^{b} Chemotherapy-resistant patients were those who had progressed during or following ≥ 1 line of prior systemic chemotherapy or within 12 months of adjuvant or neoadjuvant chemotherapy. | | | | | |
| ^{c} Chemotherapy-naive patients were those who were ineligible for cisplatin. Ineligibility for cisplatin was based on impaired renal function defined as 1) glomerular filtration rate < 60 mL/min/1.73 m2 by 24-hour urine measurement; 2) calculated by Cockcroft-Gault; or 3) grade 2 or higher peripheral neuropathy (CTCAE version 4.0). | | | | | |
| ^{d} Patients could have more than 1 *FGFR* alteration. | | | | | |

Across all regimens, 184 patients had received first-line platinum-based chemotherapy, 83 had received second-line chemotherapy, and 24 had received third-line chemotherapy before study enrolment. Across all regimens, the best ORRs per investigator assessment were 35% (33 of 94) for first-line gemcitabine plus cisplatin; 25% (15 of 59) for first-line gemcitabine plus carboplatin; 23% (5/ 22) for first-line methotrexate, vinblastine, doxorubicin, and cisplatin (MVAC); 17% (8/46) for second-line docetaxel, vinflunine, or paclitaxel; and 15% (3/20) for third-line docetaxel, vinflunine, or paclitaxel.

### Primary End Point

The confirmed ORR (40.4%, with a two-sided 95% CI of 30.7% to 50.1%) per investigator assessment and time to response among patients treated with regimen 3 are presented in Table 6. Because lower boundary of the confidence interval was >25%, the primary end point was achieved. An additional 39 (39%) patients had stable disease for ≥1 disease evaluation assessment (>36 days). Two patients had no postbaseline disease evaluations. ORRs were similar regardless of prior chemotherapy, number of prior treatment lines, presence of visceral metastases, or baseline characteristics such as age, sex, hemoglobin level, or renal function (Table 6, **FIG. 2****).** Seventy-five (77%) of 97 patients with ≥1postbaseline disease evaluation had reduction in sum of target lesion diameters, and 48 (49%) had maximum tumor reduction between 30% and 100% **(****FIG. 3A****).** ORR in regimen 3 per independent radiographic review was 34.3% (95% CI, 25% to 43.7%).

**Table 6: Antitumor Activity of 3 Dose Regimens of Erdafitinib**

| | | | | **Regimen 1 10 mg intermittent dose (n = 33)** | **Regimen 2 6 mg continuous dose (n = 78)** | **Regimen 3 8 mg continuous dose (n = 99)** | |
|---|---|---|---|---|---|---|---|
| | | | | | | | **(95% CI)** |
| **Patients - no.** | | | | -- | -- | 99 | |
| Response per investigator assessment* - no. (%) | | | | | | | |
| | Objective response rate | | | 7 (21) | 27 (35) | 40 (40.4) | (30.7 to 50.1) |
| | Complete response | | | 1 (3) | 3 (4) | 3 (3.0) | |
| | Partial response | | | 6 (18) | 24 (31) | 37 (37.4) | |
| | | Stable disease | | 18 (55) | 30 (39) | 39 (39.4) | |
| | | Progressive disease | | 6 (18) | 16 (21) | 18 (18.2) | |
| | | Not evaluable or unknown | | 2 (6) | 5(6) | 2 (2.0) | |
| Median time to response - mo | | | | 1.4 | 1.4 | 1.4 | |
| Median duration of response- mo | | | | 13.4 | 4.9 | 5.6 | (4.2 to 7.2) |
| Response per independent radiographic review committee - no. (%) (performed only for Regimen 3) | | | | | | | |
| | | Objective response rate | | -- | -- | 34 (34.3) | (25.0 to 43.7) |
| | | Complete response rate | | -- | -- | 3 (3.0) | |
| | | Partial response | | -- | -- | 31 (31.3) | |
| Objective response rate per investigator assessment among patient subgroups - no. (%) | | | | | | | |
| | | Chemotherapy-naïve | | 1/4 (25) | 0/5 (0) | 5/12 (41.7) | |
| | | Progressed or relapsed after chemotherapy | | 6/29 (21) | 27/73 (37) | 35/87 (40.2) | |
| | | Patients with prior anti-PD-(L) 1 inhibitor | | -- | -- | 13/22 (59.1) | |
| | | No. of lines of prior systemic therapy | | | | | |
| | | | 0 | -- | -- | 4/11 (36.4) | (7.9 to 64.8) |
| | | | 1 | -- | -- | 17/45 (37.8) | (23.6 to 51.9) |
| | | | 2 | -- | -- | 11/29 (37.9) | (20.3 to 55.6) |
| | | | 3 | -- | -- | 6/10 (60.0) | (29.6 to 90.4) |
| | | | ≥4 | -- | -- | 2/4 (50.0) | (1 to 99) |
| | | With visceral metastases | | 7/24 (29) | 19/59 (32) | 30/78 (38.5) | (27.7 to 49.3) |
| | | | Bone metastases | 1/6 (17) | 6/15 (40) | 7/22 (31.8) | (12.4 to 51.3) |
| | | | Liver metastases | 3/11 (27) | 6/25 (24) | 7/20 (35.0) | (14.1 to 55.9) |
| | | | Lung metastases | 4/15 (27) | 17/41 (41) | 23/57 (40.4) | (27.6 to 53.1) |
| | | Without visceral metastases | | 0/9 (0) | 8/19 (42) | 10/21 (47.6) | (26.3 to 69) |
| | | Lymph node metastases only | | 0/4 (0) | 6/9 (67) | 4/12 (33.3) | (6.7 to 60) |
| **Patients** - **no.** | | | | -- | -- | 99 | |
| | Upper tract disease† | | | 5/11 (46) | 5/22 (23) | 10/23 (43.5) | (23.2 to 63.7) |
| | Lower tract disease‡ | | | 2/22 (9) | 22/56 (39) | 30/76 (39.5) | (28.5 to 50.5) |
| | Dose individualization | | | | | | |
| | 8 mg non-uptitrated continuous dose regimen | | | -- | -- | 20/58 (34.5) | (22.3 to 46.7) |
| | 8 mg uptitrated to 9 mg continuous dose regimen | | | -- | -- | 20/41 (48.8) | (33.5 to 64.1) |
| | With *FGFR3* mutations | | | 6/27 (22) | 22/62 (36) | 36/74 (48.6) | (37.3 to 60.0) |
| | With *FGFR2*/*3* fusions | | | 0/3 (0) | 2/12 (17) | 4/25 (16.0) | (1.6 to 30.4) |
| * Confirmed with second scan at least 6 weeks after the initial observation of response. | | | | | | | |
| † Upper tract included renal pelvis and ureter. | | | | | | | |
| ‡ Lower tract included bladder, urethra, and prostatic urethra. | | | | | | | |

The ORR among patients treated on regimen 3 who had FGFR mutations (n=74) was 48.6% (Table 6). An additional 26 patients had stable disease for median 3.7 months (range, 0+ to 13.6 months). Responses were not affected by the particular mutation. Among 25 patients in regimen 3 with FGFR fusions, the ORR was 16.0% (Table 6). FGFR3-TACC3 V1 was the most common fusion (n=11; Table 5); and four (36.4%) of these patients responded.

In regimen 3, 22 patients received immunotherapy before study enrolment (Table 5); confirmed ORR to erdafitinib was 59% among these patients. Exploratory analysis determined that only one of these 22 (5%) patients had responded to prior immunotherapy per investigator assessment.

The ORRs for regimens 1 and 2 are also presented in Table 6.

Of the 99 patients treated with regimen 3, 87 patients had disease that had progressed on or after at least one prior chemotherapy (chemotherapy-relapsed/refractory disease) and that had at least 1 of the following gene alterations: FGFR3 gene mutations (R248C, S249C, G370C, Y373C) or FGFR gene fusions (FGFR3-TACC3, FGFR3-BAIAP2L1, FGFR2-BICC1, FGFR2-CASP7), as determined by a clinical trial assay performed at a central laboratory (Table 5). Among this population, the median age was 67 years (range: 36 to 87 years), 79% were male, and 74% were Caucasian. Most patients (92%) had a baseline Easter Cooperative Oncology Group (ECOG) performance status of 0 or 1. Three (3%) patients had disease progression following prior platinum-containing neoadjuvant or adjuvant therapy only. Eighty-four (97%) patients received at least one of cisplatin or carboplatin previously. Fifty-six percent of patients only received prior cisplatin-based regimens, 29% received only prior carboplatin-based regimens, and 10% received both cisplatin and carboplatin-based regimens. Twenty-four percent of patients had been treated with prior anti PD-L1/PD-1 therapy. Seventy-nine percent of patients had visceral metastases (bone, liver or lung).

Among the 87 chemotherapy-refractory patients in regimen 3, overall response rate as assessed by investigator was 40.2%; results for this population of patients are presented in Table 7A. Responders included patients who had previously not responded to anti PD-L1/PD-1 therapy. The ORR by FGFR alteration is presented in Table 8A.

**Table 7A: Efficacy Results for Chemotherapy-Refractory Patients in Regimen 3 (N=87)**

| **Endpoint** | | **Investigator assessment** |
|---|---|---|
| | | **N=87** |
| ORR (%) 95% CI (%) | | 40.2 (29.9, 50.5) |
| | Complete response (CR) (%) | 3.4 |
| | Partial response (PR) (%) | 36.8 |
| Median DoR (months) 95% CI (months) | | 5.55 (4.21, 7.00) |

| | | |
|---|---|---|
| ORR = CR + PR CI = Confidence Interval | | |

**Table 8A: Efficacy Results by FGFR Genetic Alteration for Chemotherapy-Refractory Patients in Regimen 3**

| | | **Investigator assessment** |
|---|---|---|
| FGFR3 Point Mutation | | N=64 |
| | ORR (%) 95% CI (%) | 48.4 (36.2, 60.7) |
| FGFR Fusion | | N=23 |
| | ORR (%) 95% CI (%) | 17.4 (1.9, 32.9) |

| | | |
|---|---|---|
| ORR = CR + PR CI = Confidence Interval | | |

Among the 87 chemotherapy-relapsed/refractory patients in regimen 3, overall response rate as assessed by blinded independent review committee was 32.2%; results for this population of patients are presented in Table 7B. Responders included patients who had previously not responded to anti PD-L1/PD-1 therapy. The ORR by FGFR alteration is presented in Table 8B.

**Table 7B: Efficacy Results for Chemotherapy-Refractory Patients in Regimen 3**

| **Endpoint** | | **BIRC^{a} assessment** |
|---|---|---|
| | | **N=87** |
| ORR (%) 95% CI (%) | | 32.2 (22.4, 42.0) |
| | Complete response (CR) (%) | 2.3 |
| | Partial response (PR) (%) | 29.9 |
| Median DoR (months) 95% CI (months) | | 5.4 (4.2, 6.9) |

| | | |
|---|---|---|
| ^{a} BIRC: Blinded Independent Review Committee ORR = CR + PR CI = Confidence Interval | | |

**Table 8B: Efficacy Results by FGFR Genetic Alteration for Chemotherapy-Refractory Patients in Regimen 3**

| | | **BIRC^{a} assessment** |
|---|---|---|
| FGFR3 Point Mutation | | N=64 |
| | ORR (%) 95% CI (%) | 40.6 (28.6, 52.7) |
| FGFR3 Fusion ^{b, c} | | N=18 |
| | ORR (%) 95% CI (%) | 11.1 (0, 25.6) |
| FGFR2 Fusion ^{c} | | N=6 |
| | ORR (%) | 0 |

| | | |
|---|---|---|
| ^{a} BIRC: Blinded Independent Review Committee ^{b} Both responders had FGFR3-TACC3_V1 fusion ^{c} One patient with a FGFR2-CASP7/FGFR3-TACC3_V3 fusion is reported in both FGFR2 fusion and FGFR3 fusion above ORR = CR + PR CI = Confidence Interval | | |

### Secondary End Points

Response duration among patients receiving regimen 3 is presented in Table 6; roughly 30% of responses were maintained for >12 months. Among 39 patients with stable disease, 13 (33%) had disease stabilization lasting >6 months (FIG. 4). Twenty-one percent of patients remained on treatment at the time of data cutoff.

Median progression-free survival per investigator assessment at median follow-up of 11.2 months in patients receiving regimen 3 is presented in **FIG. 5A****.** Progression-free survival rate (95% CI) at 12 months was 19% (11% to 29%). Median overall survival at median 11.0 months' follow-up for survival is presented in **FIG. 5B****.** Survival rate at 12 months was 55% (43% to 66%).

Among 99 patients receiving regimen 3, 34 (34%) went on to subsequent therapy, 25 (25%) of whom received one subsequent line and nine (9%) of whom received two subsequent lines. Nineteen (19%) received chemotherapy, and 15 (15%) received immune-therapy as first subsequent therapy. No patient had objective response to first subsequent chemotherapy; one patient had partial response to first subsequent immunotherapy.

Response durations for patients treated with regimens 1 and 2 are also presented in Table 6. Progression-free survival and overall survival among patients receiving regimens 1 and 2 are presented in **FIG. 6A-6B****.**

Median (95% CI) progression-free survival per investigator assessment was 4.8 (2.7 to 5.5) months and 5.3 (4.1 to 5.5) months among patients receiving regimens 1 and 2, respectively. Progression-free survival rates (95% CI) at 12 months in regimens 1 and 2 were 18% (7% to 33%) and 11% (5% to 19%), respectively. Median overall survival (95% CI) of patients receiving regimens 1 and 2 was 7.5 (6.0 to 10.7) months and 8.6 (6.5 to 9.7) months, respectively **(****FIG. 6A-6B****),** at a median follow-up for survival of 22.9 months in regimen 1 and 18.5 months in regimen 2. The overall survival rates (95% CI) at 12 months were 31% (16% to 48%) and 33% (22% to 44%) among patients in regimens 1 and 2, respectively.

### Prophylactic Measures

Prophylactic measures were taken to minimize risk of common adverse events related to FGFR inhibition. To reduce risk of hyperphosphatemia, a low-phosphate diet was recommended for all patients (600 to 800 mg of dietary phosphate intake per day). To reduce the risk of skin effects, the application of alcohol-free emollient moisturizing cream and avoidance of unnecessary exposure to sunlight, soap, perfumed products, and hot baths was recommended. Patients were asked to keep their fingers and toes clean and nails trimmed to reduce risk of nail effects.

As central serious retinopathy, a retinal disorder that is reversible upon temporary drug interruption, has been reported with kinase inhibitors and FGFR inhibitors, patients were tested at baseline and routinely monitored for this ocular adverse event with in-office Amsler grid testing and ophthalmology examination including fundoscopy and, if available, optic coherence tomography. Additional ophthalmology examinations were performed if clinically indicated.

### Safety

All patients in regimen 3 reported treatment-emergent adverse events (Table 14); 67% were grade 3 or 4. Serious treatment-emergent adverse events were reported in 39 patients (39%) (Table 10). Disease progression was the most common reason for treatment discontinuation in 62 patients (63%). Thirteen patients (13%) discontinued due to treatment-emergent-adverse events, including retinal pigment epithelium detachment, hand-foot syndrome, and dry mouth and skin/nail events (n=2 each). Fifty-five patients (56%) required dose reduction; the most common treatment-emergent adverse events leading to dose reduction were stomatitis in 16 patients (16%) and hyperphosphatemia in nine patients (9%). The safety profile allowed uptitration to 9 mg per day continuous erdafitinib in 41 patients in the 8 mg regimen who had not reached 5.5 mg per dl target serum phosphate by day 14. Among these 41 patients, 24 (59%) required ≥1dose reduction. Similar percentages of patients in the 8 mg per day continuous group who were uptitrated to 9 mg per day reported grade ≥3 treatment-emergent adverse events compared with the overall trial population (68% and 66%, respectively). Common treatment-emergent and treatment-related adverse events were similar among all regimens (Table 11 and Table 12). One patient died as a result of an adverse event (myocardial infarction considered unrelated to treatment). Treatment-related adverse events of special interest or clinical importance and their management are presented in Table 13. Seventy-six percent of central serious retinopathy events resolved; all unresolved events were grade 1 or 2.

**Table 9: Treatment-Emergent, All-causality Adverse Events Reported in ≥10% of Patients in Any Group Treated With Erdafitinib Erdafitinib**

| | **10 mg Intermittent, Regimen 1 (n=33)** | | | | **6 mg Continuous, Regimen 2 (n=78)** | | | | **8 mg Continuous, Selected Regimen 3 (n = 99)** | | | |
|---|---|---|---|---|---|---|---|---|---|---|---|---|
| **Patients with adverse events - no. (%)** | **Any grade** | **Grade 1** | **Grade 2** | **Grade ≥3** | **Any grade** | **Grade 1** | **Grade 2** | **Grade ≥3** | **Any grade** | **Grade 1** | **Grade 2** | **Grade ≥3** |
| Hyperphosphatemia | 16 (48) | 15 (46) | 1 (3) | 0 | 52 (67) | 44 (56) | 8 (10) | 0 | 76 (77) | 53 (54) | 21 (21) | 2 (2) |
| Stomatitis | 16 (48) | 9 (27) | 6 (18) | 1 (3) | 33 (42) | 13 (17) | 13 (17) | 7 (9) | 57 (58) | 21 (21) | 26 (26) | 10 (10) |
| Dry mouth | 16 (48) | 15 (46) | 1 (3) | 0 | 31 (40) | 23 (30) | 6 (8) | 2 (3) | 45 (46) | 34 (34) | 11 (11) | 0 |
| Diarrhea | 14 (42) | 7 (21) | 6 (18) | 1 (3) | 39 (50) | 24 (31) | 15 (19) | 0 | 50 (51) | 31 (31) | 15 (15) | 4 (4) |
| Decreased appetite | 11 (33) | 4 (12) | 6 (18) | 1 (3) | 29 (37) | 12 (15) | 13 (17) | 4 (5) | 38 (38) | 18 (18) | 20 (20) | 0 |
| Dysgeusia | 10 (30) | 7 (21) | 3 (9) | 0 | 10 (13) | 6 (8) | 4 (5) | 0 | 37 (37) | 23 (23) | 13 (13) | 1 (1) |
| Fatigue | 6 (18) | 4 (12) | 2 (6) | 0 | 20 (26) | 8 (10) | 8 (10) | 4 (5) | 32 (32) | 12 (12) | 18 (18) | 2 (2) |
| Dry skin | 9 (27) | 8 (24) | 1 (3) | 0 | 18 (23) | 10 (13) | 8 (10) | 0 | 32 (32) | 24 (24) | 8 (8) | 0 |
| Alopecia | 4 (12) | 2 (6) | 2 (6) | 0 | 10 (13) | 9 (12) | 1 (1) | 0 | 29 (29) | 23 (23) | 6 (6) | 0 |
| Constipation | 14 (42) | 8 (24) | 6 (18) | 0 | 20 (26) | 10 (13) | 10 (13) | 0 | 28 (28) | 19 (19) | 8 (8) | 1 (1) |
| Hand-foot syndrome | 2 (6) | 0 | 2 (6) | 0 | 13 (17) | 4 (5) | 9 (12) | 0 | 23 (23) | 6 (6) | 12 (12) | 5 (5) |
| Anemia | 8 (24) | 1 (3) | 1 (3) | 6 (18) | 13 (17) | 0 | 8 (10) | 5 (6) | 20 (20) | 9 (9) | 7 (7) | 4 (4) |
| Asthenia | 10 (30) | 5 (15) | 3 (9) | 2 (6) | 18 (23) | 5 (6) | 4 (5) | 9 (12) | 20 (20) | 2 (2) | 11 (11) | 7 (7) |
| Nausea | 5 (15) | 3 (9) | 2 (6) | 0 | 16 (21) | 11 (14) | 4 (5) | 1 (1) | 20 (20) | 13 (13) | 6 (6) | 1 (1) |
| Dry eye | 3 (9) | 2 (6) | 1 (3) | 0 | 6 (8) | 3 (4) | 2 (3) | 1 (1) | 19 (19) | 14 (14) | 4 (4) | 1 (1) |
| Abdominal pain | 5 (15) | 2 (6) | 2 (6) | 1 (3) | 14 (18) | 7 (9) | 5 (6) | 2 (3) | 8 (8) | 5 (5) | 2 (2) | 1 (1) |
| Onycholysis | 7 (21) | 3 (9) | 3 (9) | 1 (3) | 13 (17) | 2 (3) | 6 (8) | 5 (6) | 18 (18) | 6 (6) | 10 (10) | 2 (2) |
| Alanine aminotransferase increased | 1 (3) | 0 | 1 (3) | 0 | 9 (12) | 7 (9) | 2 (3) | 0 | 17 (17) | 13 (13) | 2 (2) | 2 (2) |
| Paronychia | 2 (6) | 0 | 2 (6) | 0 | 12 (15) | 2 (3) | 10 (13) | 0 | 17 (17) | 3 (3) | 11 (11) | 3 (3) |
| Vision blurred | 5 (15) | 4 (12) | 1 (3) | 0 | 5 (6) | 3 (4) | 1 (1) | 1 (1) | 17 (17) | 10 (10) | 7 (7) | 0 |
| Nail dystrophy | 2 (6) | 2 (6) | 0 | 0 | 7 (9) | 6 (8) | 1 (1) | 0 | 16 (16) | 5 (5) | 5 (5) | 6 (6) |
| Urinary tract infection | 4 (12) | 0 | 2 (6) | 2 (6) | 13 (17) | 0 | 9 (12) | 4 (5) | 16 (16) | 0 | 11 (11) | 5 (5) |
| Weight decreased | 3 (9) | 1 (3) | 2 (6) | 0 | 8 (10) | 4 (5) | 2 (3) | 2 (3) | 15 (15) | 7 (7) | 8 (8) | 0 |
| Peripheral edema | 5 (15) | 1 (3) | 4 (12) | 0 | 6 (8) | 2 (3) | 3 (4) | 1 (1) | 9 (9) | 5 (5) | 3 (3) | 1 (1) |
| Back pain | 5 (15) | 1 (3) | 1 (3) | 3 (9) | 11 (14) | 6 (8) | 3 (4) | 2 (3) | 5 (5) | 4 (4) | 1 (1) | 0 |
| Pyrexia | 5 (15) | 5 (15) | 0 | 0 | 14 (18) | 8 (10) | 3 (4) | 3 (4) | 13 (13) | 8 (8) | 5 (5) | 0 |
| Conjunctivitis | 4 (12) | 3 (9) | 1 (3) | 0 | 7 (9) | 4 (5) | 2 (3) | 1 (1) | 13 (13) | 6 (6) | 7 (7) | 0 |
| Vomiting | 9 (27) | 7 (21) | 2 (6) | 0 | 11 (14) | 9 (12) | 2 (3) | 0 | 13 (13) | 10 (10) | 1 (1) | 2 (2) |
| Hyponatremia | 2 (6) | 0 | 0 | 2 (6) | 7 (9) | 2 (3) | 0 | 5 (6) | 12 (12) | 1 (1) | 0 | 11 (11) |
| Pain in extremity | 5 (15) | 3 (9) | 1 (3) | 1 (3) | 9 (12) | 2 (3) | 6 (8) | 1 (1) | 12 (12) | 10 (10) | 2 (2) | 0 |
| Dyspepsia | 3 (9) | 2 (6) | 1 (3) | 0 | 9 (12) | 6 (8) | 3 (4) | 0 | 11 (11) | 10 (10) | 1 (1) | 0 |
| Lacrimation increased | 6 (18) | 5 (15) | 1 (3) | 0 | 13 (17) | 10 (13) | 3 (4) | 0 | 11 (11) | 8 (8) | 3 (3) | 0 |
| Nail discoloration | 1 (3) | 0 | 1 (3) | 0 | 8 (10) | 6 (8) | 2 (3) | 0 | 11 (11) | 8 (8) | 3 (3) | 0 |
| Aspartate aminotransferase increased | 2 (6) | 2 (6) | 0 | 0 | 9 (12) | 7 (9) | 2 (3) | 0 | 10 (10) | 8 (8) | 2 (2) | 0 |
| Blood creatinine increased | 4 (12) | 3 (9) | 1 (3) | 0 | 6 (8) | 3 (4) | 3 (4) | 0 | 10 (10) | 5 (5) | 5 (5) | 0 |
| Hematuria | 3 (9) | 1 (3) | 2 (6) | 0 | 6 (8) | 5 (6) | 0 | 1 (1) | 10 (10) | 7 (7) | 1 (1) | 2 (2) |
| Hypomagnesemia | 2 (6) | 2 (6) | 0 | 0 | 6 (8) | 6 (8) | 0 | 0 | 10 (10) | 9 (9) | 1 (1) | 0 |
| Insomnia | 3 (9) | 0 | 3 (9) | 0 | 8 (10) | 4 (5) | 2 (3) | 2 (3) | 7 (7) | 4 (4) | 3 (3) | 0 |
| Onychomadesis | 1 (3) | 1 (3) | 0 | 0 | 8 (10) | 2 (3) | 6 (8) | 0 | 7 (7) | 2 (2) | 5 (5) | 0 |
| Oropharyngeal pain | 0 | 0 | 0 | 0 | 8 (10) | 5 (6) | 3 (4) | 0 | 10 (10) | 8 (8) | 1 (1) | 1 (1) |
| Retinal detachment | 2 (6) | 1 (3) | 1 (3) | 0 | 8 (10) | 5 (6) | 3 (4) | 0 | 5 (5) | 3 (3) | 2 (2) | 0 |
| Dyspnea | 8 (24) | 3 (9) | 2 (6) | 3 (9) | 6 (7) | 1 (1) | 3 (4) | 2 (3) | 8 (8) | 4 (4) | 2 (2) | 2 (2) |
| Arthralgia | 7 (21) | 3 (9) | 2 (6) | 2 (6) | 8 (10) | 5 (6) | 2 (3) | 1 (1) | 8 (8) | 5 (5) | 3 (3) | 0 |

**Table 10: Serious Treatment-Emergent Adverse Events Reported in ≥2% of Patients**

| **Patients With Serious Treatment-Emergent Adverse Events - no. (%)** | | **10 mg Intermittent, Regimen 1 (n=33)** | **6 mg Continuous, Regimen 2 (n=78)** | **8 mg Continuous, Selected Regimen 3 (n = 99)** |
|---|---|---|---|---|
| **Total number of patients with serious treatment-emergent adverse events** | | 14 (42) | 39 (50) | 39 (39) |
| Infections and infestations | | 2 (6) | 13 (17) | 9 (9) |
| | Urinary tract infection | 0 | 4 (5) | 3 (3) |
| | Urosepsis | 0 | 3 (4) | 2 (2) |
| Gastrointestinal disorders | | 2 (6) | 8 (10) | 8 (8) |
| General disorders/administration site conditions | | 1 (3) | 7 (9) | 8 (8) |
| | General physical health deterioration | 1 (3) | 2 (3) | 3 (3) |
| Renal and urinary disorders | | 1 (3) | 5 (6) | 10 (10) |
| Eye disorders | | 1 (3) | 3 (4) | 9 (9) |
| Respiratory, thoracic, mediastinal disorders | | 4 (12) | 3 (4) | 3 (3) |
| | Dyspnea | 2 (6) | 1 (1) | 2 (2) |
| Metabolism and nutrition disorders | | 1 (3) | 3 (4) | 2 (2) |
| Musculoskeletal and connective tissue disorders | | 2 (6) | 4 (5) | 0 |
| Nervous system disorders | | 0 | 5 (6) | 1 (1) |

**Table 11: Treatment-Related Adverse Events Reported in ≥10% of Patients Treated with 8 mg per day Continuous Erdafitinib**

| | **8 mg Continuous Erdafitinib (n = 99)** | | | |
|---|---|---|---|---|
| **Patients with Adverse Events** - **no.** (%) | **Any Grade** | **Grade 1** | **Grade 2** | **Grade 3** |
| Hyperphosphatemia | 72 (73) | 49 (50) | 21 (21) | 2 (2) |
| Stomatitis | 54 (55) | 19 (19) | 26 (26) | 9 (9) |
| Dry mouth | 43 (43) | 32 (32) | 11 (11) | 0 |
| Diarrhea | 37 (37) | 21 (21) | 12 (12) | 4 (4) |
| Dysgeusia | 35 (35) | 22 (22) | 12 (12) | 1 (1) |
| Dry skin | 32 (32) | 24 (24) | 8 (8) | 0 |
| Alopecia | 27 (27) | 21 (21) | 6 (6) | 0 |
| Decreased appetite | 25 (25) | 11 (11) | 14 (14) | 0 |
| Hand-foot syndrome | 22 (22) | 5 (5) | 12 (12) | 5 (5) |
| Fatigue | 21 (21) | 8 (8) | 11 (11) | 2 (2) |
| Dry eye | 19 (19) | 14 (14) | 4 (4) | 1 (1) |
| Nail dystrophy | 16 (16) | 5 (5) | 5 (5) | 6 (6) |
| Onycholysis | 16 (16) | 4 (4) | 10 (10) | 2(2) |
| Vision blurred | 16 (16) | 10 (10) | 6 (6) | 0 |
| Paronychia | 14 (14) | 1 (1) | 10 (10) | 3 (3) |
| Asthenia | 13 (13) | 2 (2) | 9 (9) | 2 (2) |
| Alanine aminotransferase increased | 12 (12) | 9 (9) | 2 (2) | 1 (1) |
| Lacrimation increased | 11 (11) | 8 (8) | 3 (3) | 0 |
| Nail discoloration | 11 (11) | 8 (8) | 3 (3) | 0 |
| Weight decreased | 10 (10) | 5 (5) | 5 (5) | 0 |

**Table 12: Treatment-Related Adverse Events Reported in ≥10% of Patients Treated With 10 mg Intermittent and 6 mg per Day Continuous Erdafitinib**

| | **10 mg Intermittent, Regimen 1 (n=33)** | | | | **6 mg Continuous, Regimen 2 (n=78)** | | | |
|---|---|---|---|---|---|---|---|---|
| **Patients with adverse events - no. (%)** | **Any grade** | **Grade 1** | **Grade 2** | **Grade ≥3** | **Any grade** | **Grade 1** | **Grade 2** | **Grade ≥3** |
| Hyperphosph atemia | 15 (46) | 14 (42) | 1 (3) | 0 | 49 (63) | 41 (53) | 8 (10) | 0 |
| Stomatitis | 16 (49) | 9 (27) | 6 (18) | 1 (3) | 33 (42) | 13 (17) | 13 (17) | 7 (9) |
| Dry mouth | 14 (42) | 13 (39) | 1 (3) | 0 | 31 (40) | 23 (30) | 6 (8) | 2 (3) |
| Diarrhea | 13 (39) | 7 (21) | 5 (15) | 1 (3) | 29 (37) | 16 (21) | 13 (17) | 0 |
| Dysgeusia | 10 (30) | 7 (21) | 3 (9) | 0 | 10 (13) | 6 (8) | 4 (5) | 0 |
| Dry skin | 8 (24) | 7 (21) | 1 (3) | 0 | 16 (21) | 8 (10) | 8 (10) | 0 |
| Decreased appetite | 6 (18) | 2 (6) | 4 (12) | 0 | 18 (23) | 7 (9) | 9 (12) | 2 (3) |
| Onycholysis | 6 (18) | 2 (6) | 3 (9) | 1 (3) | 13 (17) | 2 (3) | 6 (8) | 5 (6) |
| Hand-foot syndrome | 2 (6) | 0 | 2 (6) | 0 | 12 (15) | 4 (5) | 8 (10) | 0 |
| Fatigue | 4 (12) | 2 (6) | 2 (6) | 0 | 12 (15) | 5 (6) | 6 (8) | 1 (1) |
| Lacrimation increased | 4 (12) | 4 (12) | 0 | 0 | 12 (15) | 9 (12) | 3 (4) | 0 |
| Nausea | 5 (15) | 3 (9) | 2 (6) | 0 | 6 (8) | 4 (5) | 2 (3) | 0 |
| Vision blurred | 5 (15) | 4 (12) | 1 (3) | 0 | 5 (6) | 3 (4) | 1 (1) | 1 (1) |
| Asthenia | 6 (18) | 2 (6) | 2 (6) | 2 (6) | 11 (14) | 3 (4) | 4 (5) | 4 (5) |
| Paronychia | 2 (6) | 0 | 2 (6) | 0 | 11 (14) | 1 (1) | 10 (13) | 0 |
| Conjunctivitis | 4 (12) | 3 (9) | 1 (3) | 0 | 2 (3) | 2 (3) | 0 | 0 |
| Alopecia | 3 (9) | 1 (3) | 2 (6) | 0 | 8 (10) | 8 (10) | 0 | 0 |
| Nail discoloration | 1 (3) | 0 | 1 (3) | 0 | 8 (10) | 6 (8) | 2 (3) | 0 |
| Onychomade sis | 1 (3) | 1 (3) | 0 | 0 | 8 (10) | 2 (3) | 6 (8) | 0 |
| Retinal detachment | 2 (6) | 1 (3) | 1 (3) | 0 | 8 (10) | 5 (6) | 3 (4) | 0 |

**Table 13. Treatment-related Adverse Events of Special Interest or Clinical Importance Among Patients Treated With 8 mg per day Continuous Erdafitinib (Regimen 3).**

| | | **8 mg Continuous Erdafitinib (n=99)** | |
|---|---|---|---|
| **Patients with adverse events - no. (%)** | | **Any grade** | **Grade ≥3** |
| Hyperphosphatemia | | 72 (73) | 2 (2) |
| Skin events | | 48 (49) | 6 (6) |
| | Dry skin | 32 (32) | 0 (0) |
| | Hand-foot syndrome | 22 (22) | 5 (5) |
| Nail events | | 51 (52) | 14 (14) |
| | Onycholysis | 16 (16) | 2 (2) |
| | Paronychia | 14 (14) | 3 (3) |
| | Nail dystrophy | 16 (16) | 6 (6) |
| Central serous retinopathy* | | 21 (21) | 3 (3) |
| Ocular events other than central serous retinopathy^{†} | | 51 (52) | 5 (5) |
| Arrhythmia-related events | | 0 | 0 |

| | | | |
|---|---|---|---|
| * Central serous retinopathy was an adverse event of special interest grouped term including the following individual preferred terms: retinal detachment, vitreous detachment, retinal edema, retinopathy, chorioretinopathy, detachment of retinal pigment epithelium, and detachment of macular retinal pigment epithelium. ^{†} Most common ocular events other than central serous retinopathy included dry eye (19%), blurry vision (16%), increased lacrimation (11%), and conjunctivitis (9%). | | | |

**Table 14. Treatment-Emergent, All-causality Adverse Events Reported in >15% of Patients or Grade ≥3 in More Than 1 Patient Treated With 8 mg Continuous Erdafitinib (Regimen 3).**

| | **8 mg Continuous, Selected** | | | |
|---|---|---|---|---|
| | **Regimen 3** | | | |
| | **(n = 99)** | | | |
| **Patients with adverse events - no. (%)** | **Any grade** | **Grade 1** | **Grade 2** | **Grade ≥3** |
| Hyperphosphatemia | 76 (77) | 53 (54) | 21 (21) | 2 (2) |
| Stomatitis | 57 (58) | 21 (21) | 26 (26) | 10 (10) |
| Dry mouth | 45 (46) | 34 (34) | 11 (11) | 0 |
| Diarrhea | 50 (51) | 31 (31) | 15 (15) | 4 (4) |
| Decreased appetite | 38 (38) | 18 (18) | 20 (20) | 0 |
| Dysgeusia | 37 (37) | 23 (23) | 13 (13) | 1 (1) |
| Fatigue | 32 (32) | 12 (12) | 18 (18) | 2 (2) |
| Dry skin | 32 (32) | 24 (24) | 8 (8) | 0 |
| Alopecia | 29 (29) | 23 (23) | 6 (6) | 0 |
| Constipation | 28 (28) | 19 (19) | 8 (8) | 1 (1) |
| Hand-foot syndrome | 23 (23) | 6 (6) | 12 (12) | 5 (5) |
| Anemia | 20 (20) | 9 (9) | 7 (7) | 4 (4) |
| Asthenia | 20 (20) | 2 (2) | 11 (11) | 7 (7) |
| Nausea | 20 (20) | 13 (13) | 6 (6) | 1 (1) |
| Dry eye | 19 (19) | 14 (14) | 4 (4) | 1 (1) |
| Abdominal pain | 8 (8) | 5 (5) | 2 (2) | 1 (1) |
| Onycholysis | 18 (18) | 6 (6) | 10 (10) | 2 (2) |
| Alanine aminotransferase increased | 17 (17) | 13 (13) | 2 (2) | 2 (2) |
| Paronychia | 17 (17) | 3 (3) | 11 (11) | 3 (3) |
| Vision blurred | 17 (17) | 10 (10) | 7 (7) | 0 |
| Nail dystrophy | 16 (16) | 5 (5) | 5 (5) | 6 (6) |
| Urinary tract infection | 16 (16) | 0 | 11 (11) | 5 (5) |

Treatment-related adverse events that were considered of special interest/clinical importance were hyperphosphatemia, skin effects, nail effects, and eye disorders, including central serous retinopathy (CSR) and other non-CSR ocular events (Table 13). Treatment-related hyperphosphatemia and effects on the skin and on the nails were reported in 73%, 49%, and 52%, respectively, of patients treated with 8 mg per day continuous erdafitinib. Most events were mild to moderate. In this group, the most common treatment-related effects on the skin were dry skin (32%) and hand-foot syndrome (22%), and the most common treatment-related nail effects were nail dystrophy and onycholysis in 16% of patients each. Overall, 63% of patients treated with 8 mg per day continuous erdafitinib and 54% of patients overall experienced some type of eye disorder, regardless of whether it was deemed related to treatment. Among patients with eye disorders (n=62), most (n=52, 84%) experienced grade 1 or 2 events. Twenty-one patients (21%) who received 8 mg per day continuous erdafitinib had treatment-related CSR, a preferred term that included chorioretinopathy, retinal detachment, and detachment of retinal pigment epithelium; only three of these patients (3%) had grade ≥3 events. Most patients with CSR events were able to continue treatment after management through dose interruption or reduction. CSR led to discontinuation in three patients; no patient had retinal vein or artery occlusion.

### Management of Adverse Events

Hyperphosphatemia, the most common treatment-related adverse event (Table 11, 12 and 14), was managed by dose interruption (23%), dose reduction (9%), and treatment with phosphate binders when medically warranted. Phosphate elevation typically peaked 6 weeks after erdafitinib initiation and normalized by cycle 5. One patient discontinued treatment due to grade 1 hyperphosphatemia. Dry skin was managed with additional topical ointments such as ammonium lactate, salicylic acid, or zinc oxide creams. Nail effects were managed with topical nail strengthener, and antibiotics or silver nitrate were applied in severe cases.

### Discussion

This study met its primary objective, with a 40% confirmed ORR after treatment with 8 mg per day continuous erdafitinib, demonstrating antitumor activity in patients with locally advanced and unresectable/ metastatic urothelial carcinoma who have certain FGFR genetic alterations compared with currently available treatment options. Responses to erdafitinib were rapid and independent of the number of prior lines and types of therapy, presence of visceral metastases, or tumor location.

Importantly, median progression-free and overall survival were 5.5 months (Fig. 5A) and 13.8 months (Fig. 5B), respectively, including patients with visceral metastases and poor kidney function who had progressed on or after multiple lines of therapy. As allowed by protocol, 13 patients continued treatment beyond progression, which was either limited progression in a target lesion or appearance of a small new lesion while the patient was assessed to have ongoing clinical benefit. The safety profile allowed 8 mg continuous daily dosing, with uptitration to 9 mg daily dosing guided by serum phosphate levels. Uptitration did not increase adverse event severity, as percentages of grade ≥3 events were similar across both groups. Hyperphosphatemia, a known class effect of FGFR inhibitors, was reported in 77% (regimen 3) and was typically manageable and reversible. Ocular events such as central serous retinopathy are known class effects of inhibitors of the mitogen-activated protein kinase pathway. Although ocular adverse events were common with erdafitinib treatment, these were mostly mild to moderate and resolved with dose interruption or reduction.

Patients with FGFR mutations or fusions may be less likely to respond to immunotherapy. In our study, only 1 of 22 (5%) patients had responded to prior immunotherapy, and 59% of those patients responded to erdafitinib after failure of immunotherapy. This observation was also noted in a study of rogaratinib in which nine of 10 patients (90%) had disease progression with prior immunotherapy, and 30% responded to rogaratinib

These results indicate that the pan-FGFR inhibitor erdafitinib had measurable benefit in patients with advance urothelial carcinoma with *FGFR* alterations.

### EXAMPLE 2: Pharmacodynamics and Pharmacokinetics

### Pharmacodynamics

### Cardiac Electrophysiology

Based on evaluation of QTc interval in an open-label, dose escalation and dose expansion study in 187 patients with cancer, erdafitinib had no large effect (i.e., > 20 ms) on the QTc interval.

### Serum Phosphate

Erdafitinib increased serum phosphate level as a consequence of FGFR inhibition. Erdafitinib should be increased to the maximum recommended dose to achieve target serum phosphate levels of 5.5-7.0 mg/dL in early cycles with continuous daily dosing

In erdafitinib clinical trials, the use of drugs which can increase serum phosphate levels, such as potassium phosphate supplements, vitamin D supplements, antacids, phosphate-containing enemas or laxatives, and medications known to have phosphate as an excipient were prohibited unless no alternatives exist. To manage phosphate elevation, phosphate binders were permitted. Avoid concomitant use with agents that can alter serum phosphate levels before the initial dose increase period based on serum phosphate levels.

### Pharmacokinetics

Following administration of 8 mg once daily, the mean (coefficient of variation [CV%]) erdafitinib steady-state maximum observed plasma concentration (Cmax), area under the curve (AUCtau), and minimum observed plasma concentration (Cmin) were 1399 ng/mL (51%), 29268 ng·h/mL (60%), and 936 ng/mL (65%), respectively.

Following single and repeat once daily dosing, erdafitinib exposure (maximum observed plasma concentration [Cmax] and area under the plasma concentration time curve [AUC]) increased proportionally across the dose range of 0.5 to 12 mg (0.06 to 1.3 times the maximum approved recommended dose). Steady state was achieved after 2 weeks with once daily dosing and the mean accumulation ratio was 4-fold.

### Absorption

Median time to achieve peak plasma concentration (tmax) was 2.5 hours (range: 2 to 6 hours).

### Effect of Food

No clinically meaningful differences with erdafitinib pharmacokinetics were observed following administration of a high-fat and high-calorie meal (800 calories to 1,000 calories with approximately 50% of total caloric content of the meal from fat) in healthy subjects.

### Distribution

The mean apparent volume of distribution of erdafitinib was 29 L in patients. Erdafitinib protein binding was 99.8% in patients, primarily to alpha-1-acid glycoprotein.

### Elimination

The mean total apparent clearance (CL/F) of erdafitinib was 0.362 L/h in patients. The mean effective half-life of erdafitinib was 59 hours in patients.

### Metabolism

Erdafitinib is primarily metabolized by CYP2C9 and CYP3A4. The contribution of CYP2C9 and CYP3A4 in the total clearance of erdafitinib is estimated to be 39% and 20% respectively. Unchanged erdafitinib was the major drug-related moiety in plasma, there were no circulating metabolites.

### Excretion

Following a single oral dose of radiolabeled erdafitinib, approximately 69% of the dose was recovered in feces (19% as unchanged) and 19% in urine (13% as unchanged).

### Specific Populations

No clinically meaningful trends in the pharmacokinetics of erdafitinib were observed based on age (21-88 years), sex, race, body weight (36-132 kg), mild (eGFR [estimated glomerular filtration rate, using modification of diet in renal disease equation] 60 to 89 mL/min/1.73 m²) or moderate (eGFR 30-59 mL/min/1.73 m²) renal impairment or mild hepatic impairment (total bilirubin ≤ ULN and AST > ULN, or total bilirubin > 1.0 - 1.5 x ULN and any AST).

The pharmacokinetics of erdafitinib in patients with severe renal impairment, renal impairment requiring dialysis, moderate or severe hepatic impairment is unknown.

### EXAMPLE 3: Drug Interactions

### Effect of Other Drugs on Erdafitinib

1. Strong CYP2C9 or CYP3A4 Inhibitors
   Clinical Impact
      - Co-administration of erdafitinib with strong inhibitors of CYP2C9 or CYP3A4 increased erdafitinib plasma concentrations;
      - Increased erdafitinib plasma concentrations may lead to increased drug-related toxicity.
   Clinical Management
      - Consider alternative therapies that are not strong inhibitors of CYP2C9 or CYP3A4 during treatment with erdafitinib.
      - If co-administration of a strong inhibitor of CYP29 or CYP3A4 is unavoidable, monitor closely for adverse reactions and consider dose modifications accordingly. If the strong inhibitor is discontinued, the erdafitinib dose may be increased in the absence of drug-related toxicity.
2. Strong CYP2C9 or CYP3A4 Inducers
   Clinical Impact
   - Co-administration of erdafitinib with strong inducers of CYP2C9 or CYP3A4 may decrease erdafitinib plasma concentrations significantly.
   - Decreased erdafitinib plasma concentrations may lead to decreased activity. Clinical Management
   - Avoid co-administration of strong inducers of CYP2C9 or CYP3A4 with erdafitinib.
3. Moderate CYP2C9 or CYP3A4 Inducers
   Clinical Impact
   - Co-administration of erdafitinib with moderate inducers of CYP2C9 or CYP3A4 may decrease erdafitinib plasma concentrations.
   - Decreased erdafitinib plasma concentrations may lead to decreased activity. Clinical Management
   - If a moderate CYP2C9 or CYP3A4 inducer must be co-administered at the start of erdafitinib treatment, administer erdafitinib dose as recommended (8 mg once daily with potential to increase to 9 mg once daily based on serum phosphate levels on Days 14 to 21 and tolerability).
   - If a moderate CYP2C9 or CYP3A4 inducer must be co-administered after the initial dose increase period based on serum phosphate levels and tolerability, increase erdafitinib dose up to 9 mg.
   - When a moderate inducer of CYP2C9 or CYP3A4 is discontinued, continue erdafitinib at the same dose, in the absence of drug-related toxicity.
4. Serum Phosphate Level-Altering Agents
   Clinical Impact
      - Co-administration of erdafitinib with other serum phosphate level-altering agents may increase or decrease serum phosphate levels.
      - Changes in serum phosphate levels due to serum phosphate level-altering agents (other than erdafitinib) may interfere with serum phosphate levels needed for the determination of initial dose increased based on serum phosphate levels.
   Clinical Management
      - Avoid co-administration of serum phosphate level-altering agents with erdafitinib before initial dose increase period based on serum phosphate levels (Days 14 to 21).

### Effect of Erdafitinib on Other Drugs

1. CYP3A4 Substrates
   Clinical Impact
      - Co-administration of erdafitinib with CYP3A4 substrates may alter the plasma concentrations of CYP3A4 substrates
      - Altered plasma concentrations of CYP3A4 substrates may lead to loss of activity or increased toxicity of the CYP3A4 substrates.
   Clinical Management
   • Avoid co-administration of erdafitinib with sensitive substrates of CYP3A4 with narrow therapeutic indices.
2. OCT2 Substrates
   Clinical Impact

   - Co-administration of erdafitinib with OCT2 substrates may increase the plasma concentrations of OCT2 substrates;
   - Increased plasma concentrations of OCT2 substrates may lead to increased toxicity of the OCT2 substrates.
      Clinical Management
   - Consider alternative therapies that are not OCT2 substrates or consider reducing the dose of OCT2 substrates (e.g., metformin) based on tolerability.
3. P-glycoprotein (P-gp) Substrates
   Clinical Impact
      - Co-administration of erdafitinib with P-gp substrates may increase the plasma concentrations of P-gp substrates;
      - Increased plasma concentrations of P-gp substrates may lead to increased toxicity of the P-gp substrates.
   Clinical Management
      - If co-administration of erdafitinib with P-gp substrates is unavoidable, separate erdafitinib administration by at least 6 hours before or after administration of P-gp substrates with narrow therapeutic index.

### EXAMPLE 4: Final FDA Approved Drug Product Label

A pharmaceutical product comprising erdafitinib for the treatment of adult patients with locally advanced or metastatic urothelial carcinoma that has
- susceptible FGFR3 or FGFR2 genetic alterations and
- progressed during or following at least one line of prior platinum containing chemotherapy including within 12 months of neoadjuvant or adjuvant platinum-containing chemotherapy
according to the following label:

HIGHLIGHTS OF PRESCRIBING INFORMATION
These highlights do not include all the information needed to use
BALVERSA™ safely and effectively. See full prescribing information for
BALVERSA.

BALVERSA (erdafitinib) tablets, for oral use
Initial U.S. Approval: 2019

----------------------------INDICATIONS AND USAGE----------------------------
BALVERSA is a kinase inhibitor indicated for the treatment of adult patients
with locally advanced or metastatic urothelial carcinoma that has
• susceptible FGFR3 or FGFR2 genetic alterations and
• progressed during or following at least one line of prior platinum-
containing chemotherapy including within 12 months of neoadjuvant or
adjuvant platinum-containing chemotherapy.

Select patients for therapy based on an FDA-approved companion diagnostic
for BALVERSA. (1, 2.1)

This indication is approved under accelerated approval based on tumor
response rate. Continued approval for this indication may be contingent upon
verification and description of clinical benefit in confirmatory trials. (1, 14)

------------------------DOSAGE AND ADMINISTRATION------------------------
• Confirm the presence of FGFR genetic alterations in tumor specimens
prior to initiation of treatment with BALVERSA. (2.1)
• Recommended initial dosage: 8 mg orally once daily with a dose
increase to 9 mg daily if criteria are met. (2.2)
• Swallow whole with or without food. (2.2)

----------------------DOSAGE FORMS AND STRENGTHS----------------------
Tablets: 3 mg, 4 mg, and 5 mg. (3)

-----------------------------CONTRAINDICATIONS-----------------------------
None. (4)

-------------------------WARNINGS AND PRECAUTIONS-------------------------
• Ocular disorders: BALVERSA can cause central serous
retinopathy/retinal pigment epithelial detachment (CSR/RPED). Perform
monthly ophthalmological examinations during the first four months of
treatment, every 3 months afterwards, and at any time for visual
symptoms. Withhold BALVERSA when CSR/RPED occurs and
permanently discontinue if it does not resolve within 4 weeks or if Grade
4 in severity. (2.3, 5.1)
• Hyperphosphatemia: Increases in phosphate levels are a
pharmacodynamic effect of BALVERSA. Monitor for
hyperphosphatemia and manage with dose modifications when required.
(2.3, 5.2)
• Embryo-fetal toxicity: Can cause fetal harm. Advise patients of the
potential risk to the fetus and to use effective contraception (5.3, 8.1,
8.3).

-----------------------------ADVERSE REACTIONS-----------------------------
The most common adverse reactions including laboratory abnormalities
(≥20%) were phosphate increased, stomatitis, fatigue, creatinine increased,
diarrhea, dry mouth, onycholysis, alanine aminotransferase increased, alkaline
phosphatase increased, sodium decreased, decreased appetite, albumin
decreased, dysgeusia, hemoglobin decreased, dry skin, aspartate
aminotransferase increased, magnesium decreased, dry eye, alopecia, palmar-
plantar erythrodysesthesia syndrome, constipation, phosphate decreased,
abdominal pain, calcium increased, nausea, and musculoskeletal pain. (6.1)

To report SUSPECTED ADVERSE REACTIONS, contact Janssen
Products, LP. at 1-800-526-7736 (1-800-JANSSEN and
www.BALVERSA.com) or FDA at 1-800-FDA-1088 or
www.fda.gov/medwatch.

--------------------------------DRUG INTERACTIONS--------------------------------
• Strong CYP2C9 or CYP3A4 inhibitors: Consider alternative agents or
monitor closely for adverse reactions. (7.1)
• Strong CYP2C9 or CYP3A4 inducers: Avoid concomitant use with
BALVERSA. (7.1)
• Moderate CYP2C9 or CYP3A4 inducers: Increase BALVERSA dose up
to 9 mg. (7.1)
• Serum phosphate level-altering agents: Avoid concomitant use with
agents that can alter serum phosphate levels before the initial dose
modification period. (2.3, 7.1)
• CYP3A4 substrates: Avoid concomitant use with sensitive CYP3A4
substrates with narrow therapeutic indices. (7.2)
• OCT2 substrates: Consider alternative agents or consider reducing the
dose of OCT2 substrates based on tolerability. (7.2)
• P-gp substrates: Separate BALVERSA administration by at least 6 hours
before or after administration of P-gp substrates with narrow therapeutic
indices. (7.2)

-----------------------USE IN SPECIFIC POPULATIONS-----------------------
• Lactation: Advise not to breastfeed. (8.2)

See 17 for PATIENT COUNSELING INFORMATION and FDA-
approved patient labeling.

Revised: 04/2019

FULL PRESCRIBING INFORMATION: CONTENTS*

1 INDICATIONS AND USAGE
2 DOSAGE AND ADMINISTRATION
2.1 Patient Selection
2.2 Recommended Dosage and Schedule
2.3 Dose Modifications for Adverse Reactions
3 DOSAGE FORMS AND STRENGTHS
4 CONTRAINDICATIONS
5 WARNINGS AND PRECAUTIONS
5.1 Ocular Disorders
5.2 Hyperphosphatemia
5.3 Embryo-Fetal Toxicity
6 ADVERSE REACTIONS
6.1 Clinical Trials Experience
7 DRUG INTERACTIONS
7.1 Effect of Other Drugs on BALVERSA
7.2 Effect of BALVERSA on Other Drugs
8 USE IN SPECIFIC POPULATIONS
8.1 Pregnancy
8.2 Lactation
8.3 Females and Males of Reproductive Potential
8.4 Pediatric Use
8.5 Geriatric Use
8.6 CYP2C9 Poor Metabolizers
11 DESCRIPTION
12 CLINICAL PHARMACOLOGY
12.1 Mechanism of Action
12.2 Pharmacodynamics
12.3 Pharmacokinetics
12.5 Pharmacogenomics
13 NONCLINICAL TOXICOLOGY
13.1 Carcinogenesis, Mutagenesis, and Impairment of
Fertility
14 CLINICAL STUDIES
14.1 Urothelial Carcinoma with Susceptible FGFR
Genetic Alterations
16 HOW SUPPLIED/STORAGE AND HANDLING
17 PATIENT COUNSELING INFORMATION

*Sections or subsections omitted from the full prescribing information are not
listed.

### FULL PRESCRIBING INFORMATION

### 1 INDICATIONS AND USAGE

BALVERSA^{™} is indicated for the treatment of adult patients with locally advanced or metastatic urothelial carcinoma (mUC), that has:
- susceptible FGFR3 or FGFR2 genetic alterations, and
- progressed during or following at least one line of prior platinum-containing chemotherapy, including within 12 months of neoadjuvant or adjuvant platinum-containing chemotherapy.

Select patients for therapy based on an FDA-approved companion diagnostic for BALVERSA *[see Dosage and Administration (2.1) and Clinical Studies (14)].*

This indication is approved under accelerated approval based on tumor response rate. Continued approval for this indication may be contingent upon verification and description of clinical benefit in confirmatory trials *[see Clinical Studies (14)].*

### 2 DOSAGE AND ADMINISTRATION

### 2.1 Patient Selection

Select patients for the treatment of locally advanced or metastatic urothelial carcinoma with BALVERSA based on the presence of susceptible FGFR genetic alterations in tumor specimens as detected by an FDA-approved companion diagnostic *[see Clinical Studies (14.1)].*

Information on FDA-approved tests for the detection of FGFR genetic alterations in urothelial cancer is available at: *http:*//*www.fda.gov*/*CompanionDiagnostics.*

### 2.2 Recommended Dosage and Schedule

The recommended starting dose of BALVERSA is 8 mg (two 4 mg tablets) orally once daily. with a dose increase to 9 mg (three 3 mg tablets) once daily based on serum phosphate (PO₄) levels and tolerability at 14 to 21 days *[see Dosage and Administration (2.3)].*

Swallow tablets whole with or without food. If vomiting occurs any time after taking BALVERSA, the next dose should be taken the next day. Treatment should continue until disease progression or unacceptable toxicity occurs.

If a dose of BALVERSA is missed, it can be taken as soon as possible on the same day. Resume the regular daily dose schedule for BALVERSA the next day. Extra tablets should not be taken to make up for the missed dose.

### Dose Increase based on Serum Phosphate Levels

Assess serum phosphate levels 14 to 21 days after initiating treatment. Increase the dose of BALVERSA to 9 mg once daily if serum phosphate level is < 5.5 mg/dL and there are no ocular disorders or Grade 2 or greater adverse reactions. Monitor phosphate levels monthly for hyperphosphatemia *[see Pharmacodynamics (12.2)].*

### 2.3 Dose Modifications for Adverse Reactions

The recommended dose modifications for adverse reactions are listed in Table 1.

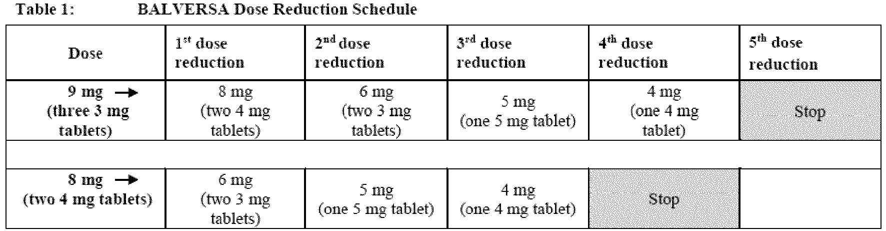

Table 2 summarizes recommendations for dose interruption, reduction, or discontinuation of BALVERSA in the management of specific adverse reactions.

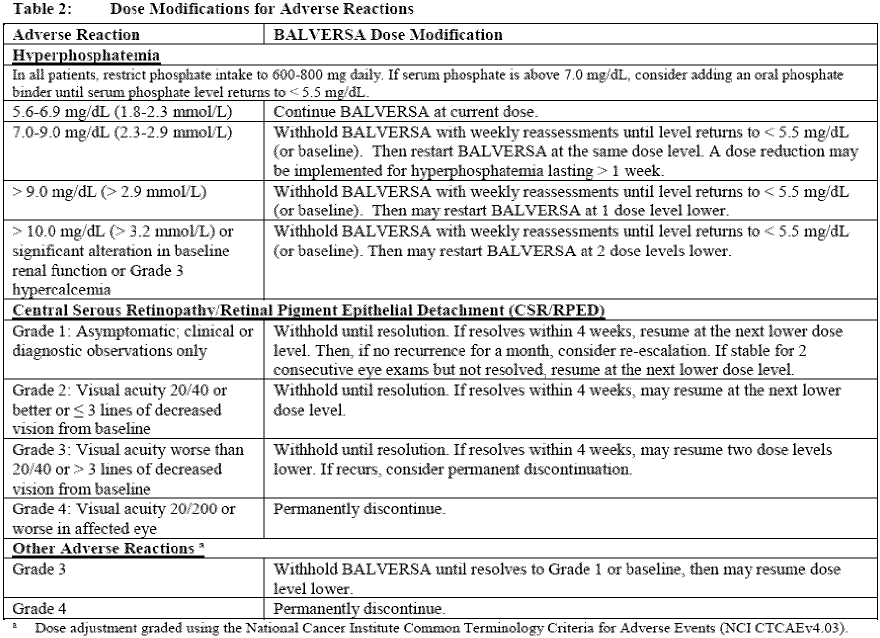

### 3 DOSAGE FORMS AND STRENGTHS

Tablets:
- 3 mg: Yellow, round biconvex, film-coated, debossed with "3" on one side: and "EF" on the other side.
- 4 mg: Orange, round biconvex, film-coated, debossed with "4" on one side: and "EF" on the other side.
- 5 mg: Brown, round biconvex, film-coated, debossed with "5" on one side: and "EF" on the other side.

### 4 CONTRAINDICATIONS

None.

### 5 WARNINGS AND PRECAUTIONS

### 5.1 Ocular Disorders

BALVERSA can cause ocular disorders. including central serous retinopathy/retinal pigment epithelial detachment (CSR/RPED) resulting in visual field detect.

CSR/RPED was reported in 25% of patients treated with BALVERSA, with a median time to first onset of 50 days. Grade 3 CSR/RPED, involving central field of vision, was reported in 3% of patients. CSR/RPED resolved in 13%) of patients and was ongoing in 13% of patients at the study cutoff. CSR/RPED led to dose interruptions and reductions in 9% and 14% of patients. respectively and 3% of patients discontinued BALVERSA.

Dry eye symptoms occurred in 28% of patients during treatment with BALVERSA and were Grade 3 in 6% of patients. All patients should receive dry eye prophylaxis with ocular demulcents as needed.

Perform monthly ophthalmological examinations during the first 4 months of treatment and every 3 months afterwards. and urgently at any time for visual symptoms. Ophthalmological examination should include assessment of visual acuity, slit lamp examination, fundoscopy, and optical coherence tomography.

Withhold BALVERSA when CSR occurs and permanently discontinue if it does not resolve within 4 weeks or if Grade 4 in severity. For ocular adverse reactions, follow the dose modification guidelines *[see Dosage and Administration (2.3)].*

### 5.2 Hyperphosphatemia

Increases in phosphate levels are a pharmacodynamic effect of BALVERSA *[see Pharmacodynamics (12.2)].* Hyperphosphatemia was reported as adverse reaction in 76% of patients treated with BALVERSA.The median onset time for any grade event of hyperphosphatemia was 20 days (range: 8 - 116) after initiating BALVERSA.Thirty-two percent of patients received phosphate binders during treatment with BALVERSA.

Monitor for hyperphosphatemia and follow the dose modification guidelines when required *[see Dosage and Administration 2.2, 2.3].*

### 5.3 Embryo-Fetal Toxicity

Based on the mechanism of action and findings in animal reproduction studies, BALVERSA can cause fetal harm when administered to a pregnant woman. In an embryo-fetal toxicity study, oral administration of erdafitinib to pregnant rats during the period of organogenesis caused malformations and embryo-fetal death at maternal exposures that were less than the human exposures at the maximum human recommended dose based on area under the curve (AUC). Advise pregnant women of the potential risk to the fetus. Advise female patients of reproductive potential to use effective contraception during treatment with BAL VERSA and for one month after the last dose. Advise male patients with female partners of reproductive potential to use effective contraception during treatment with BALVERSA and for one month after the last dose *[see* Use *in Specific Populations (8.1, 8.3) and Clinical Pharmacology (12.1)].*

### 6 ADVERSE REACTIONS

The following serious adverse reactions are also described elsewhere in the labeling:
- Ocular Disorders *[see Warning and Precautions (5.1)].*
- Hyperphosphatemia *[see Warning and Precautions (5.2)]*.

### 6.1 Clinical Trials Experience

Because clinical trials are conducted under widely varying conditions, adverse reaction rates observed in the clinical trials of a drug cannot be directly compared to rates in the clinical trials of another drug and may not reflect the rates observed in practice.

The safety of BALVERSA was evaluated in the BLC2001 study that included 87 patients with locally advanced or metastatic urothelial carcinoma which had susceptible FGFR3 or FGFR2 genetic alterations, and which progressed during or following at least one line of prior chemotherapy including within 12 months of neoadjuvant or adjuvant chemotherapy *[see Clinical Studies (14.1)]*. Patients were treated with BALVERSA at 8 mg orally once daily; with a dose increase to 9 mg in patients with phosphate levels <5.5 mg/dL on Day 14 of Cycle 1. Median duration of treatment was 5.3 months (range: 0 to 17 months).

The most common adverse reactions (ARs) including laboratory abnormalities (≥20%) were phosphate increased, stomatitis, fatigue, creatinine increased, diarrhea, dry mouth, onycholysis, alanine aminotransferase increased, alkaline phosphatase increased, sodium decreased, decreased appetite, albumin decreased, dysgeusia, hemoglobin decreased, dry skin, aspartate aminotransferase increased, magnesium decreased, dry eye, alopecia, palmar-plantar erythrodysesthesia syndrome, constipation, phosphate decreased, abdominal pain, calcium increased, nausea, and musculoskeletal pain. The most common Grade 3 or greater ARs (>1%) were stomatitis, nail dystrophy, palmar-plantar erythrodysesthesia syndrome, paronychia, nail disorder. keratitis, onycholysis, and hyperphosphatemia. An adverse reaction with a fatal outcome in 1% of patients was acute myocardial infarction.

Serious adverse reactions occurred in 41% of patients including eye disorders (10%).

Permanent discontinuation due to an adverse reaction occurred in 13% of patients. The most frequent reasons for permanent discontinuation included eye disorders (6%).

*Dosage* interruptions occurred in 68% of patients. The most frequent adverse reactions requiring dosage interruption included hyperphosphatemia (24%), stomatitis (17%), eye disorders (17%), and palmar-plantar erythro-dysaesthesia syndrome (8%).

Dose reductions occurred in 53% of patients. The most frequent adverse reactions for dose reductions included eye disorders (23%), stomatitis (15%), hyperphosphatemia (7%), palmar-plantar erythro-dysaesthesia syndrome (7%), paronychia (7%), and nail dystrophy (6%).

Table 3 presents ARs reported in ≥10% of patients treated with BALVERSA at 8 mg once daily.

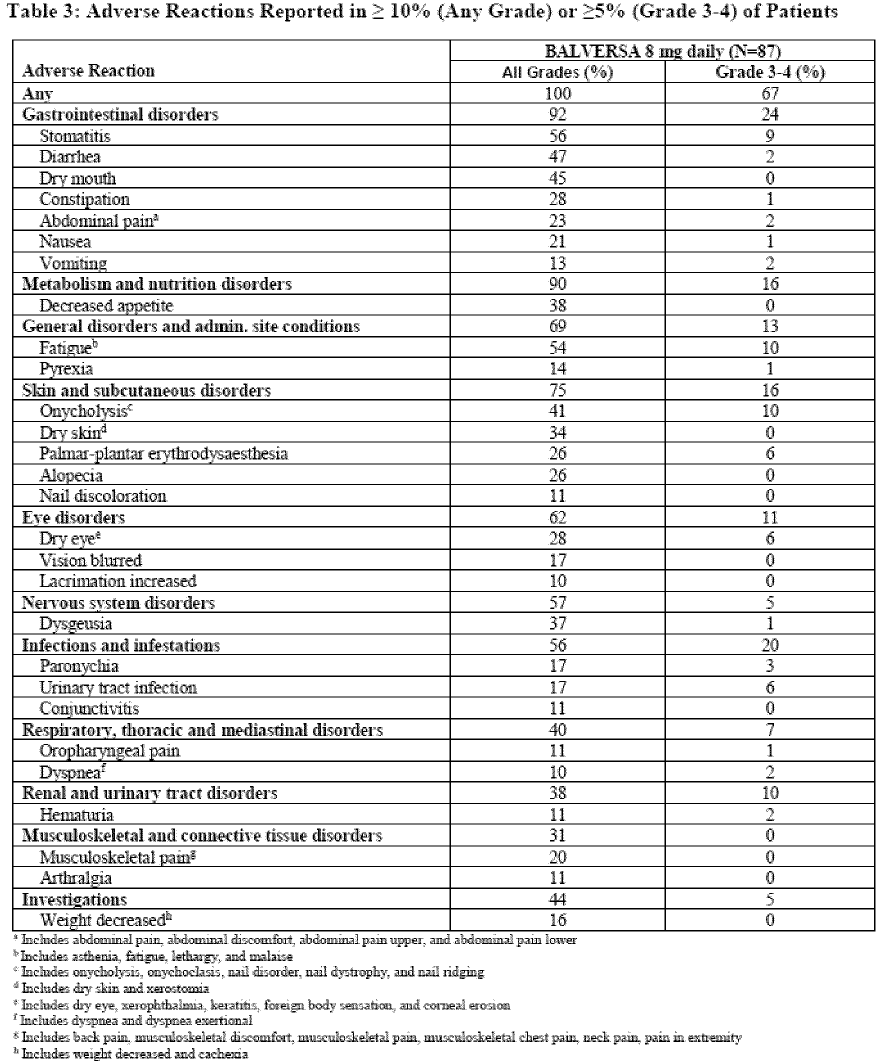

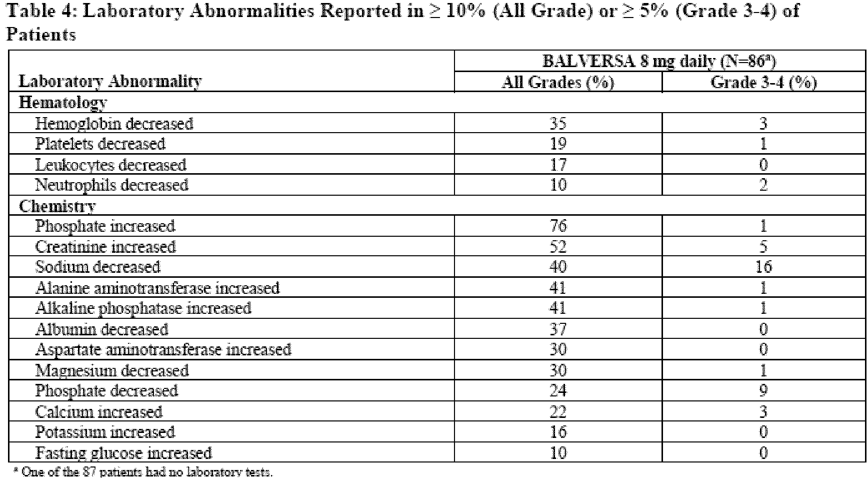

### 7 DRUG INTERACTIONS

### 7.1 Effect of Other Drugs on BALVERSA

Table 5 summanzes drug interactions that affect the exposure of BALVERSA or serum phosphate level and their clinical management.

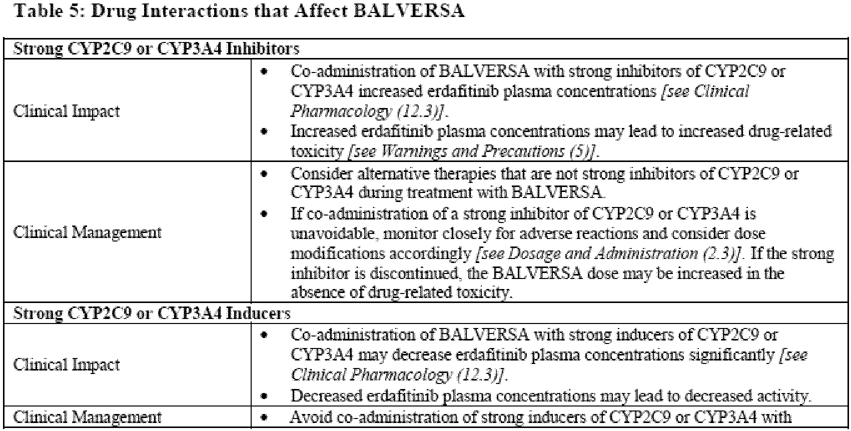

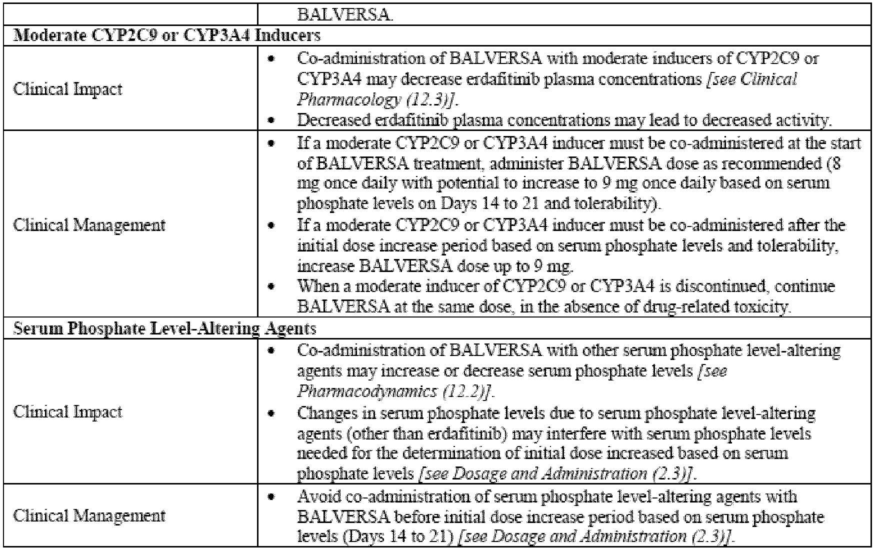

### 7.2 Effect of BALVERSA on Other Drugs

Table 6 summarizes the effect of BALVERSA on other drugs and their clinical management.

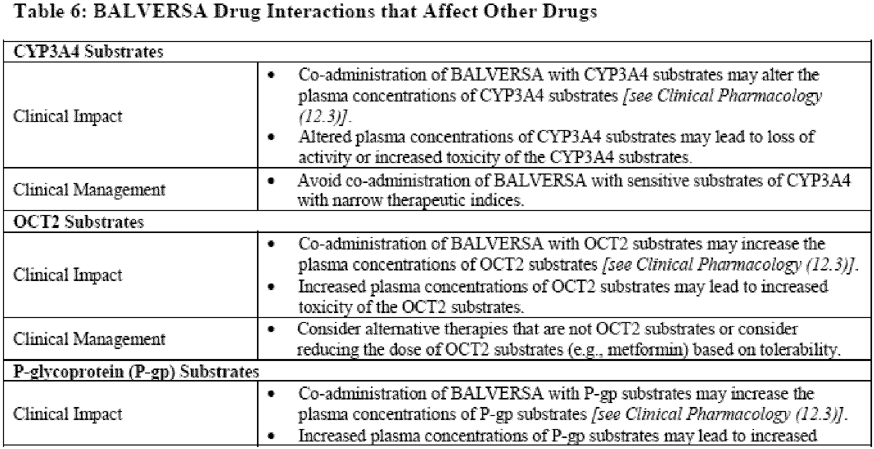

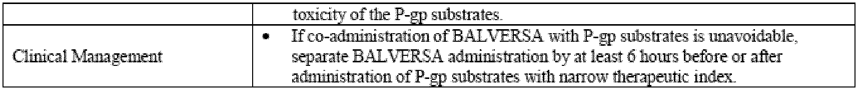

### 8 USE IN SPECIFIC POPULATIONS

### 8.1 Pregnancy

### Risk Summary

Based on the mechanism of action and findings in animal reproduction studies, BALVERSA can cause fetal harm when administered to a pregnant woman *[see Clinical Pharmacology (12.1)]*. There are no available data on BALVERSA use in pregnant women to inform a drug-associated risk. Oral administration of erdafitinib to pregnant rats during organogenesis caused malformations and embryo-fetal death at maternal exposures that were less than the human exposures at the maximum recommended human dose based on AUC (see *Data*)*.* Advise pregnant women and females of reproductive potential of the potential risk to the fetus.

The estimated background risk of major birth defects and miscarriage for the indicated population is unknown. All pregnancies have a background risk of birth defect, loss, or other adverse outcomes. In the U.S. general population, the estimated background risk of major birth defects and miscarriage in clinically recognized pregnancies is 2-4% and 15-20%, respectively.

### Data

### Animal Data

In an embryo-fetal toxicity study, erdafitinib was orally administered to pregnant rats during the period of organogenesis. Doses ≥4mg/kg/day (at total maternal exposures <0.1% of total human exposures at the maximum recommended human dose based on AUC) produced embryo-fetal death, major blood vessel malformations and other vascular anomalies, limb malformations (ectrodactyly, absent or misshapen long bones), an increased incidence of skeletal anomalies in multiple bones (vertebrae, sternebrae, ribs), and decreased fetal weight.

### 8.2 Lactation

### Risk Summary

There are no data on the presence of erdafitinib in human milk, or the effects of erdafitinib on the breastfed child, or on milk production. Because of the potential for serious adverse reactions from erdafitinib in a breastfed child, advise lactating women not to breastfeed during treatment with BALVERSA and for one month following the last dose.

### 8.3 Females and Males of Reproductive Potential

### Pregnancy Testing

Pregnancy testing is recommended for females of reproductive potential prior to initiating treatment with BALVERSA.

### Contraception

### Females

BALVERSA can cause fetal harm when administered to a pregnant woman. Advise females of reproductive potential to use effective contraception during treatment with BALVERSA and for one month after the last dose *[see Use in Specific Population (8.1)].*

### Males

Advise male patients with female partners of reproductive potential to use effective contraception during treatment with BALVERSA and for one month after the last dose *[see Use in Specific Populations (8.1)]*.

### Infertility

### Females

Based on findings from animal studies, BALVERSA may impair fertility in females of reproductive potential *[see Nonclinical Toxicology (13.1)].*

### 8.4 Pediatric Use

Safety and effectiveness of BALVERSA in pediatric patients have not been established.

In 4 and 13-week repeat-dose toxicology studies in rats and dogs, toxicities in bone and teeth were observed at an exposure less than the human exposure (AUC) at the maximum recommended human dose. Chondroid dysplasia/metaplasia were reported in multiple bones in both species, and tooth abnormalities included abnormal/irregular denting in rats and dogs and discoloration and degeneration of odontoblasts in rats.

### 8.5 Geriatric Use

Of the 416 patients treated with BALVERSA in clinical studies, 45% were 65 years of age or older, and 12% were 75 years of age or older. No overall differences in safety or effectiveness were observed between these patients and younger patients *[see Clinical Studies (14)].*

### 8.6 CYP2C9 Poor Metabolizers

*CYP2C9*3*/**3 Genotype:* Erdafitinib plasma concentrations were predicted to be higher in patients with the CYP2C9*3/*3 genotype. Monitor for increased adverse reactions in patients who are known or suspected to have CYP2C9*3/*3 genotype *[see Pharmacogenomics (12.5)].*

### 11 DESCRIPTION

Erdafitinib, the active ingredient in BALVERSA, is a kinase inhibitor. The chemical name is N-(3,5-dimethoxyphenyl)-N'-(1-methylethyl)-N-[3-(1-methyl-1H-pyrazol-4-yl)quinoxalin-6-yl]ethane-1,2-diamine. Erdafitinib is a yellow powder. It is practically insoluble, or insoluble to freely soluble in organic solvents, and slightly soluble to practically insoluble, or insoluble in aqueous media over a wide range of pH values. The molecular formula is C₂₅H₃₀N₆O₂ and molecular weight is 446.56.

Chemical structure of erdafitinib is as follows:

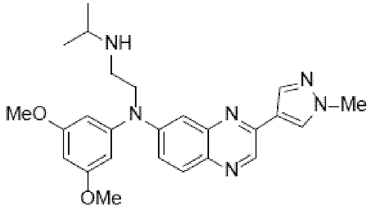

BALVERSA (erdafitinib) is supplied as 3 mg, 4 mg or 5 mg film-coated tablets for oral administration and contains the following inactive ingredients:
Tablet Core: Croscarmellose sodium, Magnesium stearate (from vegetable source), Mannitol, Meglumine, and Microcrystalline Cellulose.
Film Coating: (Opadry amb II): Glycerol monocaprylocaprate Type I, Polyvinyl alcohol-partially hydrolyzed, Sodium lauryl sulfate, Talc, Titanium dioxide, Iron oxide yellow, Iron oxide red (for the orange and brown tablets only), Ferrosoferric oxide/iron oxide black (for the brown tablets only).

### 12 CLINICAL PHARMACOLOGY

### 12.1 Mechanism of Action

Endafitinib is a kinase inhibitor that binds to and inhibits enzymatic activity of FGFR1, FGFR2, FGFR3 and FGFR4 based on in *vitro* data. Erdafitinib also binds to RET, CSF1R, PDGFRA, PDGFRB, FLT4, KIT, and VEGFR2. Erdafitinib inhibited FGFR phosphorylation and signaling and decreased cell viability in cell lines expressing FGFR genetic alterations, including point mutations, amplifications, and fusions. Erdafitinib demonstrated antitumor activity in FGFR-expressing cell lines and xenograft models derived from tumor types, including bladder cancer.

### 12.2 Pharmacodynamics

### Cardiac Electrophysiology

Based on evaluation of QTc interval in an open-label, dose escalation and dose expansion study in 187 patients with cancer, erdafitinib had no large effect (i.e., > 20 ms) on the QTc interval.

### Serum Phosphate

Erdafitinib increased serum phosphate level as a consequence of FGFR inhibition. BALVERSA should be increased to the maximum recommended dose to achieve target serum phosphate levels of 5.5-7.0 mg/dL in early cycles with continuous daily dosing *[see Dosage and Administration (2.3)]*.

In erdafitinib clinical trials, the use of drugs which can increase serum phosphate levels, such as potassium phosphate supplements, vitamin D supplements, antacids, phosphate-containing enemas or laxatives, and medications known to have phosphate as an excipient were prohibited unless no alternatives exist. To manage phosphate elevation, phosphate binders were permitted. Avoid concomitant use with agents that can after serum phosphate levels before the initial dose increase period based on serum phosphate levels *[see Drug Interactions (7.1)].*

### 12.3 Pharmacokinetics

Following administration of 8 mg once daily, the mean (coefficient of variation [CV%]) erdafitinib steady-state maximum observed plasma concentration (Cₘₐₓ), area under the curve (AUCₜₐᵤ), and minimum observed plasma concentration (Cₘᵢₙ) were 1,399 ng/mL (51%), 29,268 ng-h/mL (60%), and 936 ng/mL (65%), respectively.

Following single and repeat once daily dosing, erdafitinib exposure (maximum observed plasma concentration [Cₘₐₓ] and area under the plasma concentration time curve [AUC]) increased proportionally across the dose range of 0.3% to 12 mg (0.06 to 1.3 times the maximum approved recommended dose). Steady state was achieved after 2 weeks with once daily dosing and the mean accumulation ratio was 4-fold.

### Absorption

Median time to achieve peak plasma concentration (tₘₐₓ) was 2.5 hours (range: 2 to 6 hours).

### Effect of Food

No clinically meaningful differences with erdafitinib phannacokinetics were observed following administration of a high-fat and high-calorie meal (800 calories to 1,000 calories with approximately 50% of total caloric content of the meal from fat) in healthy subjects.

### Distribution

The mean apparent volume of distribution of erdafitinib was 29 L in patients.

Erdafitinib protein binding was 99.8% in patients, primarily to alpha-1-acid glycoprotein.

### Elimination

The mean total apparent clearance (CL/F) of erdafitinib was 0.362 L/h in patients.

The mean effective half-hfe of erdafitinib was 59 hours in patients.

### Metabolism

Erdafitinib is primarily metabolized by CYP2C9 and CYP3A4. The contribution of CYP2C9 and CYP3A4 in the total clearance of erdafitinib is estimated to be 39% and 20% respectively. Unchanged erdafitinib was the major drug-related moiety in plasma, there were no circulating metabolites.

### Excretion

Following a single oral dose of radiolabeled erdafitinib, approximately 69% of the dose was recovered in feces (19% as unchanged) and 19% in urine (13% as unchanged).

### Specific Populations

No clinically meaningful trends in the pharmacokinetics of erdafitinib were observed based on age (21-88 years), sex, race, body weight (36-132 kg), mild (eGFR [estimated glomerular filtration rate, using modification of diet in renal disease equation] 60 to 89 mL/min/1.73 m²) or moderate (eGFR 30-59 mL/min/1.73 m²) renal impairment or mild hepatic impairment (total bilirubin ≤ ULN and AST > ULN, or total bilirubin > 1.0-1.5 x ULN and any AST).

The pharmacokinetics of erdafitinib in patients with severe renal impairment. renal impairment requiring dialysis, moderate or severe hepatic impairment is unknown.

### Drug Interaction Studies

### Clinical Studies and Model-Based Approaches

### Strong CYP2C9 Inhibitors:

Erdafitinib mean ratios (90% CI) for Cₘₐₓ and AUC_{inf} were 121% (99.9, 147) and 148% (120, 182), respectively, when co-admmistered with fluconazole, a strong CYP2C9 inhibitor and moderate CYP3A4 inhibitor, relative to erdafitinib alone.

### Strong CYP3A4 Inhibitors:

Erdafitinib mean ratios (90% CI) for Cₘₐₓ and AUC_{inf} were 105% (86.7, 127) and 134% (109, 164), respectively, when co-administered with itraconazole (a strong CYP3A4 inhibitor and P-gp inhibitor) relative to erdafitinib alone.

### Strong CTP3A4/2C9 Inducers:

Simulations suggested that rifampicin (a strong CYP3A4/2C9 inducer) may significantly decrease erdafitinib Cₘₐₓ and AUC.

### In Vitro Studies

### CYP Substrates:

Erdafitinib is a time dependent inhibitor and inducer of CYP3A4. The effect of erdafitinib on a sensitive CYP3A4 substrate is unknown. Erdafitinib is not an inhibitor of other major CYP isozymes at clinically relevant concentrations.

### Transporters:

Erdafitinib is a substrate and inhibitor of P-gp. P-gp inhibitors are not expected to affect erdafitinib exposure to a clinically relevant extent. Erdafitinib is an inhibitor of OCT2.

Erdafitinib does not inhibit BCRP, OATP1B, OATP1B3, OAT1, OAT3, OCT1, MATE-1, or MATE-2K at clinically relevant concentrations.

### Acid-Lowering Agents:

Erdafitinib has adequate solubility across the pH range of 1 to 7.4. Acid-lowering agents (e.g., antacids, H₂-antagomsts, proton pump inhibitors) are not expected to affect the bioavailability of erdafitinib.

### 12.5 Pharmacogenomics

CYP2C9 activity is reduced in individuals with genetic variants, such as the CYP2C9*2 and CYP2C9*3 polymorphisms. Erdafitinib exposure was similar in subjects with CYP2C9*1/*2 and *1/*3 genotypes relative to subjects with CYP2C9*1/*1 genotype (wild type). No data are available in subjects characterized by other genotypes (e.g., *2/*2, *2/*3, *3/*3). Simulation suggested no clinically meaningful differences in erdafitinib exposure in subjects with CYP2C9*2/*2 and *2/*3 genotypes. The exposure of erdafitinib is predicted to be 50% higher in subjects with the CYP2C9*3/*3 genotype, estimated to be present in 0.4% to 3% of the population among various ethnic groups.

### 13 NONCLINICAL TOXICOLOGY

### 13.1 Carcinogenesis, Mutagenesis, and Impairment of Fertility

Carcinogenicity studies have not been conducted with erdafitinib.

Erdafitinib was not mutagenic in a bacterial reverse mutation (Ames) assay and was not clastogenic in an *in vitro* micronucleus or an *in vivo* rat bone marrow micronucleus assay.

Fertility studies in animals have not been conducted with erdafitinib. In the 3-month repeat-dose toxicity study, erdafitinib showed effects on female reproductive organs (necrosis of the ovarian corpora lutea) in rats at an exposure less than the human exposure (AUC) at maximum recommended human dose.

### 14 CLINICAL STUDIES

### 14.1 Urothelial Carcinoma with Susceptible FGFR Genetic Alterations

Study BLC2001 (NCT02365597) was a multicenter, open-label, single-arm study to evaluate the efficacy and safety of BALVERSA in patients with locally advanced or metastatic urothelial carcinoma (mUC). Fibroblast growth factor receptor (FGFR) mutation status for screening and enrollment of patients was determined by a clinical trial assay (CTA). The efficacy population consists of a cohort of eighty-seven patients who were enrolled in this study with disease that had progressed on or after at least one prior chemotherapy and that had at least 1 of the following genetic alterations: FGFR3 gene mutations (R248C, S249C, G370C, Y373C) or FGFR gene fusions (FGFR3-TACC3, FGFR3-BAIAP2L1. FGFR2-BICC1, FGFR2-CASP7), as determined by the CTA performed at a central laboratory. Tumor samples from 69 patients were tested retrospectively by the QIAGEN *therascreen*^{®} FGFR RGQ RT-PCR Kit, which is the FDA-approved test for selection of patients with mUC for BALVERSA.

Patients received a starting dose of BALVERSA at 8 mg once daily with a dose increase to 9 mg once daily in patients whose serum phosphate levels were below the target of 5.5 mg/dL between days 14 and 17; a dose increase occurred in 41% of patients. BALVERSA was administered until disease progression or unacceptable toxicity. The major efficacy outcome measures were objective response rate (ORR) and duration of response (DoR), as determined by blinded independent review committee (BIRC) according to RECIST v1.1.

The median age was 67 years (range: 36 to 87 years), 79% were male, and 74% were Caucasian. Most patients (92%) had a baseline Eastern Cooperative Oncology Group (ECOG) performance status of 0 or 1. Sixty-six percent of patients had visceral metastases. Eighty-four (97%) patients received at least one of cisplatin or carboplatin previously. Fifty-six percent of patients only received prior cisplatin-based regimens, 29% received only prior carboplatin-based regimens, and 10% received both cisplatin and carboplatin-based regimens Three (3%) patients had disease progression following prior platinum-containing neoadjuvant or adjuvant therapy only. Twenty-four percent of patients had been treated with prior anti PD-L1/PD-1 therapy.

Efficacy results are summarized in Table 7 and Table 8. Overall response rate was 32.2%. Responders included patients who had previously not responded to anti PD-L1/PD-1 therapy.

**Table 7: Efficacy Results**

| **Endpoint** | | **BIRC^{a} assessment** |
|---|---|---|
| | | **N=87** |
| ORR (95% CI) | | 32.2% (22.4, 42.0) |
| | Complete response (CR) | 2.3% |
| | Partial response (PR) | 29.9% |
| Median DoR in months (95% CI) | | 5.4 (4.2, 6.9) |

| | | |
|---|---|---|
| ^{a}BIRC: Blinded Independent Review Committee ORR = CR + PR CI = Confidence Interval | | |

**Table 8: Efficacy Results by FGFR Genetic Alteration**

| | | **BIRC^{a} assessment** |
|---|---|---|
| FGFR3 Point Mutation | | N=64 |
| | ORR (95% CI) | 40.6% (28.6, 52.7) |
| FGFR3 Fusion ^{b, c} | | N=18 |
| | ORR (95% CI) | 11.1%(0, 25.6) |
| FGFR2 Fusion ^{c} | | N=6 |
| | ORR | 0 |

| | | |
|---|---|---|
| ^{a} BIRC: Blinded Independent Review Committee ^{b} Both responders had FGFR3-TACC3_V1 fusion ^{c} One patient with a FGFR2-CASP7/FGFR3-TACC3_V3 fusion is reported in both FGFR2 fusion and FGFR3 fusion above ORR = CR + PR CI = Confidence Interval | | |

### 16 HOW SUPPLIEDISTORAGE AND HANDLING

BALVERSA^{™} (erdafitinib) tablets are available in the strengths and packages listed below:
- 3 mg tablets: Yellow, round biconvex, film-coated, debossed with ''3'' on one side and "EF" on the other side.
   - Bottle of 56-tablets with child resistant closure (NDC 39676-030-36).
   - Bottle of 84-tablets with child resistant closure (NDC 59676-030-84).
   - Two dose pack wallets of 28-tablets each (NDC 59676-030-22) in a box of 56-tablets (NDC 59676-030-55).
   - Two dose pack wallets of 42-tablets each (NDC 59676-030-44) in a box of 84-tablets (NDC 59676-030-88).
- 4 mg tablets: Orange, round biconvex, film-coated, debossed with "4" on one side and "EF" on the other side.
   - Bottle of 28-tablets with child resistant closure (NDC 59676-040-28).
   - Bottle of 56-tablets with child resistant closure (NDC 59676-040-56).
   - One starter pack wallet of 14-tablets in a box (NDC 59676-040-14).
   - One dose pack wallet of 28-tablets in a box (NDC 59676-040-22).
   - Two dose pack wallets of 28-tablets each (NDC 59676-040-22) in a box of 56-tablets (NDC 59676-040-55).
- 5 mg tablets: Brown, round biconvex, film-coated, debossed with "5" on one side and "EF" on the other side.
   - Bottle of 28-tablets with child resistant closure (NDC 59676-050-28).
   - One dose pack wallet of 28-tablets in a box (NDC 59676-050-2.2).

Store at 20°C-25°C (68°F-77°F); excursions permitted between 15°C and 30°C (59°F and 86°F) *[see USP Controlled Room Temperature].*

### 17 PATIENT COUNSELING INFORMATION

Advise the patient to read the FDA-approved patient labeling (Patient Information).

FGFR genetic alterations: Advise patients that evidence of a susceptible FGFR3 or FGFR2 mutation or gene fusion within the tumor specimen is necessary to identify patients for whom treatment is indicated *[see Dosage and Administration (2.1)].*

Ocular disorders: Advise patients to contact their healthcare provider if they experience any visual changes *[see Warnings* and *Precautions (5.1)]*. In order to prevent or treat dry eyes, advise patients to use artificial tear substitutes, hydrating or lubricating eye gels or ointments frequently, at least every 2 hours during waking hours *[see Dosage* and *Administration (2.3)].*

Skin, mucous or nail disorders: Advise patients to contact their healthcare provider if they experience progressive or intolerable skin, mucous or nail disorders *[see Adverse Reactions (6.1)].*

Hyperphosphatemia: Advise patients that their healthcare provider will assess their serum phosphate level between 14 and 21 days of initiating treatment and will adjust the dose if needed *[see Warnings* and *Precautions (5.2)]*. During this initial phosphate-assessment period, advise patients to avoid concomitant use with agents that can alter serum phosphate levels. Advise patients that, after the mitial phosphate assessment period, monthly phosphate level monitoring for hyperphosphatemia should be performed during treatment with BALVERSA *[see Drug Interactions (7.1)]*.

*Drug* Interactions: Advise patients to form their healthcare providers of all concomitant medications, including prescoption medicines, over-the-counter drugs, and herbal products *[see Drug Interactions (7.1, 7.2)].*

Dosing Instructions: Instruct patients to swallow the tablets whole once daily with or without food. If vomiting occurs any time after taking BALVERSA, advise patients to take the next dose the next day. *[see Dosage and Administration (2.1)].*

Missed dose: If a dose is missed, advise patients to take the missed as soon as possible. Resume the regular daily dose schedule for BAL VERSA the next day. Extra tablets should not be taken to make up for the missed dose *[see Dosage and Administration (2.3)].*

Embryo-Fetal Toxicity: Advise pregnant women and females of reproductive potential of the potential risk to the fetus. Advise females to inform their healthcare providers of a known or suspected pregnancy *[see Warning and Precautions (3.3) and Use in Specific Population (8.1)].*

Advise female patients of reproductive potential to use effective contraception during treatment and for one month after the last dose of BALVERSA. Advise male patients with female partners of reproductive potential to use effective contraception during treatment and for one month after the last dose of BALVERSA *[see Use in Specific Populations (8.3)].*

Lactation: Advise females not to breastfeed during treatment with BALVERSA and for one month after the last dose *[see Use in Specific Populations (8.2)]*.

Product of Switzerland

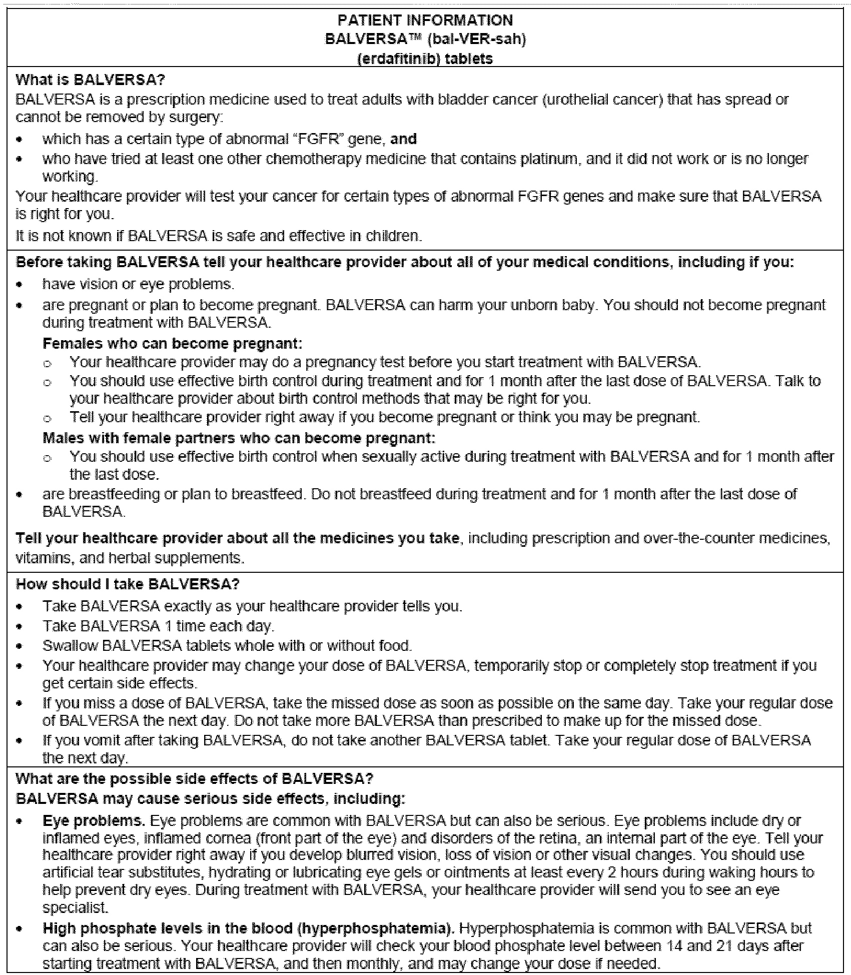

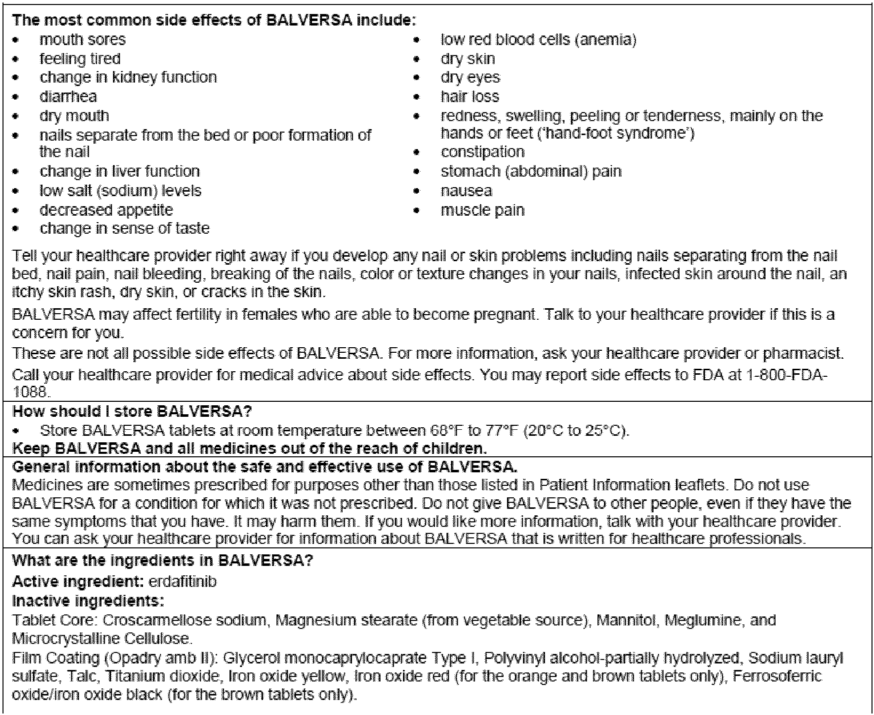

The examples and embodiments described herein are for illustrative purposes only and various modifications or changes suggested to persons skilled in the art are to be included within the spirit and purview of this application and scope of the appended claims.

It is to be understood that the method of treatment embodiments described herein can also be worded in the format of the use of erdafitinib for the manufacture of a medicament or in the format of erdafitinib for use in treatment.

The following clauses describe subject matters of the present invention.
1. A method of treating urothelial carcinoma comprising administering an approved drug product containing a fibroblast growth factor receptor (FGFR) inhibitor to a patient with a urothelial carcinoma in an amount that is described in a drug product label for said drug product.
2. The method of claim 1, wherein the urothelial carcinoma is locally advanced or metastatic.
3. The method of claim 1 or 2, wherein administration of the FGFR inhibitor provides improved anti-tumor activity as measured by objective response rate or duration of response relative to a patient with urothelial carcinoma that is not receiving treatment with an FGFR inhibitor.
4. The method of any one of the preceding claims, wherein administration of the FGFR inhibitor results in no more than a grade 3 adverse event.
5. The method of any one of the preceding claims, wherein the urothelial carcinoma is susceptible to an FGFR2 genetic alteration or an FGFR3 genetic alteration.
6. The method of claim 5, wherein the FGFR2 or FGFR3 genetic alteration is an FGFR3 gene mutation or an FGFR2 or FGFR3 gene fusion.
7. The method of claim 6, wherein the FGFR3 gene mutation is R248C, S249C, G370C, Y373C, or any combination thereof.
8. The method of claim 6, wherein the FGFR2 or FGFR3 gene fusion is FGFR3-TACC3, FGFR3-BAIAP2L1, FGFR2-BICC1, FGFR2-CASP7, or any combination thereof.
9. The method of any one of the preceding claims, further comprising evaluating a biological sample from the patient for the presence of one or more FGFR2 or FGFR3 genetic alterations prior to administration of the FGFR inhibitor.
10. The method of claim 9, wherein the biological sample is blood, lymph fluid, bone marrow, a solid tumor sample, or any combination thereof.
11. The method of any one of the preceding claims, wherein the patient received at least one prior therapy for the treatment of urothelial carcinoma.
12. The method of claim 11, wherein the at least one prior therapy for the treatment of urothelial carcinoma is platinum-containing chemotherapy.
13. The method of claim 12, wherein the urothelial carcinoma progressed during or following at least one line of the platinum-containing chemotherapy.
14. The method of claim 13, wherein the platinum-containing chemotherapy is neoadjuvant platinum-containing chemotherapy or adjuvant platinum-containing chemotherapy.
15. The method of claim 14, wherein the urothelial carcinoma progressed during or within 12 months following at least one line of the neoadjuvant platinum-containing chemotherapy or adjuvant platinum-containing chemotherapy.
16. The method of any one of the preceding claims wherein the FGFR inhibitor is erdafitinib.
17. The method of claim 16, wherein erdafitinib is administered daily.
18. The method of claim 16 or 17, wherein erdafitinib is administered orally.
19. The method of any one of claims 16 to 18, wherein erdafitinib is administered orally on a continuous daily dosing schedule.
20. The method of any one of claims 16 to 19, wherein erdafitinib is administered orally at a dose of about 8 mg once daily.
21. The method of claim 20, wherein the dose of erdafitinib is increased from 8 mg once daily to 9 mg once daily at 14 to 21 days after initiating treatment if:
   (a) the patient exhibits a serum phosphate (PO₄) level that is less than about 5.5 mg/dL at 14-21 days after initiating treatment; and
   (b) administration of erdafitinib at 8 mg once daily resulted in no ocular disorder; or
   (c) administration of erdafitinib at 8 mg once daily resulted in no Grade 2 or greater adverse reaction.
22. The method of any one of claims 16 to 21, wherein erdafitinib is present in a solid dosage form.
23. The method of claim 22, wherein the solid dosage form is a tablet.
24. The method of any one of claims 16 to 23, wherein erdafitinib is not co-administered with:
   (a) a medication that is a strong CYP2C9 inhibitor or CYP3A4 inhibitor;
   (b) a medication that is a strong CYP2C9 inducer or CYP3A4 inducer;
   (c) a medication that is a moderate CYP2C9 inducer or CYP3A4 inducer; or
   (d) a medication that is a serum phosphate level-altering agent.
25. The method of any one of claims 16 to 24, wherein erdafitinib is not co-administered with:
   (a) a medication that is a CYP3A4 substrate;
   (b) a medication that is a OCT2 substrate; or
   (c) a medication that is a P-glycotprotein (P-gp) substrate.
26. A method of treating urothelial carcinoma in a patient comprising:
   (a) evaluating a biological sample from the patient for the presence of one or more fibroblast growth factor receptor (FGFR) gene alterations; and
   (b) treating the patient with an approved drug product containing an FGFR inhibitor in an amount that is described in a drug product label for said drug product if one or more FGFR gene alterations is present in the sample.
27. A method of selling an approved drug product comprising erdafitinib, said method comprising selling such drug product, wherein a drug product label for a reference listed drug for such drug product includes instructions for treating urothelial carcinoma.
28. The method of claim 27, wherein the drug product is an ANDA drug product, a supplemental New Drug Application drug product or a 505(b)(2) drug product.
29. A method of offering for sale an approved drug product comprising erdafitinib, said method comprising offering for sale such drug product, wherein a drug product label for a reference listed drug for such drug product includes instructions for treating urothelial carcinoma.
30. The method of claim 29, wherein the drug product is an ANDA drug product, a supplemental New Drug Application drug product or a 505(b)(2) drug product.
31. A method comprising selling an approved drug product comprising erdafitinib, wherein the drug product label for a reference listed drug for such drug product comprises objective response rate or duration of response data.
32. The method of claim 31, wherein the objective response rate data for erdafitinib is about 32.2%.
33. The method of claim 31, wherein the duration of response data for erdafitinib is about 5.4 months.
34. A method of improving objective response rate or duration of response in a patient with urothelial carcinoma relative to a patient with urothelial carcinoma that is not receiving treatment with an FGFR inhibitor, said method comprising administering to said patient an approved drug product comprising erdafitinib.
35. The method of claim 34, wherein the objective response rate is about 32.2%.
36. The method of claim 34, wherein the duration of response is about 5.4 months.
37. The method of any one of claims 34 to 36, wherein the approved drug product is an ANDA drug product or a supplemental New Drug Application drug product.
38. A method of improving objective response rate or duration of response in a patient with urothelial carcinoma relative to a patient with urothelial carcinoma that is not receiving treatment with an FGFR inhibitor, said method comprising providing to said patient an approved drug product comprising erdafitinib.
39. The method of claim 38, wherein the objective response rate is about 32.2%.
   The method of claim 38, wherein the duration of response is about 5.4 months
40. The method of any one of claims 38 to 40, wherein the approved drug product is an ANDA drug product or a supplemental New Drug Application drug product.
41. The method of any one of claims 38 to 41, wherein the drug product label for a reference listed drug for such drug product includes instructions for treating urothelial carcinoma.
42. An approved drug product with at least one approved indication, wherein said approved drug product comprises erdafitinib.
43. The approved drug product of claim 43, wherein the approved drug product is an NDA drug product, an ANDA drug product, a supplemental New Drug Application drug product, or a 505(b)(2) drug product.
44. The approved drug product of claim 43, wherein a reference listed drug product for the approved drug product includes a drug product label.
45. The approved drug product of claim 45, wherein the drug product label comprises objective response rate data.
46. The approved drug product of claim 46, wherein the objective response rate data for erdafitinib is about 32.2%.
47. The approved drug product of claim 45, wherein the drug product label comprises duration of response data.
48. The approved drug product of claim 48, wherein the duration of response data for erdafitinib is about 5.4 months.
49. An approved drug product containing a fibroblast growth factor receptor (FGFR) inhibitor for use in the treatment of urothelial carcinoma in a patient, wherein the approved drug product is administered in an amount that is described in a drug product label for said drug product.
50. A use of an approved drug product containing a fibroblast growth factor receptor (FGFR) inhibitor in the manufacture of a medicament for the treatment of urothelial carcinoma in a patient, wherein the medicament is administered in an amount that is described in a drug product label for said drug product.
51. An approved pharmaceutical product comprising erdafitinib for the treatment of adult patients with locally advanced or metastatic urothelial carcinoma that has
   (a) susceptible FGFR3 or FGFR2 genetic alterations and
   (b) progressed during or following at least one line of prior platinum containing chemotherapy including within 12 months of neoadjuvant or adjuvant platinum-containing chemotherapy.

The following clauses describe subject matters of the present invention.
1. A method of treating urothelial carcinoma comprising administering erdafitinib to a patient with a urothelial carcinoma wherein erdafitinib is co-administered with a P-glycoprotein (P-gp) substrate and administration of erdafitinib is separated by at least 6 hours before or after administration of the P-gp substrate, in particular wherein the P-gp substrate has a narrow therapeutic index.
2. A method of treating urothelial carcinoma comprising administering erdafitinib to a patient with a urothelial carcinoma wherein erdafitinib is co-administered with a moderate CYP2C9 or CYP3A4 inducer.
3. The method of claim 2, wherein the moderate CYP2C9 or CYP3A4 inducer is co-administered at the start of erdafitinib treatment.
4. The method of any one of the preceding claims, wherein the urothelial carcinoma is locally advanced or metastatic.
5. The method of any one of the preceding claims, wherein the urothelial carcinoma is susceptible to an FGFR2 genetic alteration or an FGFR3 genetic alteration.
6. The method of claim 5, wherein the FGFR2 or FGFR3 genetic alteration is an FGFR3 gene mutation or an FGFR2 or FGFR3 gene fusion.
7. The method of claim 6, wherein the FGFR3 gene mutation is R248C, S249C, G370C, Y373C, or any combination thereof.
8. The method of claim 6, wherein the FGFR2 or FGFR3 gene fusion is FGFR3-TACC3, FGFR3-BAIAP2L1, FGFR2-BICC1, FGFR2-CASP7, or any combination thereof.
9. The method of any one of the preceding claims, further comprising evaluating a biological sample from the patient for the presence of one or more FGFR2 or FGFR3 genetic alterations prior to administration of erdafitinib.
10. The method of claim 9, wherein the biological sample is blood, lymph fluid, bone marrow, a solid tumor sample, or any combination thereof.
11. The method of any one of the preceding claims, wherein the patient received at least one prior therapy for the treatment of urothelial carcinoma.
12. The method of claim 11, wherein the at least one prior therapy for the treatment of urothelial carcinoma is platinum-containing chemotherapy.
13. The method of claim 11, wherein the urothelial carcinoma progressed during or following at least one line of the platinum-containing chemotherapy.
14. The method of claim 12, wherein the platinum-containing chemotherapy is neoadjuvant platinum-containing chemotherapy or adjuvant platinum-containing chemotherapy.
15. The method of claim 14, wherein the urothelial carcinoma progressed during or within 12 months following at least one line of the neoadjuvant platinum-containing chemotherapy or adjuvant platinum-containing chemotherapy.
16. The method of any one of the preceding claims, wherein erdafitinib is administered daily.
17. The method of any one of the preceding claims, wherein erdafitinib is administered orally.
18. The method of claim 16 or 17, wherein erdafitinib is administered orally on a continuous daily dosing schedule.
19. The method of claim 18, wherein erdafitinib is administered orally at a dose of about 8 mg once daily.
20. The method of claim 19, wherein the dose of erdafitinib is increased from 8 mg once daily to 9 mg once daily at 14 to 21 days after initiating treatment if:
   (a) the patient exhibits a serum phosphate (PO₄) level that is less than about 5.5 mg/dL at 14-21 days after initiating treatment; and
   (b) administration of erdafitinib at 8 mg once daily resulted in no ocular disorder; or
   (c) administration of erdafitinib at 8 mg once daily resulted in no Grade 2 or greater adverse reaction.
21. The method of any one of the preceding claims, wherein erdafitinib is present in a solid dosage form.
22. The method of claim 21, wherein the solid dosage form is a tablet.
23. A use of erdafitinib in the manufacture of a medicament for the treatment of urothelial carcinoma in a patient, wherein the medicament is co-administered with a P-glycoprotein (P-gp) substrate and administration of erdafitinib is separated by at least 6 hours before or after administration of the P-gp substrate, in particular wherein the P-gp substrate has a narrow therapeutic index.
24. A use of erdafitinib in the manufacture of a medicament for the treatment of urothelial carcinoma in a patient, wherein the medicament is co-administered with a moderate CYP2C9 or CYP3A4 inducer.
25. The use according to claim 24, wherein the moderate CYP2C9 or CYP3A4 inducer is co-administered at the start of erdafitinib treatment.
26. Erdafitinib for use in the treatment of urothelial carcinoma in a patient, wherein erdafitinib is co-administered with a P-glycoprotein (P-gp) substrate and administration of erdafitinib is separated by at least 6 hours before or after administration of the P-gp substrate, in particular wherein the P-gp substrate has a narrow therapeutic index.
27. Erdafitinib for use in the treatment of urothelial carcinoma in a patient, wherein erdafitinib is is co-administered with a moderate CYP2C9 or CYP3A4 inducer.
28. Erdafitinib for use of claim 27, wherein the moderate CYP2C9 or CYP3A4 inducer is co-administered at the start of erdafitinib treatment.
29. The use of any one of claims 23 to 25, or erdafitinib for use of any one of claims 26 to 28, wherein the urothelial carcinoma is locally advanced or metastatic.
30. The use of any one of claims 23 to 25, or erdafitinib for use of any one of claims 26 to 28, wherein the urothelial carcinoma is susceptible to an FGFR2 genetic alteration or an FGFR3 genetic alteration.
31. The use or erdafitinib for use of claim 30, wherein the FGFR2 or FGFR3 genetic alteration is an FGFR3 gene mutation or an FGFR2 or FGFR3 gene fusion.
32. The use or erdafitinib for use of claim 31, wherein the FGFR3 gene mutation is R248C, S249C, G370C, Y373C, or any combination thereof.
33. The use or erdafitinib for use of claim 31, wherein the FGFR2 or FGFR3 gene fusion is FGFR3-TACC3, FGFR3-BAIAP2L1, FGFR2-BICC1, FGFR2-CASP7, or any combination thereof.
34. The use or erdafitinib for use of any one of claims 23 to 33, further comprising evaluating a biological sample from the patient for the presence of one or more FGFR2 or FGFR3 genetic alterations prior to administration of erdafitinib.
35. The use or erdafitinib for use of claim 34, wherein the biological sample is blood, lymph fluid, bone marrow, a solid tumor sample, or any combination thereof.
36. The use or erdafitinib for use of any one of claims 23 to 35, wherein the patient received at least one prior therapy for the treatment of urothelial carcinoma.
37. The use or erdafitinib for use of claim 36, wherein the at least one prior therapy for the treatment of urothelial carcinoma is platinum-containing chemotherapy.
38. The use or erdafitinib for use of claim 36, wherein the urothelial carcinoma progressed during or following at least one line of the platinum-containing chemotherapy.
39. The use or erdafitinib for use of claim 37, wherein the platinum-containing chemotherapy is neoadjuvant platinum-containing chemotherapy or adjuvant platinum-containing chemotherapy.
40. The use or erdafitinib for use of claim 39, wherein the urothelial carcinoma progressed during or within 12 months following at least one line of the neoadjuvant platinum-containing chemotherapy or adjuvant platinum-containing chemotherapy.
41. The use or erdafitinib for use of any one of claims 23 to 40, wherein erdafitinib is administered daily.
42. The use or erdafitinib for use of any one of claims 23 to 41, wherein erdafitinib is administered orally.
43. The use or erdafitinib for use of claim 41 or 42, wherein erdafitinib is administered orally on a continuous daily dosing schedule.
44. The use or erdafitinib for use of claim 43, wherein erdafitinib is administered orally at a dose of about 8 mg once daily.
45. The use or erdafitinib for use of claim 44, wherein the dose of erdafitinib is increased from 8 mg once daily to 9 mg once daily at 14 to 21 days after initiating treatment if:
   (a) the patient exhibits a serum phosphate (PO₄) level that is less than about 5.5 mg/dL at 14-21 days after initiating treatment; and
   (b) administration of erdafitinib at 8 mg once daily resulted in no ocular disorder; or
   (c) administration of erdafitinib at 8 mg once daily resulted in no Grade 2 or greater adverse reaction.
46. The use or erdafitinib for use of any one of claims 23 to 45, wherein erdafitinib is present in a solid dosage form.
47. The use or erdafitinib for use of claim 46, wherein the solid dosage form is a tablet.

## Claims

1. Erdafitinib for use in the treatment of urothelial carcinoma in a patient, wherein the erdafitinib is administered orally at a dose of 8 mg once daily or 9 mg once daily, and wherein the dose of erdafitinib is reduced if the erdafitinib is co-administered with a strong CYP3A4 inhibitor.

2. The erdafitinib for use according to claim 1, wherein the 8 mg once daily is administered as two 4 mg tablets, or the 9 mg once daily is administered as three 3 mg tablets.

3. The erdafitinib for use according to claim 1 or claim 2, wherein the 8 mg once daily is reduced to 6 mg once daily, which is optionally administered as two 3 mg tablets, or the 9 mg once daily is reduced to 8 mg once daily, which is optionally administered as two 4 mg tablets.

4. Erdafitinib for use in the treatment of urothelial carcinoma in a patient, wherein the erdafitinib is not co-administered with grapefruit juice.

5. Erdafitinib for use in the treatment of urothelial carcinoma in a patient, wherein the erdafitinib is administered orally at a dose of 8 mg once daily, and wherein the dose of erdafitinib is increased to 9 mg once daily if the erdafitinib is co-administered with a moderate CYP3A4 inducer.

6. Erdafitinib for use in the treatment of urothelial carcinoma in a patient, wherein the erdafitinib is not co-administered with a strong CYP3A4 inducer.

7. The erdafitinib for use according to any one of the preceding claims, wherein the urothelial carcinoma is unresectable or metastatic urothelial carcinoma harboring FGFR genetic alterations.
